# EUROPEAN PATENT APPLICATION

(11) **EP 4 509 137 A1**
(43) Date of publication of application: **19.02.2025**
(21) Application number: 24181475.5
(22) Date of filing: 11.06.2024
(51) Int. Cl.: A61K 39/12

(54) **A CHIMERIC ANTIGEN RECEPTOR COMBINATORIAL MULTI-ANTIGEN TARGETED ALLOGENEIC T CELL IMMUNOTHERAPY WITH REDUCED RISK OF T CELL EXHAUSTION, HOST VERSUS GRAFT REJECTION, AND GRAFT VERSUS HOST DISEASE**

(30) Priority: 14.06.2023 US 202363521021 P
(71) Applicant: Kuiper, Inc., Jersey City, NJ 07302 (US)
(72) Inventor: Pecora, Andrew L., Rumson, New Jersey 07760 (US)
(74) Representative: Schlich

(57) **Abstract**

The present disclosure provides a scalable and practical off-the-shelf allogeneic T cell therapy for treating cancer including employing a population of nonexhausted allogeneic virus specific CD4+ T cells, CD8+ T cells or CD4+ and CD8+ T cells that express a nonexhausted PD-1^{neg}TIGIT^{neg}TCF-1^{High} TOX^{Lo} early stem cell T_{SCM} phenotype. The virus-specific population of CD4+ T cells, CD8+ T cells or CD4+ and CD8+ T cells that expresses the nonexhausted early stem cell T_{SCM} phenotype and comprises TCRs specific for a protein of a virus is genetically modified to stably express up to three chimeric antigen-receptors (CARs). Each CAR is specific for a cancer antigen. At least one of the CARs comprises a CD30 CAR expressing a CD30-specific scFv fragment, and/or a chimeric alloimmune defense receptor (ADR); and /or a chimeric HLA accessory receptor fused with a cytolytic endodomain of the T cell receptor zeta chain, thereby reducing risk of host versus graft rejection (HVGR). In some regimens, multiple populations of cells can be combined and co-infused as the off the shelf T cell therapies.

## Description

### CROSS-REFERENCE TO RELATED APPLICATIONS

The present application claims the benefit of priority to U.S. provisional application 63/521,021 (filed 14 June 2023), the entire contents of which are incorporated herein by reference.

### FIELD OF THE INVENTION

The present disclosure relates to allogeneic chimeric antigen receptor T cell immunotherapy to treat cancer.

### BACKGROUND OF THE INVENTION

### The physiological immune response

The human immune system is a complex arrangement of cells and molecules that maintain immune homeostasis to preserve the integrity of the organism by elimination of all elements judged to be dangerous. Responses in the immune system may generally be divided into two arms, referred to as "innate immunity" and "adaptive immunity." The two arms of immunity do not operate independently of each other, but rather work together to elicit effective immune responses.

Innate immunity is a nonspecific fast response to pathogens that is predominantly responsible for an initial inflammatory response via a number of soluble factors, including the complement system and the chemokine/cytokine system, and a number of specialized cell types, including mast cells, macrophages, dendritic cells (DCs), and natural killer cells (NKs). The successful activation of innate immunity informs the development of the subsequent adaptive immune response.

Adaptive immunity involves a specific, delayed and longer-lasting response by various types of cells that create long-term immunological memory against a specific antigen. It can be further subdivided into cellular and humoral branches, the former largely mediated by T cells and the latter by B cells. Adaptive immunity further encompasses cell lineage members of the adaptive arm that have effector functions in the innate arm, thereby bridging the gap between the innate and adaptive immune response.

Generally speaking, immune responses are initiated by an encounter between an individual and a foreign substance, e.g., an infectious microorganism. The infected individual rapidly responds with both a humoral immune response with the production of antibody molecules specific for the antigenic determinants/epitopes of the immunogen, and a cell mediated immune response with the expansion and differentiation of antigen-specific regulatory and effector T-lymphocytes, including cells that produce cytokines and killer T cells, capable of lysing infected cells. Primary immunization with a given microorganism evokes antibodies and T cells that are specific for the antigenic determinants/epitopes found on that microorganism; these usually fail to recognize or recognize only poorly antigenic determinants expressed by unrelated microbes [Paul, W. E., "Chapter 1: The immune system: an introduction," Fundamental Immunology, 4th Edition, Ed. Paul, W. E., Lippincott-Raven Publishers, Philadelphia, (1999), at p. 102].

As a consequence of this initial response, the immunized individual develops a state of immunologic memory. If the same or a closely related microorganism is encountered again, a secondary response ensues. This secondary response generally consists of an antibody response that is more rapid, greater in magnitude and composed of antibodies that bind to the antigen with greater affinity and that are more effective in clearing the microbe from the body, and a similarly enhanced and often more effective T-cell response. However, immune responses against infectious agents do not always lead to elimination of the pathogen [Paul, W. E., "Chapter 1: The immune system: an introduction," Fundamental Immunology, 4th Edition, Ed. Paul, W. E., Lippincott-Raven Publishers, Philadelphia, (1999), at p. 102].

### Innate lymphoid cells (ILCs)

Innate lymphoid cells (ILCs) are the innate counterparts of T lymphocytes. They lack adaptive antigen receptors generated by the recombination of genetic elements. [Vivier, E. et al. Cell (2018) 174: 1054-66, citing Spits, et al. Nat. Rev. Immunol. (2013) 13: 145-49; Eberl, G et al. Science (2015) 348: aaa6566; Artis, D. and Spits, H. Nature (2015) 517: 293-301]. All ILCs express interleukin-7 receptor-α (IL-7Rα; CD127). ILCs may be activated by signals from other cells around them upon exposure to foreign antigens (including microbes), rather than by being directly activated by foreign antigens. Some ILCs express toll-like receptors (TLRs) that recognize microbes, and the cells may be directly activated by the PAMPs of microbes. However, there have been some reports showing that ILCs express various kinds of receptors for cytokines, danger signals, neuropeptides and lipid mediators that are more dominant than TLRs. [Id.]

ILCs are generally thought to be tissue-resident cells that differentiate into mature effector cells in tissues, and show minimal movement between organs. Instead, they have functional plasticity that enables them to respond promptly to microenvironmental changes, thereby precluding any need for differentiation and/or migration of new ILC subsets adapted to a new environment. For example, transdifferentiation has been shown between ILC1s and ILC3s [Id., citing Bernink JH, et al. Immunity (2015) 43:146-160, Bernink JH, et al. Nat Immunol (2013) 14:221-229], between ILCIs and ILC2 [Id., citing Bal SM, et al. Nat Immunol (2016) 17:636-645; Silver JS, et al. Nat Immunol 2016;17:626-635; Ohne Y, et al. Nat Immunol (2016) 17:646-655], and between ILC2s and ILC3s [Id. citing Bernink JH, et al. Nat Immunol (2019) 20:992-1003, Golebski K, et al. Nat Commun (2019) 10:2162].

Group 1 ILCs currently are divided into 3 different subtypes, according to their expression of cytokines and transcription factors: group 1 ILCs (ILC1s), group 2 ILCs (ILC2s), and group 3 ILCs (ILC3s).

ILCIs are defined as ILCs that express T box-expressed in T cells (T-bet) and produce interferon (IFN-γ); they include conventional natural killer cells (cNK) and are considered to be involved in anti-viral immunity, like T_{H}1 cells. [Orimo, K. et al., Allergy Asthma Immunol. Res. (2020) 19(3): 381-98].

Natural killer (NK) cells have an ability to kill tumor cells without any priming or prior activation, in contrast to cytotoxic T cells, which need priming by antigen presenting cells. NK cells secrete cytokines such as IFNγ and TNFα, which act on other immune cells, like macrophages and dendritic cells, to enhance the immune response. Activating receptors on the NK cell surface recognize molecules expressed on the surface of cancer cells and infected cells and switch on the NK cell, while inhibitory receptors act as a check on NK cell killing. Most normal healthy cells express MHC class I receptors, which mark them as "self." Inhibitory receptors on the surface of the NK cell recognize cognate MHC-I, which switches off the NK cell, preventing it from killing. Once the decision is made to kill, the NK cell releases cytotoxic granules containing perform and granzymes, which leads to lysis of the target cell. Natural killer reactivity, including cytokine secretion and cytotoxicity, is controlled by a balance of several germ-line encoded inhibitory and activating receptors such as killer immunoglobulin-like receptors (KIRs) and natural cytotoxicity receptors (NCRs). The presence of the MHC Class I molecule on target cells serves as one such inhibitory ligand for MHC Class I-specific receptors, the Killer cell Immunoglobulin-like Receptor (KIR), on NK cells. Engagement of KIR receptors blocks NK activation and, paradoxically, preserves their ability to respond to successive encounters by triggering inactivating signals. Therefore, if a KIR is able to sufficiently bind to MHC Class I, this engagement may override the signal for killing and allows the target cell to live. In contrast, if the NK cell is unable to sufficiently bind to MHC Class I on the target cell, killing of the target cell may proceed. Consequently, those tumors which express low MHC Class I and which are thought to be capable of evading a T-cell-mediated attack may be susceptible to an NK cell-mediated immune response instead.

ICL2s are defined as ILCs that express GATA-binding protein 3 and produce such cytokines as IL-4, IL-5, IL-9, and IL-13, as well as the epidermal growth factor, amphiregulin; like T_{H}2 cells, they are considered to be involved in anti-helminth immunity. [Id.]

ILC3s are defined as ILCs that express retinoic acid receptor-related orphan receptor-γt and produce cytokines, such as IL-17A, IL-22 and GM-CSF; they include both natural cytotoxicity receptor (NCR)- ILC3s and NCR+ ILC3s, and are considered to be involved in antibacterial immunity, like T_{H}17 cells. [Id.]

In humans, ILC3s are the predominant population in mucosal tissues, including the lung and gut, whereas the proportion of ILC2s is a little higher in the skin compared to mucosal tissues [Id., citing Bal SM, et al. Nat Immunol (2016) 17:636-645). The proportion of the ILC subsets is influenced by age; although ILC3s are the predominant population in the fetal human lung, their proportion decreases while the proportions of ILC1s and ILC2s increase with age in the adult human lung. [Id., citing Bal SM, et al. Nat Immunol (2016) 17:636-645]. There is substantial heterogeneity in each subset of ILCs. Moreover ILCs show different phenotypes depending on the organ. (Id., citing Ricardo-Gonzalez RR, et al. (2018) 19:1093-1099). For example, although ILC2s from different organs share canonical markers such as GATA3 and IL-7R, expression of IL-33R, IL-25R, and IL-18R1 differs depending on the organ. [Id., citing Ricardo-Gonzalez RR, et al. Nat Immunol (2018) 19:1093-1099].

Another ILC subset, called regulatory ILCs (ILCregs), which resemble regulatory T cells (Tregs) and have regulatory functions, has been reported. [Id., citing Morita H, et al. Allergy Clin Immunol (2019) 143:2190-2201.e9; Wang S, et al. Cell (2017) 171:201-216.e18; Seehus CR, et al. Nat Commun (2017) 8:1900]. ILCregs produce regulatory cytokines such as IL-10 and/or TGFβ, but they do not express FOXP3, the canonical transcription factor of Tregs. It remains controversial wither ILCregs represents an independent effector subset, or just a temporary state of ILCs.

There is increasing evidence to suggest that like T helper cell subsets, ILC subsets also display a certain degree of plasticity, which enables them to adjust to their microenvironment. Thus, ILC subsets can change their phenotype and functional capacities. For example, although ILC2s from different organs share canonical markers such as GATA3 and IL-7R, expression of IL-33R, IL-25R, and IL-18R1 differs depending on the organ. [Id., citing Ricardo-Gonzalez RR, et al. Nat Immunol (2018) 19:1093-1099]. This requires accessible polarizing signals in the tissue in which conversion occurs, together with the expression of cognate cytokine receptors and key transcription factors in the responding ILCs. [Vivier, E. et al. Cell (2018) 174: 1054-66].

### Dendritic cells

Dendritic cells are specialized antigen-presenting cells (APCs) that represent the interface between innate and adaptive immunity; they are able to present endogenous and exogenous antigens to T cells in the context of MHC molecules. Four different lineages can be classified as DCs: classical DCs (cDCs) [Briseno, CG et al. Curr Opin. Immunol. (2014) 29: 69-781], citing Steinman, RM and Cohn, ZA. J. Ex. Med. (1973) 137: 1142-62]: plasmacytoid DCs (pDCs),[Id., citing Siegal, FP et al. Science (1999) 284L 1835037; Cella, M. et al. Nature Medicine (1999) 5: 919-23], monocyte derived DCs (moDCs) [Randolph, GJ et al. Immunity (1999) 11: 753-61; Serbina, NV et al. Immunity (2003) 19: 59-70; Geissmann, F. et al. Immunity (2003) 19: 71-82] and Langerhans cells [Id., citing Schuler, G. and Steinman, RM. J. Exp. Med. (1985) 161: 526-46].

### Immunological synapses and immune cell activation

Immune responses are initiated by the interaction between APCs, such as DCs, with responder cells, such as T cells, via a tight cellular contact interface called the immunological synapse. The immunological synapse is a highly organized subcellular structure that provides a platform for the presentation of antigen in major histocompatibility class I and II complexes MHC class I and II) on the surface of the APC to receptors on the surface of the responder cells. In T cells, these contacts lead to highly polarized membrane trafficking that results in the local release of lytic granules and in the delivery and recycling of T cell receptors at the immunological synapse. Localized trafficking also occurs at the APC side of the synapse, especially in DCs where antigen loaded in MHC class I and II is presented and cytokines are released specifically at the synapse. A functional immunological synapse between DCs and naive T cells is essential to mount functional T cell responses. [Vergoogen, DRJ et al. Biomol Concepts (2016) 7(1): 17-28].

Not only DCs and T cells, but also other APCs, such as B cells or infected cells, and other effector cells, such as NKs, form immunological synapses for intercellular communication as well as for the killing of infected target cells. [Id., citing Angus, KL and Griffiths, GM. Curr. Opin. Cell Biol. (2013) 25: 85-91; Friedl, P. et al. Nat. Rev. Immunol. (2005) 5: 53; Xie, J. et al. Immunol. Rev. (2013) 251: 65-79]. The structure of the synapse strongly depends on the cell types involved, the presence and strength of antigen recognition and additional co-stimulatory interactions. [Id., citing Friedl, P. et al. Nat. Rev. Immunol. (2005) 5: 53; Thauland, TJ and Parker, DC. Immunology (2010) 131: 466-72; Azar, GA et al. Proc. Natl. Acad. Sci. USA (2010) 107: 3675-80].

Immunological synapses can functionally be divided into two categories [Id., citing Gerard, A. et al. Immunol. Rev. (2013) 251: 80-96]: (1) primary synapses, which are the cell-cell contacts that result in initial activation of immune cells, such as the synapses between DCs and T cells [Id., citing Rodriguez-Fernandez, JL et al. Sci. Signal (2010) 3: re2], and (2) so-called secondary synapses that result from interactions established after initial priming, such as activated T cells delivering stimulatory signals via, for example, CD40-CD40L interactions to B cells [Id., citing Chaplin, DD. J. Allergy Clin. Immunol.

(2010) 125: S3-23]; this category also encompasses the synapses formed between NKs or cytotoxic T cells with their target APC where lytic granules are released to kill the APC (viral infected or mutated cancer cell) [Id., citing Stinchcombe, JC et al. Immunity (2001) 15: 751-61]. For both categories, the formation of immunological synapses can trigger intracellular signaling cascades in both the APC and the T cell that lead to reorganization of the cytoskeleton and rerouting of membrane trafficking.

### B Cells

***B Cell Activation.*** A mature B cell can be activated by an encounter with an antigen that expresses epitopes that are recognized by its cell surface immunoglobulin (Ig). The activation process may be a direct one, dependent on cross-linkage of membrane Ig molecules by the antigen (cross-linkage-dependent B cell activation) or an indirect one, occurring most efficiently in the context of an intimate interaction with a helper T cell ("cognate help process"). The soluble product of an activated B lymphocyte is immunoglobulins (antibodies).

Upon activation in the germinal centers in lymphoid organs, B cells expressing high-affinity antibodies differentiate into antibody-secreting plasma cells and memory B cells that mediate humoral immunity against pathogens [Gonzalez, H. et al. Genes & Development (2018) 32: 1267-84, citing De Silva NS, Klein U. (2015) Nat Rev Immunol 15: 137-148]. Although the presence of B cells in the tumor microenvironment (TME) has been described in different carcinomas (including melanoma and breast, ovarian, and prostate cancer, among others) [Id., citing Chin, Y. et al. (1992) Anticancer Res 12: 1463-1466; Yang, C. et al. (2013) PLoS One 8: e54029; Woo, JR et al. (2014) J Transl Med 12: 30; Pylayeva-Gupta, Y. et al. (2016) Cancer Discov 6: 247-255], the role of B cells in cancer progression is much less understood than that of T cells. Accumulating evidence indicates that B cells promote and support tumor growth; for example, using a transgenic mouse model of epithelial carcinogenesis, Coussens and colleagues [Id., citing de Visser, KE et al. (2005) Cancer Cell 7: 411-423] demonstrated that the lack of mature B cells decreases tumor progression. The adoptive transfer of B cells restores chronic inflammation, angiogenesis, and tumor growth. Different mechanisms have been described to explain the pro-tumor role of B cells, from immunosuppression via secretion of IL-10 [Id., citing Schioppa, T. et al. (2011) Proc Natl Acad Sci 108: 10662-10667and TGFβ [Id., citing Olkhanud, PB et al. (2011) Cancer Res 71: 3505-3515] to direct stimulation of tumor cell proliferation by B-cell-derived IL-35 in human pancreatic neoplasia and Kras-driven pancreatic neoplasms in mice [Id., citing Pylayeva-Gupta, Y. et al. (2016) Cancer Discov 6: 247-255]. Also, by deposition of immunoglobulins in the TME, B cells indirectly stimulate angiogenesis and chronic inflammation by activating myeloid cells via FcRy, a widely expressed adaptor bearing an immunoreceptor tyrosine-based activation motif (ITAM) that transduces activation signals from various immunoreceptors [Andreu, P. et al. (2010) Cancer Cell 17: 121-134; Hida, S. et al. Nat. Immunol. (2009) 10 (2): 214-22].

### Memory B cells.

Memory B cells are a critical reservoir for plasma cell generation in the secondary response. They are heterogeneous in isotype usage, immunoglobulin mutational content, and phenotypic marker expression. Phenotypic subsets of memory B cells are defined by PD-L2, CD80, and CD73 expression in mice, by CD27 and Fc receptor-like protein 4 (FCRL4), an immunoregulatory receptor that identifies a pro-inflammatory B cell subset expressed on human memory B cells, and by T-bet in both mice and humans. These subsets display marked functional heterogeneity, including the ability to rapidly differentiate into plasma cells versus seed germinal centers in the secondary response. Memory B cells are located in the spleen, blood, other lymphoid organs, and barrier tissues; evidence indicates that some memory B cells may be dedicated tissue-resident populations. [Cancro, MP and Tomayko, MM. Immunological Reviews (2021) 303 (1): 72-82].

### T Cells

T cells are components of the adaptive immune system that act as orchestrators and effectors of immunity. Depending on the immunological context, T cells can acquire functional and effector phenotypes whose activity has direct inflammatory or anti-inflammatory consequences [Gonzalez, H. et al. Genes & Development (2018) 32: 1267-84, citing Speiser, DE et al. (2016) Nat Rev Immunol 16: 599-611]. As the second most frequent immune cell type found in human tumors besides tumor associated macrophages TAMs), T cells are extensively studied in diverse cancer types [Id., citing Speiser, DE et al. (2016) Nat Rev Immunol 16: 599-611; Donadon, M. et al. (2017) J Gastrointest Surg 21: 1226-1236]. During the early stages of tumor initiation, if enough immunogenic antigens are produced, naive T cells will be primed in the draining lymph nodes, followed by their concomitant activation and migration to the tumor microenvironment (TME). From there, they mount a protective effector immune response, eliminating immunogenic cancer cells. Histopathological analyses of human tumors show that tumor-associated T cells extend beyond the invasive edge of the tumor and also predominate in its hypoxic core [Id., citing Halama, N. et al. (2011) Cancer Res 71: 5670-5677 ; Kirilovsky, A. et al. (2016) Int Immunol 28: 373-382]. A high level of T-cell infiltration in tumors is associated with a favorable prognosis in melanoma [Id., citing Clemente, CG et al. (1996) Cancer 77: 1303-1310] and breast [Id., citing Oldford, SA et al. (2006) Int Immunol 18: 1591-1602], lung [Id., citing Dieu-Nosjean, MC et al. (2008) J Clin Oncol 26: 4410-4417], ovarian [Id., citing Kusuda, T. et al. (2005) Oncol Rep 13: 1153-1158], colorectal [Id., citing Tosolini, M. et al. (2011) Cancer Res 71: 1263-1271, renal [Id., citing Kondo, T. et al. (2006) Cancer Sci 97: 780-786, prostate [Id., citing Vesalainen, S. et al. (1994) Eur J Cancer 30A: 1797-1803, and gastric [Id., citing Ubukata, H. et al. (2010) J Surg Oncol 102: 742-747; Fridman, WH et al. (2012) Nat Rev Cancer 12: 298-306; Kitamura, T. et al. (2015) Nat Rev Immunol 15: 73-86] cancer.

***T Cell Activation.*** T-cell activation is dependent on the interaction of the T cell receptor (TCR)/CD3 complex with its cognate ligand, a peptide bound in the groove of a class I or class II MHC molecule. Full responsiveness of a T cell requires, in addition to receptor engagement, an accessory cell-delivered costimulatory activity, e.g., engagement of CD28 on the T cell by CD80 and/or CD86 on an antigen presenting cell (APC). The soluble product of an activated T lymphocyte is lymphokines (meaning cytokines produced by lymphocytes).

Naive T cells must initially be activated by dendritic cells. [Yewdell, JW and BP Dolan. Nature (2011) 471 (73340): 581-82]. During T cell priming, the clonal selection of antigen-specific naive T lymphocytes is initiated by the engagement of the TCR by its cognate ligand on the surface of antigen-presenting mature dendritic cells. Engagement of the TCR occurs in secondary lymphoid organs during a physical interaction between the naive T cells and DCs. The stability of these contacts determined the duration of the engagement of the TCR and thereby the intensity of TCR signaling. In naive T lymphocytes, signaling through the TCR occurs during the interaction with DCs presenting the cognate TCR ligand at their surface. The genetic ablation of ICAM-1 prevents the establishment of long-lasting interactions and fails to induce effective memory. Stable DC-T cell interactions are antigen dependent, influenced by chemokines [Scholer, A. et al. Al. Immunity (2008) 28: 258-70, citing Molon, B. et al (2005) Nature Immunol. (2005) 6: 465-71] and require an active actin DC cytoskeleton [Id., citing Benvenuti, F. Science (2004) 305: 1150-3]. In addition, expression of Intercellular Adhesion Molecule-1 (ICAM-1) by mature DCs is required to establish long-lasting DC-T cell contacts. [Id.] T cell priming in the absence of ICAM-1 expression resulted in normal activation, proliferation, and effector cytotoxic T lymphocyte (CTL) generation, but the effector CD8+ T cells produced low amounts of IFN-gamma. CD8+ T cells then were deleted, and the mice became unresponsive to antigenic rechallenge. Furthermore, in ICAM^{-/-} mice, DC-T cell contacts were not stabilized. The authors conclude that ICAM-1 dependent long-lasting DC-T cell interactions during priming are required for the survival of activated CD8+ T cells and the establishment of effective memory [Id.]. Whitmire, JK et al. [J. Immunol. (2007) 179: 1190-7] reported that direct IFN-gamma signaling enhances the development of CD8+ T cell memory, suggesting that impaired CD8+ T cell immunity in ICAM ^{-/-} mice may be due to reduced IFN-gamma production by CD8+ T cells during the primary response.

### The TCR-peptide loaded MHC (pMHC) interaction.

The T cell repertoire is shaped by positive selection, which requires recognition by the TCR of complexes of self peptide-MHC with low affinity, and negative selection, which eliminates T cells with TCRs that recognize self-pMHC with high affinity, mediated by thymocytes in the thymus. This generate a repertoire with low affinity for self-pMHC but high affinity for foreign antigens. The TCR must engage both of these ligands for development, homeostasis and immune responses. [Morris, GP and Allen, PM. Nature Immunol. (2012) doi: 10.1038/ni.2190]. The total human naive TCR repertoire is about 2.5 × 10*7 different TCRs [Kessels, HWHG, et al. Proc Natl Acad Sci. USA (2000) 14578-83].

TCR recognition of peptide loaded MHC is unparalleled in the diversity of the interacting surfaces. All components of the interaction are inherently extremely diverse: generation of the TCR by somatic recombination allows a theoretical diversity of about 10*15-18 different TCRs, of which about 1.5 × 10*7 can be found within any individual [Zareie, P. et al. Viral Immunology 33 (3): 179-87 (2020), citing Davis, MM and Bjorkman, PJ. Nature (1988) 334: 395-402]; the MHC genes are the most polymorphic genes of the human genome, and the peptide cargo that can be bound by MHC is virtually limitless.

Despite this diversity, key structural characteristics have emerged, which are highly conserved across virtually all interactions. The TCR makes contact with both the peptide cargo and the MHC (co-recognition); the TCR binds over the top of the peptide, the peptide is contacted by at least one of the highly variable TCR CDR3 loops, and the TCR binds the peptide MHC (pMHC) with a highly conserved polarity. The TCR α chain sits over the top of the MHCI α2 helix or MHCIIβ chain, while the TCR β chain is positioned over the MHCI α helix or MHCII α chain. [Id., citing Rossjohn, J. et al. Annu. Rev. Immunol. (2015) 33: 169-200; Rudolph, MG et al. Annu. Rev. Immunol. (2006) 24: 419-66]. The major determinants of peptide specificity are the CDR3 loops of the TCRα and β chain, with the CDR3 of the TCRα chain primarily in contact with the N-terminal part of the MHC peptide, and the TCRβ CDR3 mainly interacting with the C-terminal part. [Kessels, HWG et al. Proc. Natl Acad. Sci. USA (2000) 97 (26): 14578-83].

In order to understand MHC restriction, there are two models under debate that address why T cells only see antigens presented by MHC molecules, the germline model and the selection model. The germline model proposes that germline TCR sequences have been selected during evolution to encode a TCR structure that efficiently interacts with MHC molecules. The opposing hypothesis comes from the selection model. Selection theories to explain T cell receptor bias for MHC propose that MHC restriction is driven ultimately by constraints imposed on the TCR during positive selection in the thymus and the nature of TCR signaling. [Zareie, P. et al. Viral Immunology 33 (3): 179-87 (2020), citing Rangarajan, S. and mariuzza, RA. Cell Mol. Life Sci. (2014) 71: 3059-68; Van Laethem, F. et al. Trends Immunol. (2012) 33: 437-41]. TCRs themselves do not possess intrinsic signaling capacity. Instead, TCR signaling relies on the delivery of the tyrosine kinase Lck to the CD3 complex when associated with the cytoplasmic tails of CD4 or CD8 co-receptors [Id., citing Turner, JM et al. Cell (1990) 60: 755-65]. Thus, the CD4 and CD8 co-receptors act to focus the TCR onto the MHC molecule. This requirement for co-receptor facilitation of signaling prevents development of T cells expressing TCRs that are not specific for MHC, ensuring MHC restriction.

***CD4+ T cells.*** CD4+ T cells play a central role in orchestrating host immune responses against cancer and infectious diseases as well as in autoimmunity [Wang, R-F. Trends in Immunol. (2001) 22 (50): 269-76, citing Paradoll, DM and Topalian, SL. Curr. Opin. Immunol. (1998) 10: 588-94; O'Garra, A. et al. (1997) Curr. Opin. Immunol. 9: 872-83; Kalams, SA and Walker, BD. J. Exp. Med. (1998) 188: 2199-2204; Zajac, AJ et al. Curr. Opin. Immunol. (1998) 10: 444-49].

When interacting with CD4+ T cells, DCs may induce their differentiation into different T helper (T_{H}) subsets [Patente, TA, et al., Frontiers Immunol. (2019) doi.org/10.3389/fimmu.2018.03176., citing Iwasaki A, Medzhitov R. Nat Immunol. (2015) 16:343-353] such as T_{H}1 [Amsen D, et al. Cell (2004) 117:515-26; Constant S, et al. J Exp Med (1995) 182:1591-6; Hosken NA, et al. J Exp Med. (1995) 182:1579-84; Kadowaki N. Allergol Int. (2007) 56:193-9; Maekawa Y, et al. Immunity (2003) 19:549-59; Pulendran B, et al. Proc Natl Acad Sci USA. (1999) 96:1036-41, T_{H}2 [Id., citing Constant S, et al. J Exp Med (1995) 182:1591-6, Hosken NA, et al. J Exp Med. (1995) 182:1579-84, Jenkins SJ, P. et al. J Immunol. (2007) 179:3515-23, Soumelis V, et al. Nat Immunol. (2002) 3:673-680], T_{H}17 [Id., citing Bailey SL, Nat Immunol. (2007) 8:172-80; Iezzi G, et al. Proc Natl Acad Sci USA. (2009) 106:876-81; Huang G, et al. Cell Mol Immunol. (2012) 9:287-95], or other CD4+ T cell subtypes [Id., citing Levings MK, et al. Blood (2005) 105:1162-9]. T cell differentiation in each subtype is a complex phenomenon that can be influenced by the cytokines in the DC tissue of origin [Id., citing Rescigno M. Immunol Rev. (2014) 260:118-28], their maturation state [Id., citing Reis e Sousa C. Nature Rev Immunol. (2006) 6:476-83] and cause of tissue imbalance [Id., citing Vega-Ramos J, et al. Curr Opin Pharmacol. (2014) 17:64-70].

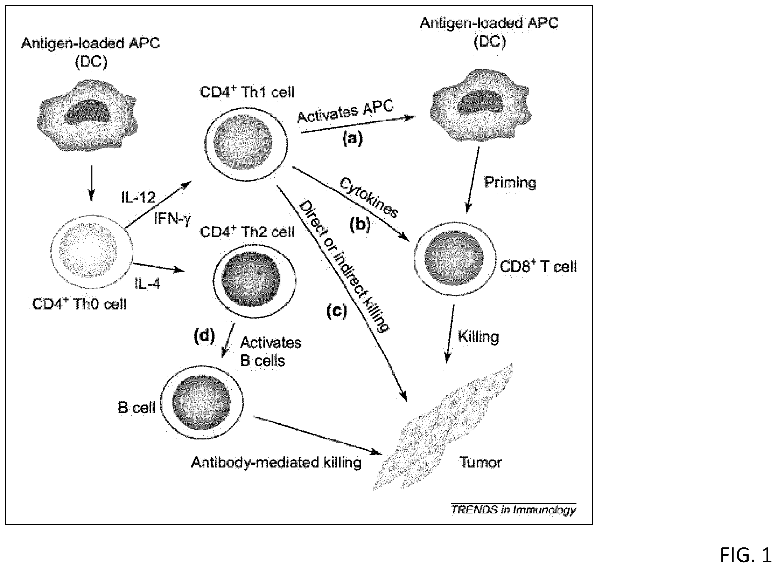

As shown in **FIG. 1****,** CD4+ T cells can be divided into Thelper 1 (T_{H}1) and Thelper 2 (T_{H}2) cells based on their cytokine secretion profile. [Wang, R-F. Trends in Immunol. (2001) 22 (50): 269-76, citing Morel, PA and Criss, TP. Crit. Rev. Immunol. (1998) 18: 275-303]. T_{H}2 cells activate B cells to become antibody-secreting plasma cells. CD4+ T_{H}1 cells help prime CD8+ T cell responses [Id., citing Toes, RE et al. J. Exp. Med. (1999) 189: 753-6]. Several studies have demonstrated that CD40-CD40 ligand (CD40L) interactions between DCs and CD4+ T cells activate DCs for effective priming and activation of CD8+ T cells. [Id., citing Schoenberger, SP et al. Nature (1998) 393: 473-4; Bennett, SR et al. Nature (1998) 393: 478-80; Ridge, JP et al. Nature (1998) 393: 474-78]. CD4+ T cells recognize an antigen presented by professional APCs, such as DCs, and, in turn activate antigen-bearing DCs [Id., citing Banchereau, J. and Steinman, RM. Nature (1998) 392: 245-52]. Once activated, DCs become competent to prime cytotoxic lymphocytes (CTLs) that recognize an MHC class I-restricted determinant on the same APC. Thus, activation of APCs by CD4+ T cells through antigen-specific recognition and CD40-CD40L engagement is essential to prime CD8+ T cells. [Id., citing Schoenberger, SP et al. Nature (1998) 393: 473-4; Bennett, SR et al. Nature (1998) 393: 478-80; Ridge, JP et al. Nature (1998) 393: 474-78]. In addition, CD4+ T cells are essential in the maintenance of CD8+ T cell effector functions by secreting cytokines such as IL-2 required for CD8+ T cell growth and proliferation. [Id., citing Rosenberg, SA et al. I Nature Med. (1998) 4: 321-7; Greenberg, PD. Adv. Immunol. (1991) 49: 281-355]

Most tumors express MHC class I, but not MHC class II molecules. CD4+ T cells recognize peptides bound to MHC class II molecules on the cell surface of APCs. The formation of MHC class II peptide complexes on the cell surface is a complicated multistep process that favors presentation of antigens derived from exogenous proteins.

MHC class II antigen processing and presentation requires at least five genes - DRα, DRβ, Ii, DMA and DMB- and the specialized MIIC compartments.

Assembly of the MHC class II α and β chains, along with the associated invariant chain (Ii), begins in the endoplasmic reticulum (ER). [Id., citing Germain, RN. Cell (1994) 76: 287-99; Cresswell, P. Cell (1996) 84: 505-7]. Ii association prevents an antigenic peptide from binding to the αβ dimers and stabilizes the αβ complexes. Ii contains an endosome-targeting sequence at the N-terminus, and a class II-associated invariant-chain peptide (CLIP) between amino acids 81 and 104. This targeting sequence in the cytoplasmic tail of Ii is responsible for the transport of nonameric (αβIi)₃ complexes from the ER to intracellular compartments with endosomal/lysosomal characteristics and, ultimately, to acidic endosomal and lysosomal-like structures called MHC class II compartments (MIIC) [Id., citing Germain, RN. MHC-dependent antigen processing and peptide presentation providing ligands for T lymphocyte activation. Cell (1994) 76: 287-99; Cresswell, P. Cell (1996) 84: 505-7]. HLA-DM molecules in this compartment facilitate dissociation of residual Ii peptide (CLIP) from the peptide-binding grooves of the mHC class II molecules and replacement with antigenic peptides. [Id., citing Cresswell, P. Cell (1996) 84: 505-7].

***CD8+ T cells.*** CD8+ T cells are the most prominent anti-tumor cells. Upon priming and activation by APCs, the CD8+ T cells differentiate into cytotoxic T lymphocytes (CTLs) and, through the exocytosis of perforin- and granzyme-containing granules, exert an efficient anti-tumoral attack, resulting in the direct destruction of target cells [Gonzalez, H. et al. Genes & Development (2018) 32: 1267-84, citing Hanson, HL et al. (2000) Immunity 13: 265-276; Matsushita, H. et al. (2012) Nature 482: 400-404]. Meanwhile, the CD4+ T helper 1 (T_{H}1)-mediated anti-tumoral response-through secretion of high amounts of proinflammatory cytokines such as IL-2, TNF-α, and IFN-γ-promotes not only T-cell priming and activation and CTL cytotoxicity but also the anti-tumoral activity of macrophages and NK cells and an overall increase in the presentation of tumor antigens [Id., citing Kalams SA, Walker BD. (1998) J Exp Med 188: 2199-2204; Pardoll DM, Topalian SL. (1998). Curr Opin Immunol 10: 588-594; Shankaran, V. et al. (2001) Nature 410: 1107-1111. The presence of tumor-infiltrating CD8+ T cells and T_{H}1 cytokines in tumors correlates with a favorable prognosis in terms of overall survival and a disease-free survival in many malignancies [Fridman, WH et al. (2012). Nat Rev Cancer 12: 298-306).

Preclinical investigations in patients and mouse models suggest that cancer cells exploit the immunosuppressive properties of T cells while impairing the effector functions of anti-tumor T cells, such as their ability to infiltrate tumors and their survival, proliferation, and cytotoxicity [Id., citing Grivennikov, SI et al. (2010) Cell 140: 883-899]. The antigen-dependent nature of the effector T cells implies that the effectiveness of the anti-tumor T-cell immune response depends on both the ability of the tumor antigen to induce an immune response and the presence-or absence-of inhibitory signals that can impair the T cells' functions [Id., citing Speiser, DE et al. (2016) Nat Rev Immunol 16: 599-611]. Accordingly, it is widely accepted that, in a T-cell-dependent process, most neoplastic cells expressing highly immunogenic antigens will be recognized and killed during the early stages of tumor development [Id., citing Matsushita, H. et al. (2012) Nature 482: 400-404]. The less immunogenic cancer cells escape the immune control of T cells and survive, a process termed cancer immune editing [Id., citing Teng, MW et al. (2015) J Clin Invest 125: 3338-3346]. The final outcome is that the surviving cancer cells adopt an immune-resistant phenotype. In parallel, during tumor development, cancer cells evolve mechanisms that mimic peripheral tolerance and are able to prevent the local cytotoxic response of effector T cells as well as those of other cells, such as tumor-associated macrophages (TAMs), NK cells, and tumor-associated neutrophils (TANs) [Id., citing Palucka AK, Coussens LM. (2016). Cell 164: 1233-47]. TANs are engaged into the tumor microenvironment by cytokines and chemokines, and can be distinguished according to their activation and cytokine status and effects on tumor cell growing in N1 and N2 TANs. N1 TANs exert an antitumor activity, by direct or indirect cytotoxicity. [Masucci, MT et al. Front Oncol. 9: 1146].

### Memory T cells

The vast majority of human memory T cells reside in tissue sites, including lymphoid tissues, intestines, lungs and skin. By the end of puberty, lymphoid tissues, mucosal sites and the skin are populated predominantly by memory T cells, which persist throughout adult life and represent the most abundant lymphocyte population throughout the body.

Memory T cells in humans are classically distinguished by the phenotype CD45RO+CD45RA-, and comprise heterogeneous populations of memory T cell subsets. [Farber, DL, et al. Nat. Rev. Immunol. (2014) 14(1): 24-35] Naive T cells uniformly express CCR7, reflecting their predominant residence in lymphoid tissue. Memory T cells are subdivided into CD45RA-CCR7+ central memory T (T_{CM}) cells, which traffic to lymphoid tissues, and CD45RA-CCR7- effector memory T (T_{EM}) cells, which can migrate to multiple peripheral tissue sites. Functionally, both T_{CM} and T_{EM} cell subsets produce effector cytokines in response to viruses, antigens and other stimuli [Id., citing Wang A, et al. Sci Transl Med. (2012) 4:149ra12030-33; Pedron B, et al. Pediatr Res. (2011) 69:106-111; Champagne P, et al. Nature. (2001) 410:106-111; Ellefsen K, et al. Eur J Immunol. (2002) 32:3756-3764], although T_{CM} cells exhibit a higher proliferative capacity. (Id. citing Wang A, et al. Sci Transl Med. (2012) 4:149ra120, Fearon DT, et al. Immunol Rev. 2006;211:104-118). T memory stem (T_{SCM}) cells, which resemble naive T cells in that they are CD45RA+CD45RO- and express high levels of the co-stimulatory receptors CD27 and CD28, IL-7 receptor α chain (IL7Rα), CD62L and CCR7, have high proliferative capacity and are both self-renewing and multipotent in that they can further differentiate into other subsets, including T_{CM} and T_{EM} cells [Id. citing Gattinoni L, et al. Nat Med. (2011) 17:1290-1297, Gattinoni L, et al. Clin Cancer Res. (2010) 16:4695-4701]. A progressive differentiation pathway based on signal strength and/or extent of activation, places naive (T_{N}), T_{SCM}, T_{CM} and T_{EM} cells in a differentiation hierarchy, serving as precursors for effector T cells [Id. citing Gattinoni L, et al. Nat Rev Cancer. (2012) 12:671-684; Klebanoff CA, et al. Immunol Rev. (2006) 211:214-224; Lanzavecchia A, Sallusto F. Nat Rev Immunol. (2002) 2:982-987].

In mice, tissue resident memory T (T_{RM}) cells are a non-circulating subset that resides in peripheral tissue sites and, in some cases, elicits rapid in situ protective responses. Mouse CD4+T_{RM} cells can be generated in the lungs from adoptive transfer or activated (effector) T cells [Id., citing Teijaro JR, et al. J Immunol. (2011) 187:5510-5514] or following respiratory virus infection [Id., citing Turner, DL, et al. Mucosal Immunol. (2014) 7 (3): 501-510], and are distinguished from splenic and circulating memory T cells by their upregulation of the early activation marker CD69, their tissue-specific retention in niches of the lung [Id., citing Turner, DL, et al. Mucosal Immunol. (2014) 7 (3): 501-510] and their enhanced ability to mediate protection to influenza virus infection compared to circulating memory CD4+ T cells [Id. citing Teijaro JR, et al. J Immunol. (2011) 187:5510-5514]. An analogous non-circulating CD4+ T_{RM} cell subset has been identified in the bone marrow of mice following systemic virus infection that exhibits enhanced helper functions. [Id., citing Hemdler-Brandstetter D, et al. J Immunol. (2011) 186:6965-6971]. CD8+ T_{RM} cells generated following infection have been identified in multiple mouse tissues, including skin [Id., citing Clark RA, et al. Sci Transl Med. (2012) 4:117ra117; Liu L, et al. Nat Med. (2010) 16:224-227], vaginal mucosa [Id., citing Mackay LK, et al. Proc Natl Acad Sci USA. (2012) 109:7037-7042, Shin H, Iwasaki A. Nature. (2012) 491:463-467], intestine [Id., citing Klonowski KD, et al. Immunity. (2004) 20:551-562, Masopust D, et al. J Exp Med. (2010) 207:553-564, Masopust D, et al. J Immunol. (2006) 176:2079-2083], lungs [Id., citing Turner, DL, et al. Mucosal Immunol. (2014) 7 (3): 501-510, Anderson KG, et al. J Immunol. (2012) 189:2702-2706] and brain [Id., citing Wakim LM, et al. Proc Natl Acad Sci USA. (2010) 107: 17872-17879]. They are distinguished from splenic and circulating memory CD8+ T cells by their increased expression of CD69 and by expression of the epithelial cell binding integrin αEβ7 (also known as CD103 [Id, citing Mueller SN, et al. Annu Rev Immunol. (2013) 31:137-161, Mackay LK, et al. Proc Natl Acad Sci USA. (2012) 109:7037-7042, Casey KA, et al. J Immunol. (2012) 188:4866-4875; Masopust D, Picker LJ. J Immunol. (2012) 188:5811-5817; Gebhardt T, Mackay LK. Front Immunol. (2012) 3:340].

In humans, memory CD4+ T cells predominate throughout the body and persist as CCR7+ or CCR7- subsets localized to lymphoid tissues and mucosal sites, respectively, whereas memory CD8+ T cells persist as mainly CCR7- subsets in all sites, with low numbers of CD8 T_{CM} cells in lymphoid tissues and negligible numbers of these cells in other sites [Id., citing Sathaliyawala T, et al. Immunity (2013) 38:187-197]. Most memory T cells in human mucosal, lymphoid and peripheral tissue sites such as skin express the putative T_{RM} cell marker CD69 [Id., citing Goronzy JJ, Weyand CM. Nat Immunol. (2013) 14:428-436; Nikolich-Zugich J, Rudd BD. Curr Opin Immunol. (2010) 22:535-540; Clark RA, et al. J Immunol. (2006) 176:4431-4439, Mueller SN, et al. Annu Rev Immunol. (2013) 31:137-161, Casey KA, et al. J Immunol. (2012) 188:4866-4875], whereas circulating blood memory T cells uniformly lack CD69 expression. [Id., citing Sathaliyawala T, et al. Immunity (2013) 38:187-197].

Human T_{RM} cells also exhibit tissue-specific properties, suggesting *in situ* influences. For example, memory T cells in the small intestine and colon express the gut-homing receptor CCR9 [Id., citing Kunkel EJ, et al. J Exp Med. (2000) 192:761-768] and the integrin α4β7 [Id., citing Agace WW. Trends Immunol. (2008) 29:514-522], and memory T cells in the lungs upregulate CCR6 expression [Id., citing Purwar R, et al. PLoS One. (2011) 6:e16245]. There is also evidence for crosstalk between mucosal sites, such as lung and intestines. For example lung dendritic cells induce migration of protective T cells to the gastrointestinal tract. [Id. citing Ruane D, et al. J Exp Med. (2013) 210:1871-1888].

There is evidence that T_{RM} can be multifunctional and also exhibit qualitative functional differences. A substantial fraction of human lung T_{RM} cells produce multiple pro-inflammatory cytokines [Id., citing Purwar R, et al. PLoS One. 2011;6:e16245], and human intestinal T_{RM} cells are also multifunctional [Id. citing Sathaliyawala T, et al. Immunity. 2013;38:187-197]. Other functions appear to be confined to specific subsets and/or tissue sites. For example, IL-17 is produced by a subset of CD4+ T_{RM} cells in mucosal sites, particularly in intestines in healthy individuals [Id., citing Sathaliyawala T, et al. Immunity (2013) 38:187-197], by CCR6+ memory T cells in peripheral blood [Id., citing Singh SP, et al. J Immunol. (2008) 180:214-221, Wan Q, et al. J Exp Med. (2011) 208: 1875-1887], and by a subset of CD161+ T cells in inflamed tissue, such as the skin of patients with psoriasis [Id., citing Cosmi L, et al. J Exp Med. 2008;205:1903-1916]. Thus, while predominant memory T cell functions, such as IFNγ production, are broadly distributed among multiple memory T cell subsets and tissues, T_{RM} cells in tissue sites can adopt multiple or distinct functional attributes, which may also depend on tissue-specific inflammation.

Despite their specificity, human memory T cells exhibit cross-reactivity to antigenic epitopes not previously encountered, which may be due to intrinsic properties of TCR recognition [Id., citing Sewell AK. Nat Rev Immunol. 2012;12:669-677] and to the range and breadth of human antigenic experience. Memory CD4+ and CD8+ T cells specific for unique epitopes of avian influenza strain H5N1 were detected in healthy individuals that were not exposed to H5N1 infection assessed by serology [Id., citing Lee LY, et al. J Clin Invest. (2008) 118 (10): 3478-90; Roti M, et al. J Immunol. (2008) 180:1758-1768]. In addition, HIV-specific memory T cells have been identified in HIV-negative individuals [Id., citing Su, LF et al. Immunity (2013) 38: 373-83]. Virus-specific memory T cells also show cross-reactivity to alloantigens, autoantigens and unrelated pathogens [Id., citing D'Orsogna LJ, et al. Transpl Immunol. (2010) 23:149-155, Wucherpfennig KW. Mol Immunol. (2004) 40:1009-1017]: EBV-specific human memory T cells generated in HLA-B8 individuals exhibit allogeneic cross-reactivity to HLA-B44 [Id., citing Burrows SR, et al. J Exp Med. (1994) 179:1155-1161], and influenza virus- and HIV-specific memory CD4+ T cells recognize epitopes from unrelated microbial pathogens [Id., citing Su LF, et al. Immunity. (2013) 38:373-383]. Furthermore, T cells specific for the autoantigen myelin basic protein (MBP) recognized multiple epitopes from viral and bacterial pathogens [Id., citing Wucherpfennig KW. Mol Immunol. (2004) 40:1009-1017, Wucherpfennig KW, Strominger JL. Cell. (1995) 80:695-705]. This cross-reactivity may enable memory T cells to mediate protection without initial disease - a phenomenon known as heterologous immunity [Id., citing Welsh RM, Selin LK. Nat Rev Immunol. (2002) 2:417-426]. Heterologous immunity has been demonstrated in humans where EBV infection expanded clones of influenza virus-specific T cells [Id., citing Clute SC, et al. J Clin Invest. 2005;115:3602-3612].

Analysis of human samples has revealed that influenza-specific T_{RM} can be found in substantial numbers in lung tissue, highlighting their role in natural infection. Despite expressing low levels of granzyme B and CD107a, these CD8+ T_{RM} had a diverse T cell receptor (TCR) repertoire, high proliferative capacities, and were polyfunctional [Muruganandah, V., et al. (2018). Front. Immunol., 9, 1574. doi: 10.3389/fimmu.2018.01574]. Influenza infection history suggests a greater level of protection against re-infections is likely due to the accumulation of CD8+ T_{RM} in the lungs. Furthermore, the natural immune response to influenza A virus infection in a rhesus monkey model demonstrated that a large portion of influenza-specific CD8+ T cells generated in the lungs were phenotypically confirmed as CD69+CD103+ T_{RM}. Unlike lung parenchymal T_{RM}, airway CD8+ T_{RM} are poorly cytolytic and participate in early viral replication control by producing a rapid and robust IFN-γ response. Bystander CD8+ T_{RM} may also take part in the early immune response to infection through antigen non-specific, NKG2D-mediated immunity. The generation of functional T_{RM} that protect against heterosubtypic influenza infection appear to be dependent on signals from CD4+ T cells. [Muruganandah, V., et al. (2018). Frontiers in Immunology, 9, 1574. doi:10.3389/fimmu.2018.01574].

According to the paradigm of a typical CD8+ T cell response to acute viruses, CD8+ T cells are effectors when an antigen is present and become memory when the antigen is eliminated. However, it has become apparent that in viral infections, a memory T cell population comprises multiple subtypes of cells, distributed in diverse anatomic compartments and possibly recirculating among them. The memory CD8+ T cell response to most viruses is diverse in phenotype and function and undergoes dynamic changes during its development and maintenance in vivo. This heterogeneity is related to the nature of the infecting virus, its cellular tropism, the anatomic location of the infection, and the location of the CD8+ T cells. In resolved acute infections, the presence of memory CD8+ T cells at the site of the original virus entry and replication is crucial for a rapid response to a secondary infection. In latent infections, the presence of memory CD8+ T cells at sites of virus persistence is important for immune surveillance of virus reactivation. [Racanelli, V. et al., Rev. Med. Virol. (2011) 21 (6): 347-357].

### The relationship between the immune system and cancer

A tumor originates from a normal cell that has undergone tumorigenic transformation. This transformed cell is the cell-of-origin for the tumor. Tumorigenesis consists of four stages [Bi, Q. J. Immunology Res. (2022) (2022) article 3128933, citing Balani, S. et al. Nature Communic. (2017) 8 (1): article 15422; Chaffer, CL and Weinberg, RA. Cancer Discovery (2015) 5 (10): 22-24; Loeb, LA and Harris, CC. Cancer Res. (2008) 68 (17): 6863-71]: (a) tumor initiation, the initial stage of tumorigenesis, is the stage in which normal cells undergo irreversible genetic alterations under the response of oncogenic factors, thus transforming into COOs with the possibility of malignant transformation; (b) tumor promotion is the period during which COOs clone selectively and transform into premalignant cells under the influence of protumor factors and other specific conditions; (c) malignant conversion is the stage in which premalignant cells start expressing malignant phenotypes; and (d) tumor progression is the final stage of tumorigenesis, in which premalignant cells develop into real tumor cells, obtain a series of new biological characteristics (including sustaining proliferative signaling, evading growth suppressors, resisting cell death, enabling replicative immortality, inducing or accessing vasculature, activating invasion and metastasis, deregulating cellular metabolism, avoiding immune destruction, and unlocking phenotypic plasticity, nonmutational epigenetic reprogramming, polymorphic microbiomes, and senescent cells) [Id., citing Hanahan , D. Cancer Discovery (2022) 12 (1): 31-46], and undergo more invasion and metastasis. These characteristics are the result of the superposition of various factors, particularly the tumor microenvironment (TME).

Tumors are edited by the immune system as they evolve and can escape rejection in many ways. In the elimination phase, when tumors arise in a tissue, a number of immune cells (e.g., NK, CD4, CD8, γδT cells) can recognize and destroy potential tumor cells. If elimination is not completely successful, what follows is an equilibrium phase, in which according to the cancer immunoediting hypothesis, tumor cells undergo changes or mutations that aid their survival as a result of the selection pressure imposed by the immune system so that a variety of different tumor cells develop. In the escape phase, tumor cells that have acquired the ability to elude the attentions of the immune system by either escaping the killing mechanism or recruiting regulatory cells to protect it spread unchallenged and become clinically detectable. [Janeway's Immunobiology, 9th Ed. Kenneth Murphy & Casey Weaver, Garland Science, New York, NY (2017), at pp 717].

Tumors can avoid stimulating an immune response or can evade it when it occurs by numerous mechanisms. For example, spontaneous tumors may initially lack mutations that produce new tumor-specific antigens that elicit T cell responses. Even when a tumor specific antigen is expressed and is taken up and presented by APCs, if co-stimulatory signals are absent the APC will tend to treat the tumor as self, rather than activating T cells. Some tumors lose the expression of a particular MHC class I molecule perhaps through immune selection by T cells specific for a peptide presented by that MHC class I molecule, such; tumors that have lost such antigens are no longer eliminated by the immune system, a process termed "antigenic modulation". Tumors also seem able to evade immune attack by creating a generally immunosuppressive microenvironment. In addition, many tumors make immunosuppressive cytokines, such as TGFβ, which tends to suppress the inflammatory T cell responses and cell mediated immunity needed to control tumor growth. TGFβ induces the development of inducible Tregs. Some tumors, such as melanoma, ovarian carcinoma, and B-cell lymphoma, produce the immunosuppressive cytokine IL-10, which can reduce dendritic cell activity and inhibit T cell activation. The microenvironment of some tumors also contain populations of myeloid-derived suppressor cells (MDSCs), which can inhibit T-cell activation within the tumor. In addition, some tumors express immune checkpoints, that directly inhibit immune responses. Tumors can produce enzymes that act to suppress local immune responses. For example, the enzyme indoleamine 2,3-dioxygenase (IDO) catabolizes tryptophan, an essential amino acid, in order to produce the immunosuppressive metabolite kynurenine. Tumor cells also can produce materials, such as, without limitation, collagen, that create a physical barrier around the tumor, preventing interaction with cells of the immune system. [Janeway's Immunobiology, 9th Ed. Kenneth Murphy & Casey Weaver, Garland Science, New York, NY (2017), at pp 718-19]. An established clinical tumor can be sustained by subpopulations of self-renewing cancer cells operationally termed cancer stem cells (CSCs) that can generate, intraclonally, both tumorigenic and non-tumorigenic cells. Rycaj, K. and Tang, DG, Cancer Res. (2015) 75 (19): 4003-11].

***Immune checkpoints.*** During immune homeostasis, a crucial mechanism of peripheral tolerance is the regulation of effector T-cell response via immune checkpoints on CTLs and activated CD4+ T cells to protect tissue from inflammatory damage. The two better described checkpoint molecules CTLA-4 and PD-1 act as negative regulators of T-cell function and have been associated with immune evasion in cancer [Gonzalez, H. et al. Genes & Development (2018) 32: 1267-84., citing Pardoll, DM (2012) Nat Rev Cancer 12: 252-264]. The involvement of CTLA-4 signaling in cancer has been described in melanoma ([Id., citing Bouwhuis, MG et al. (2010) Cancer Immunol Immunother 59: 303-312] and lung ([Id., citing Khaghanzadeh, N. et al. (2010) Cancer Genet Cytogenet 196: 171-174], breast ([Id., citing Erfani N. et al. Cancer Genet. Cytogenet. (2006) 165 (2): 114-20], gastric [Id., citing Hadinia, A. et al. (2007) J Gastroenterol Hepatol 22: 2283-2287], and colorectal ([Id., citing Hadinia, A. et al. (2007) J Gastroenterol Hepatol 22: 2283-2287; Dilmec, F. et al. (2008). Int J Immunogenet 35: 317-321] cancer. Furthermore, the engagement of PD1 with its coreceptor, PDL-1 (expressed by other immune cells, mesenchymal cells, vascular cells, and cancer cells), results in the down-regulation of T-cell activity, which inhibits their anti-tumor activities such as T-cell migration, proliferation, secretion of cytotoxic mediators, and restriction of cell killing ([Id., citing Topalian, SL et al. (2015) Cancer Cell 27: 450-461]. The use of immune checkpoint inhibitors such as anti-PD1 (e.g., lambrolizumab, pembrolizumab (KEYTRUDA^{®}) and nivolumab (OPDIVO^{®}), anti-PD-L1 (MPDL3280A, Atezolizumab, TECENTRIQ^{®}), and anti-CTLA4 (ipilimumab, YERVOY^{®}) has had success enhancing the effector anti-tumor response in different malignancies ([Id., citing Gotwals, P. et al. (2017). Nat Rev Cancer 17: 286-301], especially in melanoma and lung cancer ([Id., citing Hamid, O. et al. (2013) N Engl J Med 369: 134-144; Herbst, RS et al. (2014). Nature 515: 563-567; Topalian, SL et al. (2015) Cancer Cell 27: 450-461].

As the tumor grows and the TME changes, new antigens are produced, and the ability of the immune system to prime new repertoires of T cells and direct them toward the tumor changes, thus altering the efficacy of tumor containment. As the immune system functions to stall tumor growth, cancer cells and the TME simultaneously suppress anti-tumor function by engaging immune checkpoints and the recruitment of regulatory CD4+ T cells (Tregs). Tregs are responsible for suppressing the priming, activation, and cytotoxicity of other effector immune cells, such as T_{H}1 CD4 T cells, CTLs, macrophages, NK cells, and neutrophils ([Id., citing Ward-Hartstonge KA, Kemp RA. (2017). Clin Transl Immunology 6: e154]. The Treg-mediated immunosuppression is orchestrated by contact-dependent mechanisms such as the expression of PDL-1, LAG-3, CD39/73, CTLA4, or PD1, with the latter two even enhancing suppressive activity ([Id., citing Walker LS, Sansom DM. (2015) Trends Immunol 36: 63-70], and by contact-independent mechanisms, which involve the sequestration of IL-2 and production of immune-suppressive molecules such as IL-10, TGF-β, prostaglandin E2, adenosine, and galectin-1 [Id., citing Francisco, LM et al. (2009). J Exp Med 206: 3015-3029; Campbell, DJ (2015) Eur J Immunol 195: 2507-2513]. In squamous cell carcinoma, the inhibition of focal adhesion kinase (FAK)-a cell contact-independent mechanism-results in CCL5 secretion by cancer cells that induces the recruitment of Tregs to the tumor site, where they suppress the cytotoxic anti-tumor CD8+ T cells ([Id., citing Serrels, A. et al. (2015) Cell 163: 160-173]. In breast and lung adenocarcinoma, Tregs suppress T-cell activation and the anti-tumor immune response in tumor-associated tertiary structures. Specific Treg depletion results in tumor cell death and increased production of IFN-γ ([Id., citing Bos, PD et al. (2013) J Exp Med 210: 2435-2466; Joshi, NS et al. (2015) Immunity 43: 579-590]. Infiltration of Tregs in breast cancer was correlated with worse patient outcome [Id., citing Allaoui, R. et al. (2017) Cancer Biomark 20: 395-409].

In metastasis, CTLs exert an anti-metastatic effect in bone metastasis [Id., citing Bidwell, BN et al. (2012) Nat Med 18: 1224-1231], while prospective analyses of lung and breast cancer patients established an opposite correlation between the level of circulating cancer cells and T cells in peripheral blood [Id., citing Mego, M et al. (2016) Circulating tumor cells (CTC) are associated with defects in adaptive immunity in patients with inflammatory breast cancer. J Cancer 7: 1095-1104; Sun, WW et al. (2017) Onco Targets Ther 10: 2413-2424].

These data extend to clinical trials reporting the therapeutic efficacy of immune checkpoint inhibition in metastatic carcinomas [Id., citing Di Giacomo, AM et al. (2012) Lancet Oncol 13: 879-886; Queirolo, P. et al. (2014) J Neurooncol 118: 109-116; Motzer, RJ et al. (2015) N Engl J Med 373: 1803-1813; Furudate, S. et al. (2016) Case Rep Oncol 9: 644-649; Goldberg, SB et al. (2016) Lancet Oncol 17: 976-983; Pai-Scherf, L. et al. (2017) Oncologist 22: 1392-1399]. Checkpoint inhibitors are significantly effective in treating brain metastatic tumors from melanoma and lung cancer [Id., citing Queirolo, P. et al. (2014). J Neurooncol 118: 109-116; Goldberg, SB et al. (2016) Lancet Oncol 17: 976-983; Di Giacomo, AM et al. (2017) Cytokine Growth Factor Rev 36: 33-38]. Recent evidence suggests that the effectiveness of checkpoint inhibition in melanoma brain metastasis depends on extracranial disease and peripheral activation of CD8+ T cells [Id., citing Taggart, D. et al. (2018) Proc Natl Acad Sci 115: E1540-E1549]. On the other hand, a high level of circulating Tregs has been associated with a higher risk of metastasis in non-small lung carcinoma patients. [Id., citing Erfani, N. et al. (2012) Lung Cancer 77: 306-311]. Similar associations have been described in breast cancer [Id., citing Metelli, A. et al. (2016) Cancer Res 76: 7106-7117], colorectal carcinoma metastasis [Id., citing Wang, Q. et al. (2014) Cell Immunol 287: 100-105, and hepatocellular carcinoma [Id., citing Ye, LY et al. (2016) Cancer Res 76: 818-830].

### Chimeric antigen receptor immunotherapy

TCRs expressed on the surface of T lymphocytes can only recognize the peptide antigens presented to them through MHCs by antigen-presenting cells (APCs). For example, human T cells genetically engineered to express a high-avidity TCR that targets HPV-16 E7 through recognition of the E7₍₁₁₋₁₉₎ epitope complexed with HLA-A*02:01 demonstrated clinical activity in a phase 1 clinical trial for the treatment of metastatic human papilloma virus-associated epithelial cancers (NCT02858310). [Naagarasheth, NB et al. Nature Med. (2021) 27 (30: 419-25). However, transcriptional loss of the specific HLA genes presenting the targeted viral epitope under CD8+ T cell pressure leading to a late acquired resistance to immunotherapy has been reported in metastatic Merkel cell carcinoma. [Paulson, KG et al. Nature Communic. (2018) 9: 3868]. Bajwa, et al. demonstrated that incorporation of a transgenic CD8αβ TCR targeting the tumor-associated antigen surviving in the context of HLA-A*02:01into vectors for TCR-targetable antigens restored the anti-viral activity of the TCR transgenic virus-specific T cells (VSTs) and simultaneously supported tumor-directed activity mediated by the transgenic TCR. [Bajwa, G. et al. J. Immunotherapy of Cancer (2020) 8: e001487].

Monoclonal antibodies can recognize and bind cell surface-expressed antigens that are not presented by MHCs. This ability has been utilized to redirect the cytotoxicity of various kinds of immune cells toward tumor surface-expressed antigens of interest which can be either tumor-specific antigens (TSAs) or tumor-associated antigens (TAAs). [Kozani, PS et al. Front. Immunol. (2022) 13: 795164].

The term "chimeric antigen receptor" or "CAR" as used herein refers to a synthetic MHC-independent receptor that targets T cells to a chosen antigen and reprograms T cell function, metabolism, and persistence [Riviere, I. and Sadelain, M. Mol. Ther. (2017) 25 (5): 1117-24, citing Eshhar, Z. et al. Springer Semin. Immunopathol. (1996) 18: 199-2009; Sadalain, M. et al. Nat. Rev. Cancer (2003) 3: 35-45]. A CAR is mainly composed of three parts: an extracellular antigen recognition domain, usually a single-chain variable fragment (scFv) derived from a monoclonal antibody; a spacer/hinge region and transmembrane domain; and an intracellular signal transduction domain.

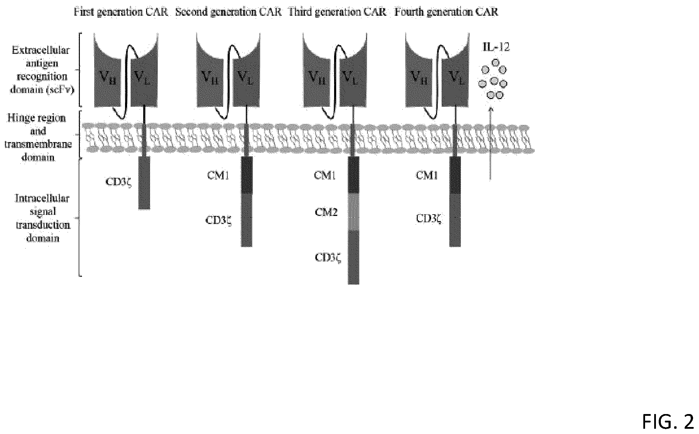

A comparison of structural changes from a first-generation CAR to a fourth generation CAR is shown in FIG. 2. In a first generation CAR, the intracellular signal transduction domain included a CD3ζ chain. In a second generation CAR, the intracellular signal transduction domain included a CD3 ζ chain and one costimulatory molecule. In a third generation CAR, the intracellular signal transduction domain included a CD3ζ chain and two costimulatory molecules. The intracellular signal transduction domain of a fourth generation CARs includes a CD3 ζ chain and one costimulatory molecule and expresses a cytokine, such as IL-12.

An optimal CAR will specifically bind an antigen expressed exclusively in tumor cells to form an effective immunological synapse leading to downstream T-cell signaling and a potent and specific anti-tumor effect.

Through their extracellular domain, CARs bind cell surface molecules independently of the major histocompatibility complex (MHC), in contrast to the physiological T cell receptor, which engages MHC/peptide complexes. CARs may thus target proteins, carbohydrates, or glycolipids and function independently of patient HLA haplotype. Binding to antigen triggers T cell activation, which is commonly mediated by the cytoplasmic domain of the CD3-ζ chain. [Riviere, I. and Sadelain, M. Mol. Ther. (2017) 25 (5): 1117-24, citing Irving, BA and Weiss, A. Cell (1991) 64: 891-901; Romeo, C., Seed, B. Cell (1991) 64: 1037-46; Letourneur, F. and Klausner, RD. Proc. Natl. Acad. Sci. USA (1991) 88: 8905-9; Eshhar, Z. et al. Proc. Natl Acad. Sci. USA (1993) 90: 720-24; Brocker, T. et al. Eur. J. Immunol. (1993) 23: 1435-9].

Merely providing T cell activation is, however, not sufficient to direct a productive immune response. [Id., citing Brocher, T. and Karjalainen, K. J. Exp. Med. (1995) 181: 1653-9; Gong, MMC et al. Neoplasia (1999) 1: 123-7; Brocker, T. Blood (2000) 96: 1999-2001]. The CARs that have provided tangible clinical benefits incorporate a costimulatory domain [Id., citing Krause, A. et al. J. Exp. Med. (1998) 188: 619-26;] which enables T cells to expand and retain their functionality upon repeated exposure to antigen. [Id., citing Maher, J. et al. Nat. Biotechnol. (2002) 20: 70-75] These receptors have been dubbed second generation CARs [Id., citing Sadelain, M. et al. Curr. Opinion. Immunol. (2009) 21: 215-33] and are key to the design of persisting engineered T cells that can attack tumors as long as they retain their functionality. Several recent reviews have addressed CAR design,[Id., citing Jensen, MC and Riddell, SR. Curr. Opinion. Immunol. (2015) 33: 9-15; van der Stegan, SJ et al. Nat. Rev. Drug Discov. (2015) 14: 499-509; Sadelain, M. J. Clin. Invest. (2015) 125: 3392-3400; Maus, MV and June, CH. Clin. Cancer Res. (2016) 22: 1875-84] CAR prospects for solid tumors, [Id., citing Hinrichs, CS and Restifo, NP Nat. Biotechnol. (2013) 999-1008; Morello, A. et al. Cancer Discov. (2016) 6: 133-46] and T cell manufacturing. [Id., citing Wang, X. and Riviere I., Mol. Ther. Oncolytics. (2016) 3: 16015; Levine, BL et al. Mol. Ther. Methods Clin. Dev. (2016) 4: 92-101; Wang, X., Riviere, I. Cancer Gene Ther. (2015) 22: 85-94].

Trials of CAR-T cells targeting the B cell antigen CD19 have shown sustained complete responses in patients with refractory relapsed B cell cancers, although with associated toxicity [Gavriiil, A. et al. Cancers (2020) 12: 2326, citing Maude, SL et al. N. Engl. J. Med. (2014) 371: 1507-17; Titov, A. et al. Cell Death Dis. (2018) 9: 897]. Solid tumors have been generally refractory for diverse reasons, including physical barriers to access of T cells, the presence of suppressive immune cells and tumor cells, the variability of expression of the target antigen, and in many tumors, low mutational burden limiting antigen spreading and generation of memory responses [Id., citing Majzner, RG and Mackall, CL. Nat. Med. (2019) 25: 1341-55; Martinez, M. et al. Front. Immunol. (2019) 10: 128; Yu, W-L et al. Cancers (2019) 11: 47].

### Tumor Escape from CAR-T cell therapy.

To date, antigen loss, immune dysfunction, exhaustion and microenvironment-mediated upregulation of anti-apoptotic pathways have been identified as major modes of escape from CAR-T cell therapy by solid tumors.

***Antigen loss.*** Loss of expression of the antigen on tumor cells targeted by the CAR extracellular domain via the selective immune pressure of CAR-T cells is a major mechanism of CAR-T cell therapy failure. While antigen downregulation or dim expression is a well-known event in lymphoma and myeloma treated with therapeutic IgG antibodies [Rasche, L. et al. N. Kroger, et al, eds. Chapter 4 in The EBMT/EHA CAR-T Cell Handbook (2002) doi.org/10.1007/978-3-030-94353-0_4, citing Plesner, T. et al. Cell (2020) 9 (2): 378; Jilani, I. et al. (2003) Blood (2003) 102 (10): 3514-20], complete target loss is a phenomenon typically occurring after T-cell based therapy, such as CAR-T cell or T cell engaging bispecific antibodies (TCE) therapy, and rarely after treatment with antibody-drug conjugates. For example, antigen loss is a key mechanism of resistance to B cell immunotherapies targeting CD19, CD20 and CD22. [Id., citing Orlando, EJ, et al. Nat. Med. (2018) 24 (10): 1504-6; Bannerji, R. et al. Blood (2018) 132 (Suppl. 1): 1690, Paul, MR et al. J. Pediatr. Hematol. Oncol. (2019) 41 (8): e546-e9]. Case reports have described irreversible B cell maturation antigen (BCMA) loss after anti-BCMA CAR-T cell treatment of multiple myeloma (MM) [Id., citing Da Via, MC et al. Nat. Med. (2021) 27: 616-9; Leblay, N. et al. Blood (2020) 136 (Suppl. 1) 11-2].

In addition to antigen loss, there are other mechanisms that limit or abrogate the effective recognition of cancer cells by CAR-T cells; these mechanisms are either conveyed directly by tumor cells or through a rewiring of the microenvironment. In preclinical models, especially in solid tumors, it was shown that tumor-infiltrating CAR-T cells undergo rapid loss of functionality, limiting their therapeutic efficacy. This hyporesponsiveness appears to be reversible when the T cells are isolated away from the tumor and is associated with upregulation of intrinsic T cell inhibitory enzymes [diacylglycerol kinase and SHP-1, an SH2 domain-containing protein tyrosine phosphatase] and with the expression of surface inhibitory receptors (PD-1, LAG3, TIM3, and 2B4) [Id., citing Moon, EK et al. Clin. Cancer Res. (2014) 20 (16): 4262-73].

### Dysfunctional T cell states

Although TCR and CAR signaling are different, most of the framework for understanding the influence of CAR signaling on CAR-T function and differentiation is derived from work in the TCR field.

TCRs have a hierarchical threshold of antigen density for induction of cell lysis, proliferation and cytokine product [Watanabe, K., et al. Front. Immunol. (2018 )9: 2486, citing Au-Yeung, BB et al. Proc. Nat. Acad. Sci. USA (2014) 111: E3679-88], where less antigen density is required for cell lysis than for cytokine production. CAR-T cells can recognize target cells with considerably lower levels of target antigen and have hierarchical T cell signaling thresholds for cell lysis, proliferation and individual cytokine production. Watanabe demonstrated that the target antigen density required to induce T cell proliferation and cytokine production was higher than that required to induce CAR medicated lysis. [Id., citing Watanabe, K. et al. J. Immunol. (2015) 194: 911-20].

Functional T cells perform a range of activities upon stimulation with their cognate antigen. They can (1) expand, (2) secrete effector cytokines, and lyse target cells; (3) survive after removal of antigen stimulation and (4) do all of the above upon secondary antigen challenge. A T cell is considered dysfunctional if at least one of these activities is not met.

Some studies suggest that the efficacy of immunotherapy is limited by the generation of dysfunctional T cells in the tumor microenvironment (TME).

### T cell exhaustion

T cell exhaustion is a state of T cell dysfunction that arises during many chronic infections and cancer. It is defined by poor effector function, sustained expression of inhibitory receptors and a transcriptional state distinct from that of functional effector or memory T cells.

Exhausted T cells become dysfunctional via a progressive loss of functionality that is mainly mediated by upregulation of multiple inhibitory receptors, including the inhibitory pathways mediated by PD1 in response to binding of PD1 ligand 1 (PDL1) and/or PDL2. [Wherry EJ and Kurachi, M. Nature (2015) 15: 486-99., citing Okazaki T, et al., Nature Immunol. (2013) 14:1212-1218, Odorizzi PM, Wherry EJ. J. Immunol. (2012) 188:2957-2965, Araki K, et al. Cold Spring Harb. Symp. Quant. Biol. (2013) 78:239-247]. Exhausted T cells can co-express PD1 together with lymphocyte activation gene 3 protein (LAG3), 2B4 (also known as CD244), CD160, T cell immunoglobulin domain and mucin domain-containing protein 3 (TIM3; also known as HAVCR2), CTLA4 and many other inhibitory receptors [Id., citing Blackbum SD, et al. Nat. Immunol. (2009) 10:29-37]. Typically, the higher the number of inhibitory receptors co-expressed by exhausted T cells, the more severe the exhaustion. It has been suggested that inhibitory receptors such as PD1 might regulate T cell function in several ways [Id., citing Schietinger A, Greenberg PD. Trends Immunol. (2014) 35:51-60; Odorizzi PM, Wherry EJ. J. Immunol. (2012) 188:2957-2965], e.g., first, by ectodomain competition, which refers to inhibitory receptors sequestering target receptors or ligands and/or preventing the optimal formation of microclusters and lipid rafts (for example, CTLA4); second, through modulation of intracellular mediators, which can cause local and transient intracellular attenuation of positive signals from activating receptors such as the TCR and co-stimulatory receptors [Id., citing Parry RV, et al. Molec. Cell. Biol. (2005) 25:9543-9553; Yokosuka T, et al. J. Exp. Med. (2012) 209:1201-1217; Clayton KL, et al. J. Immunol. (2014) 192:782-791]; and third, through the induction of inhibitory genes [Id., citing Quigley M, et al. Nat. Med. (2010) 16:1147-1151]. Co-stimulatory receptors also are involved in T cell exhaustion [Id., citing Odorizzi PM, Wherry EJ. J. Immunol. (2012) 188:2957-2965].. It has also been possible to exploit the potential beneficial role of co-stimulation to reverse exhaustion by combining agonistic antibodies to positive co-stimulatory pathways with blockade of inhibitory pathways. 4-1BB (also known as CD137 and TNFRSF9) is a TNFR family member and positive co-stimulatory molecule that is expressed on activated T cells. Combining PD1 blockade and treatment with an agonistic antibody to 4-1BB dramatically improved exhausted T cell function and viral control [Id, citing Vezys V, et al. J. Immunol. (2011) 187:1634-1642]. Soluble molecules are a second class of signals that regulate T cell exhaustion; these include immunosuppressive cytokines such as IL-10 and transforming growth factor-β (TGFβ) and inflammatory cytokines, such as type I interferons (IFNs) and IL-6. [Id.]

Antigen-independent (tonic) signaling of a CAR-T cell also can result in an exhausted phenotype. [Gavriil, A. et al. Cancers (2020) 12: 2326, citing Long, AH et al. Nat. Med. (2015) 21]. Long et al showed that second generation CAR-T cells with scFVs targeting 2-ganglioside (G(D2)) derived from antibody 14g2a, CD22, and ErbB2 plus a CD28-CD3z endodomain, in contrast to CD19 CARs, signal constitutively during their in vitro expansion. These CARs had enhanced T cell exhaustion and diminished anti-tumor activity in vivo. The authors demonstrated that scFv clustering, due to interaction between scFv framework regions, was responsible. Similarly, Frigault et al. have shown that high level expression of certain scFv-containing CARs leads to long term antigen-independent signaling and functional exhaustion. [Id., citing Frigault, , MJ et al., Cancer Immunol. Res. (2015) 3: 356-67].

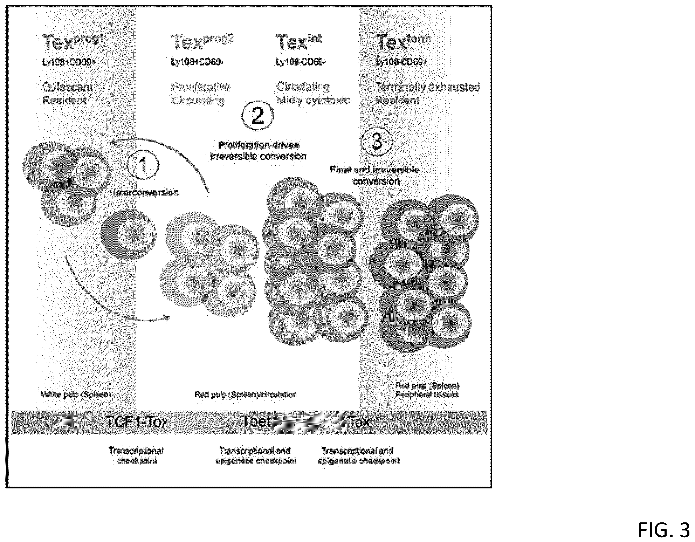

Beltra, et al. [Beltra, JC, et al. Immunity (2020) 19: 825-41] identified a four cell stage developmental framework for T cell exhaustion during chronic lymphocytic choriomeningitis virus (LCMV) infection in mice with complementary analysis in mice. A similar pattern of four Tex subsets was found in mouse B16 tumors and also for tumor-infiltrating lymphocytes from human melanoma, mouse and human tumors. The four distinct Tex subsets were based on Ly208 (Slamf6) and CD69 expression. FIG. 3 depicts the four stage developmental framework.

Two interconverting TCF1+ progenitor states were identified, one quiescent and blood inaccessible and a second that initiated robust cell cycling and gained access to circulation. This second TCF1+Tex subset gave rise to a TCF-1- Tbet^{hi} intermediate Tex subset that (re)acquired some effector-like features. These intermediate Tex cells ultimately became terminally differentiated, losing T-bet (and gaining Eomes) and permanently exiting the cell cycle. This final transition was coordinated by TOX-mediated antagonism of T-bet. PD-1 pathway blockage preferentially expanded the second progenitor and the T-bet^{hi} intermediate Tex subsets. These cell subset transitions were regulated by the transcription factors TCF-1, T-bet and TOX in a hierarchical developmental pathway. The level and duration of chronic antigen stimulation and infection seemed to be key factors that lead to T cell exhaustion and correlated with the severity of dysfunction during chronic infection.

### T cell senescence

Another dysfunctional state is senescence, which occurs when T cells permanently arrest their cell cycle and proliferation while retaining cytotoxic capability. Senescent T cells have a distinct phenotype including downregulated expression of the costimulatory molecules CD27 and CD28, and high expression of CD57, killer cell lectin-like receptor subfamily G member 1 (KLRG-1), and CD45RA [Zhang, J. et al. EBioMedicine (2021) 68: 103409, citing Barbarin, A. et al. Front. Immunol. (2017) 8: 316; Covre, LP et al. Aging Cell (2020) 19: e13272; Liu, W. et al. Cancers (2020):12]. Senescent T cells also show a terminally differentiated phenotype with the downregulation of the chemokine receptors CCR7 and CD45RO but the upregulation of CD45RA [Id., citing Henson, SM et al. J. Clin. Invest. (2014) 124: 4004-16; Huang, B. et al. J. Immunotherapy Cancer (2020) 8; Kunert, A. et al. J. Immunother. Cancer (2019) 7: 149]. In senescent T cells, the efficiency of TCR signaling is compromised in that the expression of key components of TCR signaling (CD3, Lck, Zap70, SLP-76, LAT, and PLCγ1) [Id., citing Pereira, BI et al. Nature Immunol. (2020) 21: 684-94; Lanna, A. et al. Nature Immunol. (2014) 15: 965-72], as well as the costimulatory molecules CD27 and CD28 [Id., citing Ye, J. et al. Blood (2012) 120: 2021-31; Lanna, A. et al. J. Immunol. (2013) 191: 3744-52] is decreased. In addition, the phosphorylation of Zap70 is impaired following CD3 activation [Id., citing Pereira, BI et al. Nature Immunol. (2020) 21: 684-94; Lanna, A. et al. Nature Immunol. (2014) 15: 965-72]; phosphorylation of Zap-70 is required to initiate T cell receptor signaling [Yan, Q. et al. Mol. Cell Biol. (2013) 33 (11): 2188-2201].. Senescent T cells, when stimulated with anti-CD3 plus anti-CD28, especially those isolated from old individuals, produce significantly lower amounts of IL-2, interferon gamma (IFN-γ), tumor necrosis factor alpha (TNF-α), and granzyme B than other types of T cells [Zhang, J. et al. EBioMedicine (2021) 68: 103409, citing Henson, SM et al. Eur. J. Immunol. (2015) 45: 1441-51; Song, Y. et al. Aging Cell (2018) 17]. Moreover, TCRβ chain diversity roughly declines linearly with age, especially in senescent CD8+ T cells [Id., citing Britanova, OV et al. J. Immunol. (2014) 192: 2689-98; Bjorkstrom, NK et al. Blood (2012) 120: 3455-65]. These findings led to the conclusion that senescent T cells probably dampen TCR-dependent antigen-specific killing.

Senescent T cells also upregulate the expression of NK cell receptors (NKRs), including NKG2A/C and killer cell lectin like receptor G1 (KLRG-1) [Id., citing Barbarin, A. et al. Front. Immunol. (2017) 8: 316; Pereira, BI et al. Nature Immunol. (2020) 21: 684-94]. On NK cells, NKG2C delivers activating signals through immunoreceptor tyrosine-based activation motifs at the cytoplasmic tails of DAP12 transmembrane proteins [Ma, M. et al. Front. Immunol. (2017) 8: 1176, citing Call, ME et al. Nat. Immunol. (2010) 11: 1023-9], while NKG2A appears to inhibit NK activation via immunoreceptor tyrosine-based inhibitory motifs in the cytoplasm [Id., citing Lanier, LL. Annu. Rev. Immunol. (1998) 16: 359-63]. KLRG1 plays an inhibitory role in human NK cells and T cells [Yang, X. et al. BMC Cancer (2021) 21: 752].

Senescent T cells display higher levels of CD107a, granzyme B, and perform than other subsets with no stimulation [Zhang, J. et al. EBioMedicine (2021) 68: 103409., citing Song, Y. et al. Aging Cell (2018) 17] and under NKG2D stimulation [Id., citing Pereira, BI et al. Nature Immunol. (2020) 21: 684-94]. In vitro experiments have shown that senescent T cells kill tumor cells independent of TCR, with the same efficiency as natural killer (NK) cells [Id., citing Pereira, BI et al. Nature Immunol. (2020) 21: 684-94]. This evidence led to the conclusion that although antigen specific killing is lost, senescent T cells with strong nonspecific killing potential fight antitumor immunity to some extent.

### T cell anergy

Anergy, which is generally described as an induced hyporesponsive state with low IL-2 production or incomplete activation to which naive T cells fall upon low co-stimulatory and/or high co-inhibitory stimulation, is another dysfunctional state. [Crespo, J. et al. Curr. Opin Immunol. (2013) 25 (2): 214-221].

The evidence for T cell anergy in the tumor context has been indirect. A core problem has been a lack of positive markers to characterize a loss of function state of T cells. Nonetheless, the following observations support that T cell anergy can be an important phenomenon in cancer:
(a) There is an active imbalance between stimulatory and inhibitory B7 family members in the tumor microenvironment [Id., citing Zou, W. Nature Rev. Cancer (2005) 5: 263-74; Pardoll, DM. Nat. Rev. Cancer (2012) 12: 252-64; Zou W. and Chen, L. Nat. Rev. Immunol. (2008) 8: 467-77; Blank, C. et al. Cancer Res. (2004) 64: 1140-1145]. Human tumors and tumor associated antigen presenting cells (APCs) often express high levels of B7-H1 (CD274 or PD-L1), B7-H2 (CD275 or ICOS-L), B7-H3 (CD276), B7-H4 (B7S1 or B7x), and B7-DC (CD273 or PD-L2) with low-to-absent expression of B7.1 (CD80) and B7.2 (CD86) [Id., citing Zou W. and Chen, L. Nat. Rev. Immunol. (2008) 8: 467-77; Blank, C. et al. Cancer Res. (2004) 64: 1140-1145; Curiel, TJ et al. Nat. Med. (2003) 9: 562-67; Kryczek, I. et al. J. Exp. Med. (2006) 203: 871-81]. This indicates a poor co-stimulatory, high co-inhibitory and therefore anergy-promoting environment.
(b) Animal model studies have shown that introduction of B7.1 into tumors by transfection or blockade of inhibitory B7 family members can reduce tumor growth or result in spontaneous tumor rejection in vivo [Id., citing Zou W. and Chen, L. Nat. Rev. Immunol. (2008) 8: 467-77; Blank, C. et al. Cancer Res. (2004) 64: 1140-1145; Curiel, TJ et al. Nat. Med. (2003) 9: 562-7; Kryczek, I. et al. J. Exp. Med. (2006) 203: 871-81; Chen, L. et al. Cell (1992) 71: 1093-1102; Gajewski, TF. J. Immunol. (1996) 156: 465-72].
(c) Homeostatic proliferation of anti-tumor T cells in a lymphopenic host both reverses anergy and promotes tumor rejection in vivo [Id., citing Brown, IE et al. J. Immunol. (2006) 177: 4521-29].
(d) There is evidence indicating antigen specific, T cell-intrinsic dysfunction in the tumor microenvironment [Id., citing Nazareth, MR et al. J. Immunol. (20007) 178: 5552-62; Broderick, L. et al. Clin. Immunol. (2006) 118: 159-69].

Based on this evidence, it has been suggested that T cell anergy may be a functional mechanism in patients with cancer. However, the relative impact of T cell anergy on tumor immunity remains to be defined.

Cellular and molecular mechanisms controlling T cell anergy are insufficiently understood. It is generally accepted that T cells that are presented antigen along with suboptimal CD28 co-stimulation [Id., citing Schwartz, RH. Science (1990) 248: 1349-56; Schwartz, RH. (2003) 21: 305-34] and/or high co-inhibition [Id., citing Greenwald, RJ et al. Immunity (2001) 14: 145-55] result in anergic phenotypes, as characterized by their low IL-2 production and cell cycle arrest at the G1/S phase. Early growth response gene 2 (Egr2) may be a central transcription factor that regulates the T cell anergic state [Id., citing Zeng, Y. et al. J. Exp. Med. (2012) 209 (12): 2157-63].

It has been suggested that the anergy program is initiated by improper mTOR and Ras/MAPK signaling in the cell, a pathway which lies directly downstream of TCR/CD28 engagement. Specifically, sole binding of TCR by MHC promotes Ca2+ imbalance on T cells and retention of active-RAP-1 in the cytosol, an imbalance that would normally be corrected by co-stimulation through CD28 (Ras/MAPK) [Id., citing Boussiiotis, VA et al. Science (1997) 278: 124-8; Dolmetsch, RE et al. Nature (1998) 392: 933-6]. The effects of this imbalance on the genetic reprogramming of these cells have been hypothesized to be mediated by NFAT homodimer formation and transcription of anergy-inducing genes [Id., citing Anandasabapathy, N. et al. Immunity (2003) 18: 535-47; Soto-Nieves, N. et al. J. Exp. Med. (2009) 206: 867-721]. The E3 ubiquitin ligase family can affect PI3K, mTOR, and Ras/MAPK signaling pathways and help to actively maintain anergy [Id., citing Anandasabapathy, N. et al. Immunity (2003) 18: 535-47;Jeon, MS et al. Immunity (2004) 21: 167-77; Mueller, DL. Nat. Immunol. (2004) 5: 883-90]. Epigenetic factors such as IKAROS (through acetylation), an inhibitory C-type lectin with an intracellular immunoreceptor tyrosine-based inhibitory motif (ITIM), and Sirt1 are involved in histone modifications that promote T cell anergy [Id., citing Gao, B. et al Proc. Natl Acad. Sci. USA (2012) 109: 899-904; Bandyopadhyay, S. et al. Blood (2007) 109: 2878-86]. Thus, anergy may be the combined result of factors that negatively regulate proximal TCR-coupled signal transduction, together with a program of active transcriptional silencing that is reinforced through epigenetic mechanisms [Id., citing Wells, AD. J. Immunol. (2009) 182: 7331-41].

### Tumor microenvironment

The TME comprises cellular components, noncellular components and signaling molecules [Ameth, B. Medicina (2020) 56: 15, citing Spill, F. et al. Curr. Opin. Biotechnol. (2016) 40: 41-48; Del Prete, A. et al. Curr. Opin. Pharmacol. (2017) 35: 40-47]. The cellular components include endothelial cells, which play a role in tumor development and tumor cell protection from the immune system; immune cells, such as granulocytes, lymphocytes and macrophages, which are involved in various immune responses and activities, such as inflammatory reactions orchestrated by the tumor to promote survival; and fibroblasts, which allow cancer cells to migrate from the primary tumor location in to the bloodstream for systemic metastasis and provide a passage for endothelial cells undergoing angiogenesis in the tumor. The extracellular matrix (ECM), which is mainly secreted by cancer-associated fibroblasts (CAFs), which produce more ECM proteins than normal fibroblasts, is composed of various macromolecules, including collagens, glycoproteins (fibronectin and laminins), proteoglycans and polysaccharides with different physical and biological properties. [Brassart-Pasco, S. et al. Front. Oncology (2020) 10: 397]. Interstitial matrix, primarily synthesized by stromal cells, is rich in fibrillary collagens and proteoglycans. CAF secretome analyses show an increased secretion of bone morphogenetic protein (BMP) 1, thrombospondin-1 and elastin interface 2 [Id. citing Santi, A. et al. Proteomics (2018) 18: e1700167; Socovich, AM and Naba, A. Semin. Cell Dev. Biol. (2019) 89: 157-66]. Several splice variants of fibronectin ED-A and ED-B and tenascins C and W may be secreted by CAFs. [Id., citing Grahovac, J. and Wells, A. Lab Investig. (2014) 94: 31-40].

Tumor cells can influence the microenvironment through the release of extracellular paracrine signals that induce peripheral immune tolerance and support tumor angiogenesis [Ameth, B. Medicina (2020) 56: 15, citing Korneev, KV et al. Cytokine (2017) 89: 127-35]. The composition and structure of the TME is known to vary among cancer types and among patients. [Id., citing Bussard, K. et al. Breast Cancer Res. (2016) 18: 84].

Cellular interactions in the TME also affect cancer development and progression. Within the heterogeneous TME, T cells are a major part of the immune infiltrate. [Zhang, Z. et al. Frontiers Cell & Devel. Biol. (2020) 8: 17]. The intratumoral T cell populations comprise naive, memory, effector, and regulatory T cells (Tregs). [Id., citing Hashimoto, M. et al. Annu. Rev. Med. (2018) 69: 301-18]. In a murine model, the tumor-specific T cell dysfunctional exhaustion state was found to be initiated early after tumor initiation and antigen encounter. [Id., citing Schietinger, A.et al., Immunity (2016): 45: 389-401]. Dysfunctional CD8+ T cells are characterized by a loss of effector functions, such as cytotoxicity and proliferation. In addition, the upregulation of immune checkpoints and changes in transcriptional and metabolic molecules have been described as hallmarks of T cell dysfunction. Emerging evidence indicates that epigenetic states, including DNA methylation and histone modifications, and chromatin landscapes are closely associated with the functional state of dysfunctional or exhausted CD8+ T cells. [Id., citing Pauken, KE et al. Science (2016) 342: 1242454; Sen, DR et al. Science (2016) 354: 1165-9; Kartikasari, AER et al. Front. Immunol. 9: 3109].

In many hematological cancers, the bone marrow tumor microenvironment (BMME) is known to upregulate anti-apoptotic mechanisms in tumor cells through tight cross-talk between MSCs and tumor cells; tumor cell lysis by T and NK cells also is largely mediated via activation of extrinsic and intrinsic apoptotic pathways [Rasche, L. et al. N. Kroger, et al, eds. Chapter 4 in The EBMT/EHA CAR-T Cell Handbook (2002) doi.org/10.1007/978-3-030-94353-0_4, citing Hanabuchi, S. et al. Proc. Natl Acad. Sci. 1994 91(1)]: 4930-4; Falschlehner, C. et al. Immunology (2009) 127 (2): 145-54; Carneiro, BA and El-Deiry, WS. Nat. Rev. Clin. Oncol. (2020) 17 (7): 395-417; Culen, SP et al. Cell Death Differ. (2010) 17 (4): 616-23; Sutton, VR et al. J. Exp. Med. (2000) 192 (10): 1403-14]. Myeloma cell-bone marrow mesenchymal stromal cell (BMMSC) interactions have been reported to protect myeloma cells from conventional cytotoxic T cells and from daratumumab (DARZALEX^{®}) redirected NK cells [Id., citing McMillin, DW et al. Blood (2012) 119 (15): e131-8; de Haart, SJ et al. Clin. Cancer Res. (2013) 19 (20): 5591-601; de Haart, SJ et al. Haematologica (2016) 101 (8): e339-42].

***The importance of the tumor stroma in the efficacy of CAR-T cells.*** These studies were extended to CAR-T cells by testing a panel of nine different myeloma-reactive CAR-T cells that were reactive to three different myeloma-associated antigens (CD138, B Cell Maturation Antigen (BCMA), and CD38) with different target affinities and with different costimulatory domains (CD28, 4-1BB, or CD28 plus 4-1BB [Id., citing Holthof, L. et al. Clin. Cancer Res. (2021) 27 (13): 3793-803]. In the absence of BMMSCs, BCMA^{bb2121} CAR-T cells, high affinity CD38 CAR-T cells, and intermediate affinity CD38 CAR-T cells containing CD28 costimulatory domains showed high levels of anti-myeloma cell lysis, whereas other CAR-T cells showed moderate cytotoxic activity against myeloma cells. BMMSCs did not modulate the lytic activity of highly lytic CAR-T cells but readily protected myeloma cells against all other CAR-T cells with intermediate killing capacity. Overall, a strong inverse correlation was demonstrated between the lytic capacity of the CAR-T cells and the extent of BMMSC-mediated protection. Furthermore, the BMMSC-mediated protection of myeloma cells from these CAR-T cells was readily abrogated by inhibition of survivin (an apoptosis inhibitor), myeloid cell leukemia 1 (MCL-1), and X-linked inhibitor of apoptosis (XIAP) using the small molecule surviving inhibitor FL118. These results confirmed that BMMSC-mediated immune resistance was mediated by negative regulation of apoptotic pathways. In addition, in a solid tumor mouse model, destruction of the tumor stroma contributed to eradication of large tumors by HER2-specific CAR-T cells (Id., citing Textor, A. et al. Cancer Res. (2014) 74 (23): 6796-805).

### Current Challenges of CAR-T therapy

Toxicities with CAR-T cells can be classified as on-target (likely to achieve the intended outcome at the target) or off-target (adverse effects resulting from modulation of other targets). The longest running clinical experience in the CAR-T cell field has been with CD19-directed CAR T cells, for which two on-target toxicities, cytokine release syndrome (CRS) and immune effector cell associated neurotoxicity syndrome (ICANS) have been described. [Finck, AV et al. Nat. Med. (2022) 28 (4): 678-89, citing Lee, DW et al. Biol. Blood Marrow Transpl. (2019) 25: 625-38]. CRS and ICANS toxicities, while now regarded as a class effect of CAR-T cells, as they have been observed in patients treated with CAR-T cells targeting CD19, BCMA, and many other cell surface structures [Id., citing Lateau, CA et al. Cancer Cell (2021) 39: 1553-7] may be more of a feature of increasingly potent therapies. Also, the expression of targets in healthy patients may not be the same as in patients with cancer.

Cytokine release syndrome (CRS) is a potentially life-threatening, systemic inflammatory response observed following administration of antibodies and adoptive T cell therapies, including CAR-T cell therapy. With immune activation, an associated increase in a wide array of systemic proinflammatory cytokines, such as IL-6, C-reactive protein (CRP), ferritin, and IL-2, occurs, which coincides with peak-T cell expansion. Severity can vary from mild symptoms including fever, myalgia, and fatigue, to severe symptoms including but not limited to acute respiratory distress syndrome, hypotension, disseminated intravascular coagulation, and/or renal and liver toxicities. [Shalabi, H. et al. Chapter 12 in Novel Designs of Early Phase Trials for Cancer Therapeutics (2018) Academic Press, pages 175-91].

ICANS was initially thought to result from a cascade of off-target effects due to systemic inflammation and cytokine release [Finck, AV et al. Nat. Med. (2022) 28 (4): 678-89, citing Taraseviciute, A. et al. Cancer Discov. (2018) 8: 750-63]; however, some aspects of the syndrome may be related to the expression of CD 19 in pericytes in the central nervous system [Id., citing Parker, KR et al. Cell (2020) 183: 126-42], which was unexpected since CD19 initially was described as a B-lineage-restricted protein.

On-target, off-tumor toxicity can occur because most tumor-associated antigens are not tumor-specific but are only overexpressed on tumor cells. Although target antigens are expressed on both tumor and normal cells, not all CAR-T cell therapies exhibit observable on-target off-tumor effects. [Han, et al. J. Hematol. & Oncol. (2919) 12: 128, citing Brown, CE et al. Clin. Cancer Res. (2015) 21 (18): 4062-72; Katz, SC et al. Clin. Cancer Res. (2015) 21 (14): 3149-50; Klampatsa, A. et al. Cancers (Basel) (2017) 9 (9); Kakarla, S. et al. Mol. Ther. (2013) 21 (8): 1611-20; Wang, LC et al. Cancer Immunol. Res. (2014) 2 (2): 154-66]. During solid tumor treatment, on-target, off-tumor toxicities following the infusion of CAR-T cells can cause serious adverse events or be lethal. [Han, X. et al. J. Hematol & Oncol. (2019) 12: 128, citing Morgan, RA et al. Mol. Ther. (2010) 18 (4): 843-51; Lamers, CH et al. J. Clin. Oncol. (2006) 24 (13): a20-2; Tran, E. et al. J. Exp. Med. (2013) 210 (6): 1125-35].

***Disease relapse after CAR-T cell infusions.*** There are two main forms of disease relapse: antigen-positive relapse in the early phase and antigen escape relapse in a later phase. The disease relapse of antigen-positive cells is closely related to CAR-T cell persistence, meaning how long the functional CAR-T cells exist after infusion into the subject. CAR-T cell persistence is a major challenge. Preclinical and clinical trials have shown that the 4-1BB (CD137) costimulatory domain induced longer-term persistence of CAR-T cells than did CD 28 [Id., citing Zhao, Z. et al. Cancer Cell (2015) 28 (4): 415-28; Kachenderfer, JN et al. J. Clin. Oncol. (2015) 33 (6): 540-9].

Antigen escape, including antigen loss or down regulation also can drive disease relapse following CAR-T immunotherapy. Complete antigen loss is not necessary for CAR-T cell resistance, and in some cases, a reduction in antigen density is sufficient for tumor cells to evade CAR-T cells [Id., citing Fry, TJ et al. Nat. Med. (2018) 24 (10: 20-8]. Investigators have shown that combinatorial multi-antigen targeted strategies could effectively prevent the tumor escape caused by low antigen density that results from CAR-T cell trogocytosis [Id., citing Hamieh, M. et al. Nature (2019) 568 (7750): 112-6]. Immune escape caused by low antigen level and/or heterogeneous expression is a cause of solid tumor resistance to CAR-T cells.

***Optimization of CAR design.*** Optimization of the CAR design is equally important for better persistence and overall treatment efficacy.

The concepts of Boolean Logic gates have been applied to CAR-T cell design primarily as a tool for increasing specificity by integrating signals from two or more target antigens. Such studies have focused on AND gates (where two target antigens are required for full activation as a means to limit toxicity), OR gates (involving two equally powered CARS against alternate target antigens in the same tumor) and NOT gates (an off-signal associated with a target antigen present on normal tissue as a means to limit off-target toxicity). A CAR with a endodomain providing signal 2 but no signal 1 is also referred to as a chimeric co-stimulatory receptor (CCR) and is said to provide "co-stimulation in trans," thereby enhancing proliferation and survival without an effect on cytotoxicity. [Gavriil, A. et al. Cancers (2020) 12: 2326, citing Kloss, CC et al. Nat. Biotechnol. (2013) 31: 71-75; Lanitis, E. et al. Cancer Immunol. Res. (2013) 1: 43-53].

Several paired target antigens have been evaluated using AND gate approaches in preclinical models [Gavriil, A. et al. Cancers (2020) 12: 2326, citing Wilkie, S. et al. J. Clin. Immunol. (2012) 32: 1059-70; Kloss, CC et al. Nat. Biotechnol. (2013) 31: 71-75]. Several groups have further designed bi-specific CARS, which utilize the OR -gate circuit. [Id., citing Zah, E. et al. Cancer Immunol. Res. (2016) 4: 498-508; Munter, SD et al. Int. J. Mol. Sci. (2018) 19: 403]. The NOT gate logic has been applied to generate CARs coupled with an immune-inhibitory signal (iCAR), such as PD-1 or CTLA4, to divert off-target immunotherapy responses [Id., citing Fedorov, VD, et al. Sci. Transl. Med. (2013) 5: 215ra172].

The basic logic principles above have been extended to the concept of the sequential AND gate, whereby engagement with one target antigen triggers the expression of a CAR against a second target antigen. Since the first antigen does not provide activating T cell signals, and the CAR against the second antigen is not expressed until the T cell reaches the tumor, this arrangement potentially can limit both tonic signaling and exhaustion, as well as off-target toxicity. [Id., citing Morsut, L. et al. Cell (2016) 164: 780-91; Roybal, KT et al. Cell (2016) 167: 419-32; Roybal, KT et al. Cell (2016) 164: 770-79].

Immune checkpoint blockade is another approach to overcome tumor-associated immune suppression [Finck, AV et al. Nat. Med. (2022) 28 (4): 678-89, citing Rafiq, S. et al. Nat. Biotechnol. (2018) 36: 847-56] and revitalize T cells [Id., citing Xia, Y. et al. Blood Rev. (2016) 30: 189-200]. In clinical trials, the combination of the PD-1 immune checkpoint inhibitor pembrolizumab (KEYTRUDA^{®}) with lesothelin-targeting CAR-T cells further enhanced the persistence and function of the latter in patients with malignant pleural diseases [Id., citing Adusumillli, PS et al. Cancer Discov. (2021) 11: 2748-63]. One study demonstrated that two CARS, one targeting EGFRvIII and the other targeting IL-13Ra2, preferred different checkpoint blockades within the same tumor model [Id., citing Yin, Y. et al. Mol. Ther. Oncolytics (2018) 11: 20-38]. An alternative approach is to engineer CAR-T cells to produce bispecific antibodies, as shown in a preclinical study for the treatment of glioblastoma. [Id., citing Choi, BD et al. Nat. Biotechnol. (2019) 37: 1049-58].

### Allogeneic CAR-T cells

Autologous CAR-T cell therapy does not carry a significant risk of graft-versus host disease because the cells are patient -derived, and theoretically, such cells would have undergone thymic selection, which is aimed at eliminating auto-reactive T-cell clones. [Sanber, K. et al. Br. J. Haematology (2021) 195: 660-68, citing Cheng, M. and Anderson, MS. Nat. Immunol. (2018) 19: 659-64] Nevertheless, anti-CAR immunity has been documented clinically [Wagner, DLl et al. Nat. Rev. Clin. Oncol. (2021) 18 (6): 373-93].

In some patients, genetically engineered autologous T cells might not be effective due to T-cell dysfunction, which is a hallmark of many cancers and is associated with multiple mechanisms of immunosuppression derived from the TME. [Depil, S. et al. Nature Reviews Drug Discovery (2020) 19: 185-99]. The biological characteristics of autologous T cells also can be adversely affected by previous lines of treatment leading to product failure.

There is growing interest in developing off-the-shelf allogeneic CAR-T cells , which potentially could address these issues, and also allow the creation of batches of products, which can be available if redosing is necessary without a prolonged waiting time. Off-the-shelf allogeneic CAR-T cells also may allow combinations of CAR-T cells directed against different targets.

However, allogeneic approaches are associated with two main issues. First, the administered allogeneic T cells may cause life-threatening graft-versus host disease (GVHD). Second, the allogeneic T cells may be rapidly eliminated by the host immune system, limiting their antitumor activity.

Classic acute GVHD occurs before day 100 and is staged according to the percentage of body surface area with rash, total bilirubin elevation, and volume of diarrhea. Late acute GVHD occurs after day 100 and is defined as signs and symptoms of acute GVHD without chronic GVHD. Late acute GVHD is further subdivided into "persistent" if it is a continuation of classic acute GVHD, "recurrent" if classic acute GVHD resolves then recurs after day 100, or "de novo" if initial onset is after day 100 without any prior acute GVHD. [Lee, SJ. Blood (2017) 129 (1): 30-37]. Chronic GVHD is a pleiotropic, multi organ syndrome involving tissue inflammation and fibrosis that often results in permanent organ dysfunction. [Id.]

Several systems for grading acute GVHD have been developed. The two most popular are the Glucksberg grade (I-IV) and the International Bone Marrow Transplant Registry (IBMTR) grading system (A-D) [Glucksberg, H. et al. Transplantation (194) 18: 295; Rowlings, PA et al. Br. J. Haematol. (1977) 97: 855]. The severity of acute GVHD is determined by an assessment of the degree of involvement of the skin, liver, and gastrointestinal tract. The stages of individual organ involvement are combined with (Glucksberg) or without (IBMTR) the patient's performance status to produce an overall grade, which has prognostic significance. Grade I(A) GVHD is characterized as mild disease, grade II(B) GVHD as moderate, grade III(C) as severe, and grade IV(D) life-threatening [Przepiorka, D. et al. Bone Marrow Transplant (1995) 15: 825; Cahn, JY et al. Blood (2005) 106: 1495].

The IBMTR grading system defines the severity of acute GVHD as follows [Rowlings, PA et al. Br. J. Haematol. (1977) 97: 855]:
- Grade A - Stage 1 skin involvement alone (maculopapular rash over <25 percent of the body) with no liver or gastrointestinal involvement
- Grade B - Stage 2 skin involvement; Stage 1 to 2 gut or liver involvement
- Grade C - Stage 3 involvement of any organ system (generalized erythroderma; bilirubin 6.1 to 15.0 mg/dL; diarrhea 1500 to 2000 mL/day)
- Grade D - Stage 4 involvement of any organ system (generalized erythroderma with bullous formation; bilirubin >15 mg/dL; diarrhea >2000 mL/day or pain or ileus)

### Post-HCT setting CAR-T therapy

Although hematopoietic cell transplantation (HCT) from haploidentical or HLA-matched unrelated donors is the only potentially curative therapeutic option in many patients with relapsed/refractory hematological malignancies [Sanber, K. et al. Br. J. Haematology (2021) 195: 660-68, citing Gyurkocza, B. et al. Expert Rev. Hematol. (2010) 3: 285-99; Kanakry, CG et al. Nat. Rev. Clin. Oncol. (2016) 13: 10-24], disease relapse post-HCT remains a leading cause of mortality. [Id., citing McCurdy, SR et al. Haematologica (2017) 102: 391-400; Barrett, AJ and Battiwalla, M. Expet Rev. Hematol. (2010) 3: 429-41].

HCT carries the risk of GVHD, which occurs when donor T cells recognize recipient alloantigens expressed in the recipient's normal tissues. [Id., citing Copelan, EA. N. Engl. J. Med. (2006) 354: 1813-26]. Various interventions have been introduced to minimize this risk. For example, in haploidentical donor HCT, post-transplant cyclophosphamide has been shown to lower the risk for GVHD. [Id., citing Luznik, L. et al. Biol. Blood Marrw Transplant. (2008) 14: 641-50].

CAR-T cell therapy represents a potential therapeutic option for patients in this setting. CAR-T cells may be manufactured from either true allogeneic (donor derived) CAR-T cells or from the recipient's T cells post-HCT [pseudo-allogeneic or recipient CAR-T cells]. However, GVHD remains a potential complication of HCT, and allogeneic CAR-T cells may increase the risk of developing de novo GVHD or exacerbating pre-existing GVHD.

### Donor-derived CAR-T therapy

Different strategies have been utilized to reduce the risk of GVHD when using true-allogeneic (or donor-derived) CAR T cells. Pioneering studies utilized allogeneic donor-derived virus-specific T cells (VSTs) to manufacture CAR T cells (or CAR VSTs) [Id., citing Cruz, CRY et al. Blood (2013) 122: 2965-73; Lapteva, N. et al. Clin. Cancer Res. (2019) 25: 7340-50; Davila, ML et al. Sci. Transl. Med. (2014) 6: 224ra25; Kebriaei, P. et al. J. Clin. Invest. (2016) 126: 3363-76; Brudno, JN et al. J. Clin. Oncol. (2016) 34: 1112-21; Chen, Y. et al. Br. J. Haematol. (2017) 179: 598-605; Zhang, C. et al. Leukemia (2020) 35: 1563-70; Liu, P. et al. Front. Oncol. (2020) 10: 573822; Dai, H. et al. Oncoimmunol. (2015) 4: e1027469]. In these studies, the donor T cells were first exposed to libraries of peptides derived from viruses that commonly cause post-HCT infections, such as Epstein-Barr virus (EBV), cytomegalovirus (CMV) and adenoviruses, with the aim of selecting T cells whose T-cell receptors (TCRs) recognize foreign viral antigens rather than alloantigens.

Several groups have reported encouraging results using donor-derived CAR T cells, with a relatively low incidence of GVHD in the majority of these studies [Id., citing Davila, ML et al. Sci. Transl. Med. (2014) 6: 224ra25; Kebriaei, P. et al. J. Clin. Invest. (2016) 126: 3363-76; Brudno, JN et al. J. Clin. Oncol. (2016) 34: 1112-21; Chen, Y. et al. Br. J. Haematol. (2017) 179: 598-605; Zhang, C. et al. Leukemia (2020) 35: 1563-70].

For example, Ghosh et al. [Id., citing Ghosh, A. et al. Nat. Med. (2017) 23: 242-9] showed that donor-derived CAR T cells expressing a second-generation anti-C19 CAR with the CD28 endodomain (CD28z.CAR T cells) mediated significant graft-versus-lymphoma effect with minimal GVHD in a mouse model of post-HCT relapse. In this study, a mouse model of major histocompatibility complex (MHC)-mismatched HCT was used whereby B6 hematopoietic stem cells were transplanted into BALB/c mice and then inoculated with recipient matched CD19+ lymphoma cells to mimic post-HCT relapse. True-allogeneic donor anti-CD19 CAR T cells were then adoptively transferred. In this model, alloreactive CD28z.CAR T cells exhibited increased phosphorylation of activating signaling pathways when compared to their non-alloreactive counterparts. This more potent activation was associated with functional exhaustion and activation-induced cell death in alloreactive CAR T cells. The authors hypothesized that this phenomenon limited GVHD while the nonexhausted, non-alloreactive CD28z.CAR T cells mediated significant graft-versus-lymphoma activity. [Id., citing Ghosh, A. et al. Nat. Med. (2017) 23: 242-9]. In contrast, CAR T cells expressing a first-generation CAR (carrying the CD3ζ signaling domain without additional co-stimulatory domains) or a second-generation CAR utilizing the 4-1BB co-stimulatory domain (41BBz.CAR) led to a lower level of activation, less exhaustion and an increased incidence and severity of GVHD.

In another study, Jacoby et al. [Id., citing Jacoby, E. et al. Blood (2016) 127: 1361-70] used an MHC-matched minor mismatch mouse model to evaluate donor-derived anti-CD19 CAR T cells. The CAR used in this study utilized an anti-CD19 scFv and CD28 co-stimulatory domain (similar to the study by Ghosh et al.), but the first and third immunoreceptor tyrosine-based activation motif (ITAM) domains of the CD3ζ domain were mutated. [Id., citing Jacoby, E. et al. Blood (2016) 127: 1361-70, Kochenderfer, JN et al. Blood (2010) 116: 3875-86]. These mutations may have attenuated CAR-mediated signaling leading to reduced exhaustion of alloreactive CAR T cells. In this study, acute GVHD was dependent on the interaction of CD4+ CAR T cells with tumor cells and subsequent interleukin 6 production.

After second generation donor CAR-T therapy, Liu et al. [Id., citing Liu, P. et al. Front. Oncol. (2020) 10: 573822] reported a relatively high incidence of acute GVHD in 10 of the 15 patients evaluated of which eight patients had prior acute GVHD post-HCT (activity/status at time of CAR T-cell infusion was not reported) and two patients had new onset acute GVHD post-CAR T-cell infusion.

Other studies have included a small number of patients with pre-existing GVHD and did not report the incidence of severe GVHD. A 41BBz.CAR T-cell product was used in this study, which, based on the limited pre-clinical data available, may be associated with a higher risk of GVHD. [Id., citing Ghosh, A. et al. Nat. Med. (2017) 23: 242-9].

Similarly, a study by Dai et al. [Dai, H. et al. Oncoimmunol. (2015) 4: e1027469] utilizing a second-generation 41BBz.CAR T cell, also reported acute GVHD in both of the treated patients.

### Pseudo-allogeneic CAR-T cells.

The use of recipient T cells to make CAR T cells in patients who previously received HCT may reduce the risk of GVHD. Assuming these patients were not exhibiting evidence of GVHD at the time of peripheral T-cell collection, those cells would likely either be non-alloreactive or peripherally tolerized. However, this may come at the expense of a poorer T-cell quality resulting from exposure to the stresses of the HCT and cytotoxic agents [Id.].

Overall, several groups have reported clinically meaningful responses in the absence of significant GVHD in patients treated with allogeneic recipient-derived CAR T cells for relapse post-HCT [Id., citing Maude, SL et al. N. Engl. J. Med. (2014) 371: 1507-17; Lee, DW et al. Lancet (2015) 385: 517-28; Turtle, CJ et al. J. Clin. Invest. (2016) 126: 2123-38; Gardner, RA et al. Blood (2017) 129: 3322-31; Fry, TJ et al. Nat. Med. (2018) 24: 20-8; Park, JH et al. N. Engl. J. Med. (2018) 378: 449-59; Maude, SL et al. N. Engl. J. Med. (2018) 378: 439-48; Jain, T. et al. Leukemia (2019) 33: 2540-4; Zhang, X. et al. Blood Adv. (2020) 4: 2325-38; Ramos, CA et al. J. Clin. Oncol. (2020) 38: 3794-804; Wei, G. et al. Ann. Hematol. (2018) 97: 781-9; Frigault, MJ et al. Blood (2019) 134: 860-6]. In all of these studies, the number of patients treated was small and most of these studies did not include patients with active or uncontrolled GVHD.

Consistent with the study by Ghosh et al. [Id., citing Ghosh, A. et al. Nat. Med. (2017) 23: 242-9] suggesting a higher risk for GVHD with the use of the 41BB co-stimulatory domain, the clinical studies that reported the occurrence of GVHD with recipient-derived CAR T-cell therapy utilized second-generation 41BBz.CAR T cells targeting CD19. [Id., citing Turtle, CJ et al. J. Clin. Invest. (2016) 126: 2123-38; Gardner, RA et al. Blood (2017) 129: 3322-31]. In the study by Turtle et al., one out of 11 patients had chronic GVHD, while one out of 27 patients in the study by Gardner et al. had acute GVHD. In the study by Jain et al.[Id., citing Jain, T. et al. Leukemia (2019) 33: 2540-4], a CD28z.CAR T cell was used and one out of four patients had pre-existing chronic GVHD that persisted but was not exacerbated.

### Non-HCT setting CAR-T Therapy

Allogeneic CAR T cells carry two major risks. First, the native TCRs of the donor CAR T cells may recognize and attack normal recipient tissues as foreign resulting in GVHD. Second, there is a risk of recipient immune cells rejecting the infused allogeneic CAR T cells. Different strategies have been proposed to address these obstacles.

To reduce the risk of GVHD, different groups are testing third-party allogeneic donor-derived virus specific T cells (VSTs) engineered to express CARs in patients with relapsed or refractory lymphomas (third-party anti-CD19 CAR VSTs in patients with prior autologous hematopoietic stem cell transplantation: NCT01430390; third-party anti-CD30 CAR VSTs: NCT04288726). This approach was based on encouraging safety and efficacy data from trials using VSTs to effectively treat post-transplant viral infections. [Id., citing Leen, AM et al. Blood (2013) 121: 5113-23; Qasim, W. et al. Br. J. Haematol. (2011) 154: 150-3; Doubrovina, E. et al. Blood (2012) 119: 2644-56; Tzannou, I. et al. J. Clin. Oncol. (2017) 35: 3547-57; Prockop, S. et al. J. Clin. Invest. (2020) 130: 733-47].

In another approach, the native TCR α/β constant (TRAC/TRBC) genes have been disrupted using different gene-editing technologies such as transcription activator-like effector nucleases (TALENS) and clustered regularly interspaced short palindromic repeat (CRISPR)-associated proteins. [Id., citing Ren, J. et al. Clin. Cancer Res. (2017) 23: 2255-66; Cooper, ML et al. Leukemia (2018) 32: 1970-83, Poirot, L. et al. Cancer Res. (2015) 75: 3853-64]. Thus, CAR T cells lacking native TCR expression on their surface are unable to mount an alloreactive immune response against normal tissues of the recipient. Another approach to achieve simultaneous CAR expression and TCR knock-out has employed CRISPR technology to knock-in the CAR-encoding DNA at the TRAC locus, thereby preventing TCRαβ expression. [Id., citing Eyquem, J. et al. Nature (2017) 543: 113-7; Qasim, W. Blood I2o23O 141 (6): 835-844]. Despite achieving high gene-editing efficiencies using these techniques, some unedited, TCR-bearing T cells may persist and can potentially cause GVHD.

To simultaneously reduce the risk of allogeneic CAR T-cell rejection the β2-microglobulin gene (B2M), an essential component of the MHC Class I, has also been disrupted using multiplex gene-editing approaches. [Id., citing Ren, J. et al. Clin. Cancer Res. (2017) 23: 2255-66; Cooper, ML et al. Leukemia (2018) 32: 1970-83, Poirot, L. et al. Cancer Res. (2015) 75: 3853-64]. Other groups have deleted CD52 on the surface of CAR T cells to allow for the use of alemtuzumab (commercially available as CAMPATH^{®} and LEMTRADA^{®} (a humanized monoclonal antibody specific for the CDw52 antigen, present on cell membranes of lymphocytes and monocytes), which otherwise would also have diminished CD52-expressing CAR T cells after infusion) to mediate T-cell depletion in patients before CAR T cell infusion. However, this comes at an increased risk of cytopenias and severe viral infections due to blunting of T cell-mediated responses and has been proposed as a bridge to transplantation. [Id., citing Qasim, W. et al. Br. J. Haematol. (2011) 154: 150-3].

Mo et al. developed another chimeric receptor, called an alloimmune defense receptor (ADR) that recognizes the 4-1BB receptor and is transiently upregulated on the surface of alloreactive T and natural killer (NK) cells. [Mo, F. et al. Nat. Biotechnol. (2021) 39: 56-63]. They reported that engineered T cells co-expressing the ADR and CAR resisted allo-rejection, persisted and retained their anti-tumor activity.

Another group generated anti-timor T cells in which HLA class I and class II as well as endogenous TCR expression were disrupted by genetically ablating B2M, CIITA and TRAC through Cas9/sgRNA electroporation. Although HLA-deficient T cells were targeted by NK cells, they persisted better than HLA-sufficient T cells in the presence of allogeneic PBMCs in immunodeficient mice. When transduced with a CD19 CAR and stimulated by tumor cells, the triple knockout CAR-T cells persisted better when cultured with allogeneic PBMCs compared with TRAC and B2M double knockout T cells. The CD19 triple knockout CART cells did not induce GvHD but retained antitumor responses. [Kagoya, Y. et al. Cancer Immunol. Res. (2020) 8: 926-36].

In yet another strategy to protect allogeneic cell products, Quach et al fused beta-2-microglobulin (B2M) to the cytolytic endodomain of the T cell receptor zeta chain to create an inducible chimeric HLA accessory receptor (CHAR). [Quach, DH et al. J. Transl. Med. (2019) 17: 240]. They found that the CHAR could complex with endogenous human HLA class I molecules and carry them to the cell surface. When expressed in stimulator CMV-specific VSTs, the CHAR could eliminate alloreactive T cells in mixed lymphocyte cocultures with responder allogeneic PBMCs without eliminating pathogen-specific T cells, and in contrast to unmodified VSTs, were protected from allospecific elimination. By eliminating the alloreactive T cells, CHAR-expressing VSTs could prevent the rejection of allogeneic cell therapy products increasing their persistence.

Another similar approach, intended to develop off the shelf allogeneic CAR T cells resistant to host versus graft rejection (HVGR), and unlikely to cause graft versus host disease, included using Ebstein Barr Virsus Specific T cells that expressed a chimeric antigen receptor for CD 30. [Quach DH et. Al., Blood 138 (2021) 1763-64].

In two published clinical trials of 'off-the-shelf allogeneic anti-CD19 CAR T cells in children and adults [Sanber, K. et al. Br. J. Haematology (2021) 195: 660-68, citing Benjamin, R. et al. Lancet (2020) 396: 1885-94] the CAR T cells were edited using TALENS to disrupt the genes encoding TRAC and CD52 [Id., citing Qasim, W. et al. Sci. Transl. Med. (2017) 9: eaaj2013] and alemtuzumab was used for T-cell depletion. Only two out of 14 patients (14%) developed Grade I acute skin GVHD. In all, 14 of 21 patients (67%) achieved complete response (CR) or CR with incomplete hematological recovery, and 10 of 14 responders subsequently received an HCT. Multiple clinical trials are ongoing to further evaluate the safety and efficacy of other multiply edited, 'off-the-shelf allogeneic CAR T cells. [Id., citing Depil, S. et al. Nat. Rev. Drug Discov. (2020) 19: 185-99].

In the autologous setting, the use of lymphodepleting chemotherapy before CAR T cell administration has been associated with greater CAR T-cell expansion and improved outcomes.[ Sanber, K. et al. Br. J. Haematology (2021) 195: 660-68, citing Ramos, CA et al. J. Clin. Oncol. (2020) 38: 3794-804; Hegde, M. et al. Nat. Commun. (2020) 11: 3549; Hirayama, AV, et al. Blood (2019) 133: 1876-87; Qasim, W. Blood (2023) 141 (8): 835-44]. This is thought to be mediated by multiple effects including promoting a favorable homeostatic milieu for the infused CAR T cells to expand [Id., citing Turtle, CJ et al. J. Clin. Invest. (2016) 126: 2123-38; Kochenderfer, JN et al. J. Clin. Oncol. (2017) 35: 1803-13; Turtle, CJ et al. Sci. Transl. Med. (2016) 8: 355ra116] and possibly by reducing anti-CAR T-cell immunity. [Id., citing [Id., citing Turtle, CJ et al. J. Clin. Invest. (2016) 126: 2123-38; Wagner, DL et al. Nt. Rev. Clin. Oncol. (2021) 18: 379-93] However, studies of donor-derived CAR T cells have largely omitted the use of lymphodepleting chemotherapy in part because of the hypothetical increased risk and/or severity of GVHD.

Shrestha, B. et al. developed anti-C83 CAR T cells that effectively prevented/treated GVHD in pre-clinical models and had promising anti-AML activity. [Id., citing J. Clin. Invest. (2020) 130 (9): 4652-62]

Different groups are also investigating the use of other immune cell types that are less prone to causing GVHD in the allogeneic setting as platforms for cellular immunotherapy. These include CAR-engineered NK cells, [Id., citing Xie, G. et al. EBioMedicine (2020) 59: 102975], invariant NK T cells [Id., citing Karadimitris, A. et al. HemaSphere (2019) 3: 31-4] and γδ T cells. [Id., citing Kabelitz, D. et al. Cell Mol. Immunol. (2020) 17: 925-39].

### CD8 T cells in T1D autoimmunity

A number of reports have shown that small molecules, for example, the mTOR inhibitor rapamycin enhances the formation of CD8+ memory T cells and augments their antitumor functions. [Gattinoni, L., et al. Nature Reviews Cancer (2012) 12: 671-84, citing Rao, RR et al. Immunity (2010) 32: 67-78], metformin [Id. citing Pearce, EL et al. Nature (2009) 460: 103-7], and inhibitors of GSK30 can potentiate the WNT-β-catenin signaling pathway in T cells to generate self-renewing multipotent TSCM-like cells [Id., citing Gattinoni, L. Nature Med. (2011) 17: 1290-7; Gattinoni, L. et al. Nature Med. (2009) 15: 808-813]. Although these reagents are effective withholding T cell differentiation and potentiating in vivo antitumor function, they also inhibit T cell proliferation. Inhibition of AKT1 and AKT2 has been shown to inhibit the acquisition of effector molecules and function while preserving a TCM-like phenotype and migratory capacity without a detrimental effect on cell yield [Id., citing Macintyre, AN et al. Immunity (2011) 34: 224-36].

In autoimmunity, self-reactive T cells acquire cytotoxic effector functions and cause destruction of self. [Gearty, SV et al. Nature (2022) 602 (7895): 156-61, citing Bluestone, JA et al. J. Clin. Invest. (2015) 125: 22250-60]. Despite presumed chronic self-antigen exposure on normal tissues, autoimmune T cells do not become dysfunctional, but retain effector functions and progressively destroy the tissue.

The NOD/ShiLtJ (WT NOD) mouse strain (the Jackson Laboratory, www.jax.org/strain/001976) is a polygenic model for autoimmune type 1 diabetes. Diabetes in NOD mice is characterized by hyperglycemia and insulitis, a leukocytic infiltration of the pancreatic islets. Marked decreases in pancreatic insulin content occur in females at about 12 weeks of age and several weeks later in males. A 2022 phenotyping study found that 86% of females and 48% of males became diabetic by 30 weeks of age; median female incidence was 18 weeks. Immune phenotypes in the NOD background consist of defects in antigen presentation, T lymphocyte repertoire, NK cell function, macrophage cytokine production, wound healing, and C5 complement.

Gearty et al. isolated cells from lymph nodes, spleens, and pancreas in order to follow the fate of beta-cell specific CD8 T cells in this model throughout the course of type 1 diabetes. A stem-like autoimmune progenitor population of endogenous beta-cell specific CD8 T cells specific for the beta cell protein islet-specific glucose-6-phosphatase catalytic subunit-related protein (IGRP) amino acid 206-214 epitope [hereinafter IGRP-T cells] was identified and followed in NOD mice over the entire development course of T1D (from 5 to 30 weeks of age).

The data showed that the autoimmune IGRP CD8 T cells were distinct from IGRP CD8 T cells generated after infection. More specifically:

IGRP T cells in NOD mice are primed in the pancreatic draining lymph node (pLN) before they infiltrate the pancreas [Id., citing Anderson, MS and Bluestone, JA. Annu. Rev. Immunol. (2005) 23: 447-85; Ferris, ST et al. Immunity (2014) 41: 657-69].

The IGRP-specific autoimmune T cell population comprised three distinct T cell subsets: pLN TCF1^{hi}, pLN TCF1^{lo}, and pancreatic TCF1^{lo} cells.

pLN IGRP T cells expressed moderate levels of TOX and pancreatic IGRP T cells expressed minimal levels of TOX, in contrast to exhausted and dysfunctional T cells in chronic viral infections and tumors.

IGRP T cells in the pLN segregated into two distinct populations based on TCF1 expression: TCF1^{hi} and TCF1^{lo}. This bifurcation was unique to IGRP T cells in the pLN. pLN TCF1^{hi} cells were enriched for pathways involved in MYC and E2F signaling and DNA replication, while pLN TCF1^{lo} cells were enriched for pathways associated with T cell activation, cytotoxicity and apoptosis.

Pancreatic TCF1^{lo} cells were transcriptionally distinct from pLN TCF1^{lo} cells, indicating that pLN TCF1^{lo} cells continued to differentiate in the pancreas.

Genes that were uniquely expressed in pLN TCF1^{hi} and progressively lost in pLN and pancreatic TCF1^{lo} populations included genes associated with T cell survival, proliferation, and self-renewal. Genes that became progressively upregulated from pLN TCF1^{hi} to pLN TCF1^{lo} to pancreatic TCF1^{lo} cells included *Entpd1* (ectonucleoside triphosphate diphosphohydrolase 1, a plasma membrane protein that hydrolyzes extracellular ATP and ADP to AMP), *Gzmb* (granzyme B), Id2 (inhibitor of DNA binding 2, which affects the gene expression profile of established KLRGlhi effector/effector-memory CD8+ T cells), *Ifng* (IFN-gamma expression), Lag3 (immune checkpoint) and PDccd1 (programmed cell death protein 1, also known as PD-1). Effector genes associated with terminal differentiation and apoptosis were shared by TCF1^{lo} populations.

The pLN TCF1^{hi} cells function as a stem-like population that is able to self-renew and give rise to TCF1^{lo} cells.

When pLN TCF1^{hi} and pancreatic TCF1^{lo} cells were isolated (using Ly108 and CD39 as surrogate markers) and transferred into second NOD/SCID hosts, NOD/SCID hosts that received pLN TCF1^{hi} cells became diabetic whereas hosts that received TCF1^{lo} cells did not. Even as few as 100 TCF1^{hi} cells were sufficient to induce T1D, whereas as many as 10^5 TCF1^{lo} cells were not. Donor TCF1^{hi} cells could be found in all tissues evaluated and gave rise to all three T cell subpopulations, demonstrating that pLN TCF1^{hi} can both self-renew and produce differentiated progeny. In contrast, donor TCF1^{lo} cells could not be detected in any tissue. Serial transplantations (over a 7 month period) to assess long-term self-renewal capacity and sternness of the pLN TCF1^{hi} cells showed that with each transplant, TCF1^{hi} cells gave rise to stable TCF1^{hi} and TCF1^{lo} populations that persisted for at least five months. However, pancreatic TCF^{lo} cells could not persist in the pancreas without continual replenishment by pLN TCF1^{hi} cells.

Single cell T cell receptor sequencing of IGRP CD8 T cells from matched NOD pLN and pancreas showed that in almost all mice, the vast majority of pLN and pancreatic TCF1^{lo} cells harbored clonotypes that were shared with the pLN TCF1^{hi} population, demonstrating that TCF1^{lo} cells are derived from the reservoir of pLN TCF1^{hi} cells.

In conclusion, these studies showed that pLN TCF1^{hi} cells represent a stem-like autoimmune progenitor (AP) population of CD8+ T cells that self-renews long term and gives rise to terminally differentiated pancreatic TCF1^{lo} autoimmune mediators (AM) that destroy beta cells in the pancreastin a mouse model of T1D. The APs are necessary to generate and replenish the pool of pancreatic AMs, which are short-lived and thus unable to independently sustain the prolonged beta cell destruction that causes T1D. The results therefore suggest that the induction and maintenance of diabetogenic T cell responses requires two functionally distinct populations: short-lived pancreatic AM to destroy beta cells and self-renewing pLN AP to replenish the AM population, thereby enabling sustained beta-cell damage that culminates in T1D. The results showed that pancreatic AM are short lived and are constantly replenished by pLN AP instead of undergoing chronic antigen stimulation.

There are few options available for a patient with an advanced solid tumor cancer once treatment has exhausted conventional therapies including checkpoint inhibitors (CPIs). Even chimeric antigen receptor T cell (CAR-T) therapies, which have proved so successful in some leukemias and lymphomas, have not been effectively applied to solid tumors; indeed, the multiple mechanisms used by solid tumors to suppress tumor-specific immune responses are a major barrier to the success of adoptive transfer of tumor-specific T cells. There is therefore a great unmet need for new therapies for such solid tumors.

The present disclosure provides a scalable and practical off-the-shelf allogeneic T cell therapy for treating cancer including employing a population of allogeneic viral specific T cells comprising a TCF-1^{High} TOX^{Lo} early stem cell T_{scm} phenotype that is genetically modified to stably express one, two or three chimeric antigen-receptors (CARs), wherein each CAR is specific for a cancer antigen; and wherein one of the CARs comprises a CD30 CAR expressing a CD30-specific scFv fragment, and/or a chimeric alloimmune defense receptor (ADR); and /or a chimeric HLA accessory receptor fused with a cytolytic endodomain of the T cell receptor zeta chain, thereby reducing risk of host versus graft rejection (HVGR), to create a population of cancer killing off the shelf allogeneic CD3⁺ Viral Specific TCF-1^{High} TOX^{Lo} -CAR-T cells.

### SUMMARY OF THE INVENTION

According to one aspect, an (off-the shelf) allogeneic CAR-T cell immunotherapy is provided, suitable for treating a cancer. A composition of the invention comprises, or is prepared by the following method, comprising one or more of the following step(s) or features:
a) creating a population of allogeneic multivirus-specific T cells (MV-VSTs), suitably comprising a (reduced risk of) graft versus host disease (GVHD), such as by transducing allogeneic T cells with a viral vector (encoding a protein of a virus), wherein the population of VSTs can comprise T cell receptors (TCRs) specific for the protein of the virus;
b) creating a population of cancer killing VST-CAR-T cells (by genetically modifying the population of allogeneic VSTs) to stably express up to three chimeric antigen-receptors (CARs), wherein each CAR can be specific for a cancer antigen, suitably to reduce risk of host versus graft rejection (HVGR); suitably one or more of the CARs comprise: (i) a-CD30 CAR expressing a CD30-specific scFv fragment, or (ii) a chimeric alloimmune defense receptor (ADR), or (iii) a chimeric HLA accessory receptor fused with a cytolytic endodomain of T cell receptor zeta chain; or (iv) a CD30 CAR expressing a CD30-specific scFv fragment, and a chimeric HLA accessory receptor fused with a cytolytic endodomain of T cell receptor zeta chain; or (v) a CD30 CAR expressing a CD30-specific scFv fragment, and a chimeric alloimmune defense receptor (ADR); or (vi) a chimeric alloimmune defense receptor (ADR), and a chimeric HLA accessory receptor fused with a cytolytic endodomain of T cell receptor zeta chain; and/or (vii) a combination thereof;
c) suitably expanding the population of allogeneic VST-CAR-T cells in (b), such as in the presence of one or more cytokines (in vitro), preferably to achieve a therapeutic dose from or of at least 40 × 10⁶ and/or up to 800 × 10⁶ cancer killing T cells; and/or
d) (the composition being suitable for) infusing an eligible patient with the allogeneic VST-CAR-T cells, such as in (c), one or more times in an allogeneic setting; and optionally e) creating VST-CAR-T cells that express a nonexhausted PD-1^{neg} TIGIT^{neg} TCF-1^{hi}TOX^{lo} early T stem cell (T_{SCM}) phenotype, suitably by: (i) enriching for a population of CD4+ T cells, CD8+ T cells or a combination thereof that express a nonexhausted PD-1_{neg} TIGIT^{neg} TCF-1^{hi}TOX^{lo} early T stem cell (T^{SCM}) phenotype; (ii) generating by transducing the population of CD4+ T cells, CD8+ T cells or a combination thereof with a viral vector encoding a protein of a virus an expanded population of allogeneic virus-specific T cells (VSTs) comprising T cell receptors (TCRs) specific for the protein of the virus; and/or (iii) genetically modifying the population of allogeneic VSTs in (ii) to stably express up to three chimeric antigen-receptors (CARs), wherein each CAR is specific for a cancer antigen, with the aim to reduce risk of host versus graft rejection (HVGR). One or more of the CARs can comprise: (1) a CD30 CAR expressing a CD30-specific scFv fragment, or (2) a chimeric alloimmune defense receptor (ADR), or (3) a chimeric HLA accessory receptor fused with a cytolytic endodomain of T cell receptor zeta chain, or (4) a CD30 CAR expressing a CD30-specific scFv fragment, and a chimeric HLA accessory receptor fused with a cytolytic endodomain of T cell receptor zeta chain; or (5) a CD30 CAR expressing a CD30-specific scFv fragment, and a chimeric alloimmune defense receptor (ADR); or (6) a chimeric alloimmune defense receptor (ADR), and a chimeric HLA accessory receptor fused with a cytolytic endodomain of T cell receptor zeta chain;and/or (7) a combination thereof; and optionally f) iteratively dosing the patient with the PD-1^{neg} TIGIT^{neg} TCF-1^{hi}TOX^{lo} early stem cell T cell (T_{SCM}) phenotype VST-CAR-T of step (e), suiatably as needed (until all cancer in the body is destroyed).

According to some embodiments, the allogeneic T cells of step (a) can be derived from umbilical cord blood (UCB); or from adult peripheral blood (PB); and/or from adult peripheral blood (PB), suitably after mobilization of peripheral blood stem cells with an infusion of G-CSF.

According to some embodiments, CD3+ allogeneic T cells can be isolated from the allogeneic T cells (by negative selection).

According to some embodiments, step (a) can comprise transducing CD3+ allogeneic T cells with a viral vector, suitably encoding proteins of one or more viruses.

According to some embodiments, the viruses are selected from one or more of the viruses in table 5.

According to some embodiments, in step (b) and/or step (e) the VSTs can be transduced by a retroviral vector, comprising a nucleic acid encoding a synthetic chimeric antigen receptor (CAR), that may specifically bind the cancer antigen, such as to stably express the cancer antigen-specific CAR.

According to some embodiments, the cancer antigen is a tumor associated antigen or a tumor selective antigen; and for each cancer antigen, the CAR may comprise an extracellular antigen recognition domain suitably comprising a single-chain variable fragment (scFv) derived from a monoclonal antibody specific for the antigen, optionally a spacer/hinge region and transmembrane domain; and/or an intracellular signal transduction domain comprising a CD3ζ T cell activation chain and optionally one or more costimulatory molecule(s).

According to some embodiments, the alloimmune defense receptor (ADR) may comprise an anti 4-1BB receptor; the HLA accessory receptor can comprise a beta2-microglobulin fused with the cytolytic endodomain of the T cell receptor zeta chain; and/or the alloimmune defense receptor (ADR) is an anti 4-1BB receptor and/or the HLA accessory receptor comprises beta2-microglobulin fused with the cytolytic endodomain of the T cell receptor zeta chain.

According to some embodiments, the tumor-associated antigen is selected from table 6.

According to some embodiments, the tumor specific antigen is encoded by a gene from table 7.

According to some embodiments, in step (c), the cytokine is one or more of IL-2, IL-7, and/or IL-15.

According to some embodiments, in step (d), the recipient eligible patient may undergo infusion of the population of VST-CAR-T cells following lymphodepleting chemotherapy.

According to some embodiments, the lymphodepleting chemotherapy comprises cyclophosphamide (CYTOXAN^{®}) therapy (500-600 mg/m2 for 3 days) and/or fludarabine (20 mg/m2), such as for 3-4 days, optionally with a stagger of up to 7 days between participants (e.g. to evaluate dose limiting toxicities or an equivalent clinical regimen).

According to some embodiments, to suitably further decrease the risk of the VST-CAR-T cells being killed by the recipient's cells, the recipient eligible patient can be at least partially HLA matched (against HLA stocks of the VST-CAR-T cells).

According to some embodiments, in step (e), the population of CD4+ T cells, CD8+ T cells or both CD4+ T cells and CD8+ T cells, expressing a nonexhausted phenotype comprising a PD-1^{neg}TIGIT^{neg}TCF-1^{hi}TOX^{lo} early stem cell (T_{SCM}) phenotype, can be derived from umbilical cord blood (UCB) and/or are derived from healthy donor adult peripheral blood.

According to some embodiments, the recipient can be at least partially HLA matched against the umbilical cord blood and/or adult peripheral blood.

According to some embodiments, the population of CD4+ T cells, CD8+ T cells, or both CD4+ and CD8+ T cells is/are isolated from mononuclear cells, e.g. by negative selection.

According to some embodiments, the population of CD4+ T cells, CD8+ T cells and/or both CD4+ T cells and CD8+ T cells expressing a nonexhausted phenotype comprising a PD1^{neg}TIGIT^{neg}TCF-1^{hi}TOX^{lo} early stem cell (T_{SCM}) phenotype (a) can be expanded in vitro in the presence of one or more cytokines; or (b) is expanded in vitro in the presence of an epigenetic modification agent; and/or (c) is expanded in vitro in the presence of one or more cytokines and an epigenetic modification agent.

According to some embodiments, the epigenetic modification agent is selected from belinostat, vorinostat, romidepsin, panobinostat, decitabine, and/or azacitidine.

According to some embodiments, the cytokine is at least two cytokines selected from IL-7, IL-15, and/or IL-21.

According to some embodiments, an expanded nonexhausted population of CD4+ T cells, CD8+ T cells, or CD4+ T cells and CD8+ T cells expressing a nonexhausted phenotype comprising a PD-1^{neg}TIGIT^{neg}TCF-1^{hi}TOX^{lo} early stem cell (T_{SCM}) phenotype of step (e) can be administered to the subject periodically as needed.

According to some embodiments, the method further includes administering an (approved) immune checkpoint inhibitor (at a dose standard for the cancer indication).

According to some embodiments, the checkpoint inhibitor includes anti-PD1 (e.g., lambrolizumab, pembrolizumab (KEYTRUDA^{®}) and nivolumab (OPDIVO^{®}), anti-PD-L1 (MPDL3280A, Atezolizumab, TECENTRIQ^{®}), and/or anti-CTLA4 (ipilimumab, YERVOY^{®}).

According to some embodiments, the cancer is a solid tumor, a leukemia, a lymphoma, or a plasma cell dyscrasia (myeloma).

According to some embodiments, the population of CD4+ T cells, CD8+ T cells or both CD4+ T cells and CD8+ T cells expressing a nonexhausted phenotype comprising a PD1^{neg}TIGIT^{neg}TCF-1^{hi}TOX^{lo} early stem cell (T_{SCM}) phenotype is genetically engineered: a. to overexpress FOXO1; or b. by biallelic disruption of TET2 via CAR integration into intron 9; or c. by deleting DNMT3A; or d. by disrupting histone methyltransferase SUV39H1; or e. by generating the CAR-T cells in presence of exogenous transforming factor beta; or f. by exposure to Iosine; or g. by EZH2 inhibition and overexpression of c-Jun; and/or h. or by a combination thereof. The invention in a second aspect relates to a composition prepared by, or preparable by, a method as previously described (for the first aspect). The composition may be used in a method of treating cancer. Preferred features and/or characteristics of one aspect of the invention are applicable to another aspect *mutatis mutandis.*

### BRIEF DESCRIPTION OF THE DRAWINGS

**FIG. 1** is a schematic showing the role of CD4+ T cells in modulating immune responses. (a) CD4+ T cells provide crucial help in the priming of CD8+ T cells via activation of antigen-presenting cells. (b) CD4+ T cells secrete cytokines required for maintaining CD8+ T cell function and proliferation. (c) CD4+ T cells can inhibit tumor cell growth directly or indirectly; (d) CD4+ T cells provide help for B cell activation. Taken from Wang, R-F , Trends in Immunology (2001) 22 (5): 269-76, Fig. 1].
**FIG. 2** is a schematic showing pathways to antigen presentation [taken from Yewdell, JW and Dolan, BP. Nature (2011) 471 (7340): 581-82]. (a) Direct presentation occurs when an APC is infected and displays processed antigenic peptides in complex with MHC class I molecules on its surface, thereby activating T cells. (b) In cross-presentation, dendritic cells acquire antigens obtained by infected cells through endocytosis and phagocytosis and, with or without some processing, load them onto class I molecules for presentation to T cells. (c) In cross-dressing, dendritic cells acquire preformed MHC class I molecules in complex with antigens from other cells by the process of trogocytosis or through gap junctions.
**FIG. 3** is a schematic illustrating a hierarchical developmental pathway for CD8+ T cell exhaustion, revealing four stages and multistep transcriptional and epigenetic dynamics underlying subset transitions and subset-associated biological changes. [Taken from Beltra, J-C et al. (2020) Immunity 52: 825-41].

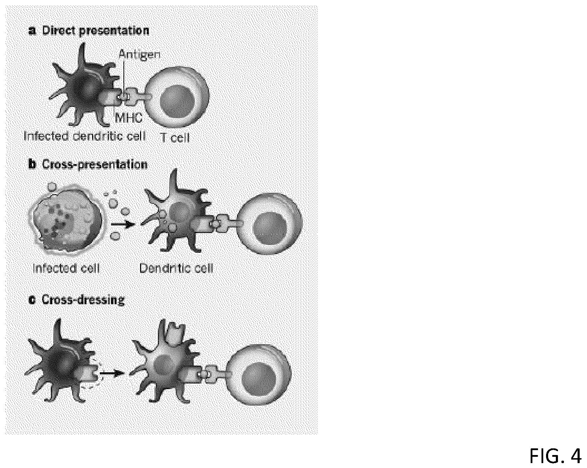

**FIG. 4** is a schematic depicting, from left to right, structural changes from the first generation CAR to the fourth generation chimeric antigen receptors (CARs). Each CAR consists of three parts: an extracellular antigen recognition domain; a hinge region and transmembrane domain (CD8), and an intracellular signal transduction domain. The intracellular signal transduction domain of the first generation CAR includes a CD3 zeta chain; the intracellular signal transduction domain of the second generation or 4^{th} generation CAR includes the CD3 zeta chain and one co-stimulatory molecule; the intracellular signal transduction domain of the third generation CAR contains two costimulatory molecules in addition to the CD3 zeta chain. The fourth generation CAR also expresses a cytokine, such as IL-12. [Taken from Feng, D. and Sun, J. Scand. J. Immunol. (2020) 92: e12910].

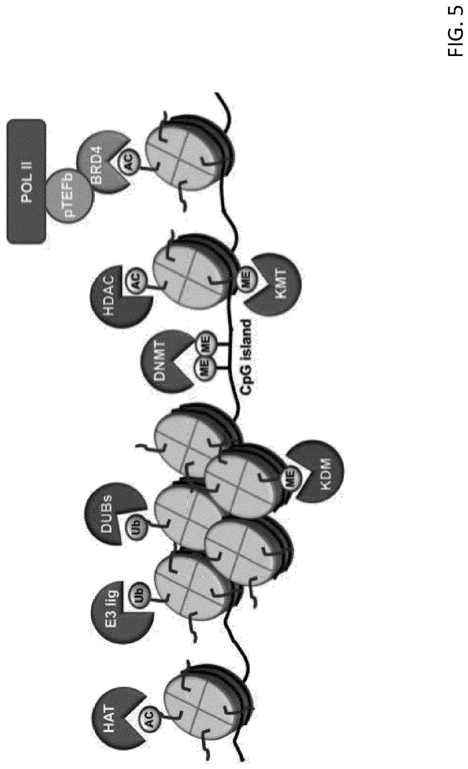

**FIG. 5** is an illustration depicting sites of dynamic regulation of chromatin structure in eukaryotic cells by DNA methylation, nucleosome positioning, and histone modifications. [Taken from Bennett, RL and Licht, JD. Annu. Rev. Pharmacol. Toxicol. (2018) 58: 187-207]. Key: ac-acetyl group; me=methyl group; Ub=ubiquitin-modified protein; bromodomain-containing protein 4 (BRD4); deubiquitinating enzymes (DUB); DNA methyltransferases (DNMT); e3 ligase (e3 lig.); histone acetyltransferase (HAT); histone deacetylase (HDAC; histone lysine demethylases (KDMs); lysine methyl transferases (KMTs); positive transcription elongation factor (P-TEFb); RNA polymerase II (POL II).

### DETAILED DESCRIPTION OF THE INVENTION

### Glossary

The term "4-1BB" or "CD137" (or tumor necrosis factor receptor super family 9) as used herein refers to an inducible costimulatory receptor expressed on activated T and natural killer (NK) cells. [Chester, C. et al. Blood (2018) 131 (1): 49-57]. There is wide expression of 4-1BB throughout the hematopoietic and nonhematopoietic compartments. 4-1BB is expressed on DC activated monocytes and NK cells, neutrophils, eosinophils and mast cells. [Id., citing Wilcox, RA et al. J. Immunol. (2002) 168 (9): 4262-67; Heinisch IV, et al. Eur. J. Immunol. (2000) 39 (120): 3441-6; Schwarz, H. et al. Blood (1995) 85 (4): 1043-52].

On T cells, 4-1BB is transiently expressed after T-cell receptor engagement and, when 4-1BB is engaged by its natural or artificial ligand, provides CD28-independent costimulation resulting in enhanced proliferation and T_{H}1 cytokine production. [Id., citing DeBenedette, MA e al. J. Immunol. (1997) 158 (2): 551-9; Saoullli, K. et al. J. Exp. Med. (1998) 187 (11): 1849-62]. The major biological ligand of 4-1BB, 4-1BBL, is expressed on activated APCs, including dendritic cells (DCs) and macrophages and B cells [Id., citing Goodwin, RG et al. Eur. J. Immunol. (1993) 23 (10): 2631-41; Alderson, MR et al. Eur. J. Immunol. (1994) 24 (9): 2219-27; Futagawa, T. et al. Intl Immunol. (2002) 14 (3): 275-86; Pollok, KE et al. Eur. J. Immunol. (1994) 24 (2): 367074]. Ligation of 4-1BB recruits TNFR-associated factor (TRAF) 1 and TRAF2 and induces signaling through the master transcription factor NF-κB and MAPKs. [Id., citing Lee, DY et al. PLoS One (2013) 8 (7): e69677; Vinay, DS and Kwon, BS. Semin. Immunol. (1998) 10 (6): 481-9]. Upon ligation with agonist mAbs, 4-1BB rapidly internalizes to an endosomal compartment from which it keeps signaling through this pathway. [Id., citing Bradley, JR and Pober, JS. Oncogene (2001) 20 (44): 6482-91; Kim, CM et al. Sci Rep. (2016) 6 (1): 25526]. 4-1BB signaling ultimately contributes to the secretion of IL-2 and IFN-gamma and upregulation of the antiapoptotic Bcl-2 family members Bcl-xL and Bfl-1, which provide strong protection against activation-induced T cell death. [Id., citing Hurtado, JC et al. J. Immunol. (1997) 158 (6): 2600-9 see. Lee, H-W et al. J. Immunol. (2002) 169 (9): 4882-8; Maus, MV et al. Nat. Biotechnol. (2002) 20 (2): 143-8; Takahashi, C. et al. J. Immunol. (1999) 162 (9): 5037-40]. Even though 4-1BB and CD28 costimulation are said to be functionally independent, CD28 costimulation is a powerful stimulus for 4-1BB upregulation,

Upon Fc-receptor triggering, NK cells upregulate 4-1BB and increase cytotoxic function in response to 4-1BB agonism, but 4-1BB agonism on resting NK cells may reduce NK cell frequency and compromise NK cell cytotoxic function. [Id., citing Choi, BK et al. J. Immunol. (2010) 185 (30): 1404-11; Lee, S-H and Hahm, D. J. Immunol. (2016) 196 (1 suppl.) Abstract 61.4; Chester, C. et al. Cancer Immunol. Immunother. (2016) 65 (10): 1243-8]. Endothelial cells also can upregulate 4-1BB following stimulation with TNF alpha, lipopolysaccharide, and IL-1beta. Expression of 4-1BB along blood vessel walls at the sites of inflammation, chiefly including tumor microvasculature and atherosclerotic areas, suggests that 4-1BB may mediate leukocyte extravasation and migration. [Id., citing Drenkard, D. et al. FASEB J. (2007) 21 (20: 456-63; Broll, K. et al. Am J. Clin. Pathol. (2001) 115 (4): 543-9; Teijeira, A. et al. FASEB J. (2012) 26(8): 3380-92]. Tregs also express 4-1BB, but its role on this immune subset remains poorly understood. [Id., citing Smith, SE et al. Cancer Immunol. Immunother. (2011) 60 (12): 1775-87; Zhang, P. et al. Scand. J. Immunol. (2007) 66 (4): 435-40].

Endothelial cells in hypoxic blood vessels within tumors can upregulate 4-1BB in a hypoxia-inducible factor 1-alpha mediated fashion. [Id., citing Palazon, A. et al. Cancer Res. (2011) 71 (30: 801-11]. In this compartment, anti-4-1BB antibodies give rise to an increased expression of homing receptors for T-cell infiltration. 4-1BB signaling can break and reverse established anergy in cytotoxic T lymphocytes. 4-1BB signaling also plays a role in restoring the functionality of exhausted CD8+ T cells. [Id., citing Williams, JB et al. J. Exp. Med. (2017) 214 (2): 381-400]. On NK cells, 4-1BB signaling can increase antibody-dependent cell mediated cytotoxicity. Agonistic monoclonal antibodies targeting 4-1BB can lead to tumor clearance and durable antitumor immunity. [Id. citing Kohrt, HE et al. Blood (2011) 117 (8): 2423-32; Kohrt, HE et al. J. Clin. Invest. (2012) 122 (3): 1066-75; Kohrt, HE et al. J. Clin. Invest. (2014) 124 (6): 2668-82].

The term "adoptive immunotherapy" also known as "cellular immunotherapy" is a type of immunotherapy in which T cells are given to a recipient patient to help the body fight diseases. Types of adoptive cell therapy include chimeric antigen receptor (CAR) T-cell therapy and tumor-infiltrating lymphocyte (TIL) therapy.

The term "adaptor protein" as used herein refers to a protein that contains a series of protein-binding sites that link respective interaction partners to each other and facilitate the generation of larger signaling complexes.

The term "adverse event" or "AE" as used herein refers to an unfavorable medical event that occurs in a subject who is given a therapeutic product, but does not necessarily have a causal relationship with the treatment. It may be any adverse and unwanted signs (including an abnormal laboratory result), symptoms, or temporary illness associated with the use of the product, whether or not it is related to the product. The correlation between adverse events and test medications is: affirmative, likely related, may be relevant, may be irrelevant, and certainly not relevant. The severity of adverse events according to the National Cancer Institute's Common Terminology Criteria for Adverse Events (CTCAE) Scale is showing in Table 2 below.

**Table 2. Severity of Adverse Events**

| **CTCAE Scale** | **Description** |
|---|---|
| Mild (Grade 1) | Asymptomatic or mild; or only seen clinically or diagnostically; or no treatment required |
| Moderate (Grade 2) | Requires minor, local or non-invasive treatment; age-appropriate instrumental activities of daily life are limited |
| Severe (Grade 3) | Serious or medically significant but not immediately life-threatening; resulting in hospitalization or prolonged hospital stay; disability; limited daily activities of the individual |
| Life Threatening (Grade 4) | Life-threatening; urgent treatment required |
| Death (Grade 5) | Death associated with AE |

The term "allele" as used herein refers to any of one or more alternative forms of a given gene.

The term "alloantigen" as used herein refers to an antigen from a genetically different individual of the same species.

The term "allogeneic" as used herein refers to being derived from a genetically different individual of the same species.

The term "alloimmune defense receptor" or "ADR" as used herein refers to a receptor that, when expressed by allogeneic T cells, mediates deletion of activated host T and NK cells, preventing rejection of allogeneic T cells by activated lymphocytes, while sparing resting lymphocytes. [Mo, et al. Nat. Biotechnol. (2021) 39 (1): 56-63]. For example, a CD30 CAR expressing a CD30-specific scFv fragment is an ADR [see Savoldo, B. et al. Blood (2007) 110 (7): 2620-30].

The term "alloreactive" as used herein refers to a strong primary T cell response against allelic variants of major histocompatibility complex (MHC) molecules in a species.

The term "anergy" as used herein refers to a state of lymphocyte nonresponsiveness to specific antigen induced by an encounter of the lymphocyte with cognate antigen under less than optimal conditions, such as in the absence of costimulation.

As used herein, the term "antibody" includes, by way of example, both naturally occurring and non-naturally occurring antibodies. Specifically, the term "antibody" includes polyclonal antibodies and monoclonal antibodies, and fragments thereof. Furthermore, the term "antibody" includes chimeric antibodies and wholly synthetic antibodies, and fragments thereof.

As used herein, the term "antibody" is used in the broadest sense and encompasses various antibody structures, including but not limited to monoclonal antibodies, polyclonal antibodies, antibody fragments, chimeric antibodies and wholly synthetic antibodies as long as they exhibit the desired antigen-binding activity. In nature, antibodies are serum proteins the molecules of which possess small areas of their surface that are complementary to small chemical groupings on their targets. These complementary regions (referred to as the antibody combining sites or antigen binding sites) of which there are at least two per whole antibody molecule, and in some types of antibody molecules ten, eight, or in some species as many as 12, may react with their corresponding complementary region on an antigen (the antigenic determinant or epitope) to link several molecules of multivalent antigen together to form a lattice. The basic structural unit of a whole antibody molecule consists of four polypeptide chains, two identical light (L) chains (each containing about 220 amino acids) and two identical heavy (H) chains (each usually containing about 440 amino acids). The two heavy chains and two light chains are held together by a combination of noncovalent and covalent (disulfide) bonds. The molecule is composed of two identical halves, each with an identical antigen-binding site composed of the N-terminal region of a light chain and the N-terminal region of a heavy chain. Both light and heavy chains usually cooperate to form the antigen binding surface.

The basic structural unit of a whole antibody molecule consists of four polypeptide chains, two identical light (L) chains (each containing about 220 amino acids) and two identical heavy (H) chains (each usually containing about 440 amino acids). The two heavy chains and two light chains are held together by a combination of noncovalent and covalent (disulfide) bonds. The molecule is composed of two identical halves, each with an identical antigen-binding site composed of the N-terminal region of a light chain and the N-terminal region of a heavy chain. Both light and heavy chains usually cooperate to form the antigen binding surface.

Human antibodies show two kinds of light chains, κ and λ; individual molecules of immunoglobulin generally are only one or the other. In mammals, there are five classes of antibodies, IgA, IgD, IgE, IgG, and IgM, each with its own class of heavy chain. All five immunoglobulin classes differ from other serum proteins in that they show a broad range of electrophoretic mobility and are not homogeneous. This heterogeneity - that individual IgG molecules, for example, differ from one another in net charge - is an intrinsic property of the immunoglobulins.

The principle of complementarity, which often is compared to the fitting of a key in a lock, involves relatively weak binding forces (hydrophobic and hydrogen bonds, van der Waals forces, and ionic interactions), which are able to act effectively only when the two reacting molecules can approach very closely to each other and indeed so closely that the projecting constituent atoms or groups of atoms of one molecule can fit into complementary depressions or recesses in the other. Antigen-antibody interactions show a high degree of specificity, which is manifest at many levels. Brought down to the molecular level, "specificity" means that the combining sites of antibodies to an antigen have a complementarity not at all similar to the antigenic determinants of an unrelated antigen. Whenever antigenic determinants of two different antigens have some structural similarity, some degree of fitting of one determinant into the combining site of some antibodies to the other may occur; this phenomenon gives rise to cross-reactions. Cross reactions are of major importance in understanding the complementarity or specificity of antigen-antibody reactions. Immunological specificity or complementarity makes possible the detection of small amounts of impurities/contaminations among antigens.

Monoclonal antibodies (mAbs) can be generated by fusing mouse spleen cells from an immunized donor with a mouse myeloma cell line to yield established mouse hybridoma clones that grow in selective media. A hybridoma cell is an immortalized hybrid cell resulting from the in vitro fusion of an antibody-secreting B cell with a myeloma cell. In vitro immunization, which refers to primary activation of antigen-specific B cells in culture, is another well-established means of producing mouse monoclonal antibodies.

Diverse libraries of immunoglobulin heavy (V_{H}) and light (V_{L}=V_{κ} and V_{λ}) chain variable genes from peripheral blood lymphocytes also can be amplified by polymerase chain reaction (PCR) amplification. Genes encoding single polypeptide chains in which the heavy and light chain variable domains are linked by a polypeptide spacer (single chain Fv or scFv) can be made by randomly combining heavy and light chain V-genes using PCR. A combinatorial library then can be cloned for display on the surface of filamentous bacteriophage by fusion to a minor coat protein at the tip of the phage.

The technique of guided selection is based on human immunoglobulin V gene shuffling with rodent immunoglobulin V genes. The method entails (i) shuffling a repertoire of human V_{L} chains with the heavy chain variable region (V_{H}) domain of a mouse monoclonal antibody reactive with an antigen of interest; (ii) selecting half-human Fabs on that antigen (iii) using the selected V _{L} genes as "docking domains" for a library of human heavy chains in a second shuffle to isolate clone Fab fragments having human light chain genes; (v) transfecting mouse myeloma cells by electroporation with mammalian cell expression vectors containing the genes; and (vi) expressing the V genes of the Fab reactive with the antigen as a complete IgG1 antibody molecule in the mouse myeloma.

The term antibody may include an oligoclonal antibody, a polyclonal antibody, a monoclonal antibody, a chimeric antibody, a CDR-grafted antibody, a multi-specific antibody, a bi-specific antibody, a catalytic antibody, a chimeric antibody, a humanized antibody, a fully human antibody, an anti-idiotypic antibody, and an antibody that can be labeled in soluble or bound form, as well as fragments, variants or derivatives thereof, either alone or in combination with other amino acid sequences provided by known techniques.

An antibody may be from any species. The term antibody also includes binding fragments of the antibodies of the invention. Binding fragments of an antibody can be produced by recombinant DNA techniques, or by enzymatic or chemical cleavage of intact antibodies. Exemplary fragments include Fv, Fab, Fab', single stranded antibody (svFC), dimeric variable region (Diabody) and di-sulfide stabilized variable region (dsFv). Structural and functional domains can be identified by comparison of the nucleotide and/or amino acid sequence data to public or proprietary sequence databases. For example, computerized comparison methods can be used to identify sequence motifs or predicted protein conformation domains that occur in other proteins of known structure and/or function. Methods to identify protein sequences that fold into a known three-dimensional structure are known. See, for example, Bowie et al. Science 253:164 (1991), which is incorporated by reference in its entirety. An antibody other than a "bispecific" or "bifunctional" antibody is understood to have each of its binding sites identical.

The term "antibody construct" as used herein refers to a polypeptide comprising one or more the antigen-binding portions linked to a linker polypeptide or an immunoglobulin constant domain. Linker polypeptides comprise two or more amino acid residues joined by peptide bonds and are used to link one or more antigen-binding portions. Such linker polypeptides are well known in the art (see e.g., Holliger, P., et al. (1993) Proc. Natl. Acad. Sci. USA 90:6444-6448; Poljak, R.J., et al. (1994) Structure 2: 1121-1123). An immunoglobulin constant domain refers to a heavy or light chain constant domain. Human IgG heavy chain and light chain constant domain amino acid sequences are known in the art. Antibody portions, such as Fab and F(ab')2 fragments, can be prepared from whole antibodies using conventional techniques, such as papain or pepsin digestion, respectively, of whole antibodies. Moreover, antibodies, antibody portions and immunoadhesion molecules can be obtained using standard recombinant DNA techniques.

The term "antigen" as used herein refers to a molecule containing one or more epitopes (either linear, conformational or both) that will stimulate a host's immune-system to make a humoral and/or cellular antigen-specific response. The term is used interchangeably with the terms "immunogen" or "epitope." Normally, a B-cell epitope will include at least about 5 amino acids but can be as small as 3-4 amino acids. A T-cell epitope, such as a CTL epitope, will include at least about 7-9 amino acids, and a helper T-cell epitope will include at least about 12-20 amino acids. Normally, an epitope will include between about 7 and 15 amino acids, such as, 7, 8, 9, 10, 11, 12, 13, 14, or 15 amino acids. The term includes polypeptides which include modifications, such as deletions, additions and substitutions (generally conservative in nature) as compared to a native sequence, as long as the protein maintains the ability to elicit an immunological response, as defined herein. These modifications may be deliberate, as through site-directed mutagenesis, or may be accidental, such as through mutations of hosts which produce the antigens.

The term "antigen binding site" as used herein refers to the structure formed at the amino-terminal ends (variable domains) of the light and heavy chains, which are folded to form 3-dimensional (globular) variable domains, V_{H} and V_{L}. The antigen binding site makes physical contact with the antigen and binds it noncovalently. The antigen specificity of the site is determined by its shape and the amino acids present.

The term "antigen presentation" as used herein, generally refers to the display to a T cell of antigen on the surface of a cell, e.g., in the form of peptide fragments bound to MHC molecules. **FIG. 4** diagrammatically illustrates the pathways to antigen presentation [taken from Yewdell, JW and Dolan, BP. Nature (2011) 471 (7340): 581-82].

As used herein, the term "antigen presenting cell (APC)" refers to a class of cells capable of displaying on its surface ("presenting") one or more antigens in the form of peptide-MHC complex recognizable by specific effector cells of the immune system, and thereby inducing an effective cellular immune response against the antigen or antigens being presented. Examples of professional APCs are dendritic cells and macrophages, though any cell expressing MHC Class I or II molecules can potentially present peptide antigen. An APC can be an "artificial APC," meaning a cell that is engineered to present one or more antigens. Before a T cell can recognize a foreign protein, the protein has to be processed inside an antigen presenting cell or target cell so that it can be displayed as peptide-MHC complexes on the cell surface.

As used herein the term "antigen processing" refers to the intracellular degradation of foreign proteins into peptides that can bind to MHC molecules for presentation to T cells.

The term "apheresis" as used herein refers to a medical technology in which the blood of a donor or patient is passed through an apparatus that separates out one particular constituent and returns the remainder back to the donor or patient's circulation. Leukapheresis is one type of apheresis where leukocytes (white blood cells) are selectively removed.

The terms "apoptosis" or "programmed cell death" refer to a highly regulated and active process that contributes to biologic homeostasis comprising a series of biochemical events that lead to a variety of morphological changes, including blebbing, changes to the cell membrane, such as loss of membrane asymmetry and attachment, cell shrinkage, nuclear fragmentation, chromatin condensation, and chromosomal DNA fragmentation, without damaging the organism.

Apoptotic cell death is induced by many different factors and involves numerous signaling pathways, some dependent on caspase proteases (a class of cysteine proteases) and others that are caspase independent. It can be triggered by many different cellular stimuli, including cell surface receptors, mitochondrial response to stress, and cytotoxic T cells, resulting in activation of apoptotic signaling pathways.

The caspases involved in apoptosis convey the apoptotic signal in a proteolytic cascade, with caspases cleaving and activating other caspases that then degrade other cellular targets that lead to cell death. The caspases at the upper end of the cascade include caspase-8 and caspase-9. Caspase-8 is the initial caspase involved in response to death domain (DD) containing receptors like Fas.

Receptors in the TNF receptor family are associated with the induction of apoptosis, as well as inflammatory signaling. The Fas receptor (CD95) mediates apoptotic signaling by Fas-ligand expressed on the surface of other cells. The Fas-FasL interaction plays an important role in the immune system and lack of this system leads to autoimmunity, indicating that Fas-mediated apoptosis removes self-reactive lymphocytes. Fas signaling also is involved in immune surveillance to remove transformed cells and virus infected cells. Binding of Fas to oligimerized FasL on another cell activates apoptotic signaling through a cytoplasmic domain termed the death domain (DD) that interacts with signaling adaptors including FAF, FADD and DAX to activate the caspase proteolytic cascade. Caspase-8 and caspase-10 first are activated to then cleave and activate downstream caspases and a variety of cellular substrates that lead to cell death.

Mitochondria participate in apoptotic signaling pathways through the release of mitochondrial proteins into the cytoplasm. Cytochrome c, a key protein in electron transport, is released from mitochondria in response to apoptotic signals, and activates Apaf-1, a protease released from mitochondria. Activated Apaf-1 activates caspase-9 and the rest of the caspase pathway. Smac/DIABLO is released from mitochondria and inhibits inhibitor of apoptosis (IAP) proteins that normally interact with caspase-9 to inhibit apoptosis. Apoptosis regulation by Bcl-2 family proteins occurs as family members form complexes that enter the mitochondrial membrane, regulating the release of cytochrome c and other proteins. TNF family receptors that cause apoptosis directly activate the caspase cascade, but can also activate Bid, a Bcl-2 family member, which activates mitochondria-mediated apoptosis. Bax, another Bcl-2 family member, is activated by this pathway to localize to the mitochondrial membrane and increase its permeability, releasing cytochrome c and other mitochondrial proteins. Bcl-2 and Bcl-xL prevent pore formation, blocking apoptosis. Like cytochrome c, AIF (apoptosis-inducing factor) is a protein found in mitochondria that is released from mitochondria by apoptotic stimuli. While cytochrome c is linked to caspase-dependent apoptotic signaling, AIF release stimulates caspase-independent apoptosis, moving into the nucleus where it binds DNA. DNA binding by AIF stimulates chromatin condensation, and DNA fragmentation, perhaps through recruitment of nucleases.

The mitochondrial stress pathway begins with the release of cytochrome c from mitochondria, which then interacts with Apaf-1, causing self-cleavage and activation of caspase-9. Caspase-3, -6 and-7 are downstream caspases that are activated by the upstream proteases and act themselves to cleave cellular targets.

Granzyme B and perforin proteins released by cytotoxic T cells induce apoptosis in target cells, forming transmembrane pores, and triggering apoptosis, perhaps through cleavage of caspases, although caspase-independent mechanisms of Granzyme B mediated apoptosis have been suggested.

Fragmentation of the nuclear genome by multiple nucleases activated by apoptotic signaling pathways to create a nucleosomal ladder is a cellular response characteristic of apoptosis. One nuclease involved in apoptosis is DNA fragmentation factor (DFF), a caspase-activated DNAse (CAD). DFF/CAD is activated through cleavage of its associated inhibitor ICAD by caspases proteases during apoptosis. DFF/CAD interacts with chromatin components such as topoisomerase II and histone H1 to condense chromatin structure and perhaps recruit CAD to chromatin. Another apoptosis activated protease is endonuclease G (EndoG). EndoG is encoded in the nuclear genome but is localized to mitochondria in normal cells. EndoG may play a role in the replication of the mitochondrial genome, as well as in apoptosis. Apoptotic signaling causes the release of EndoG from mitochondria. The EndoG and DFF/CAD pathways are independent since the EndoG pathway still occurs in cells lacking DFF.

Hypoxia, as well as hypoxia followed by reoxygenation, can trigger cytochrome c release and apoptosis. Glycogen synthase kinase (GSK-3) a serine-threonine kinase ubiquitously expressed in most cell types, appears to mediate or potentiate apoptosis due to many stimuli that activate the mitochondrial cell death pathway. [Loberg, RD, et al., J. Biol. Chem. (2002)277 (44): 41667-673]. It has been demonstrated to induce caspase 3 activation and to activate the proapoptotic tumor suppressor gene p53. It also has been suggested that GSK-3 promotes activation and translocation of the proapoptotic Bcl-2 family member, Bax, which, upon aggregation and mitochondrial localization, induces cytochrome c release. Akt is a critical regulator of GSK-3, and phosphorylation and inactivation of GSK-3 may mediate some of the antiapoptotic effects of Akt.

The term "autoimmune disease" as used herein refers to a condition in which the body's immune system mistakes its own healthy tissues as foreign and attacks them. Most autoimmune diseases cause inflammation that can affect many parts of the body.

The term "autologous" as used herein refer to being present in or derived from an individual's own tissues.

The term "B7 molecules" as used herein refers to cell surface proteins on specialized APCs, such as dendritic cells, which are the major costimulatory molecules for T cells. B7.1 (CD80) and B7.2 (CD86) are closely related members of the immunoglobulin superfamily and both bind to CD28 and CTLA-4 proteins on T cells.

The term "BAF chromatin remodeling complex" or "BRG1/BRMassociated factor complex" as used herein refers to a SW1/SNF-like chromatin remodeling complex needed for packing a DNA strand while the DNA remains accessible to the transcription machinery. The BAF complex is one of four ATP-dependent chromatin remodelling complex families known in mammals (the others being INO80/SWR1, ISWI and CHD) [Id., citing Hargreaves, DC and Crabtree, GR. Cell Res. (2011) 21: 396-420] for a review). The BAF complex is crucial for the regulation of gene expression and differentiation. The assembly of the BAF complex begins with the dimerization of a BAF155::155 homodimer or a BAF155::170 heterodimer. This is the platform for further BAF assembly, and the core module is formed by first incorporating BAF60 and later BAF47 and BAF57. The next steps are the integration of BAF250 and later BAF45C. Not until this core intermediate is formed, can the ATPase module (consistent of BRG1 or BRM, actin, SS18, BRD7 and BAF53A) bind and complete the BAF complex [Alfert, A. et al. Epigenetics & Chromatin (2019) 12: Article 19, citing Mashtalir, N. et al. Cell (2018) 175: 1272-88]. Both core subunits and variable ones contain DNA- and/or histone-binding domains such as zinc fingers, AT-hooks and chromo- and bromodomains. As a result, BAF complexes do not only recognize binding sites based on DNA sequence but more importantly also based on architectural characteristics and pre-existing regulatory histone modifications [Id., citing Wang, W. et al. EMBO J. (1996) 15: 5370-82; Wang, W. et al. Proc. Natl Acad. Sci. USA (1998) 95: 492-8;Wang, W. et al. Genes Dev. (1996) 4: 2117-30]. The BAF complex has an immense influence on both differentiation and gene expression. It is the chromatin remodeling complex most frequently involved in human malignancies. [Id., citing Kadoch, C. et l. Nat. Genet. (2013) 45: 592-601].

The term "batch" as used herein refers to a specific quantity of a material that is intended to have uniform character and quality, within specified limits.

"The term "beta2-microglobulin" as used herein refers to the light (β) chain of the MHC class I proteins, encoded outside the MHC. It binds noncovalently to the heavy or α chain and is required for MHC class I expression.

The term "binding" and its various grammatical forms means a lasting attraction between chemical substances.

The term "binding specificity" as used herein involves both binding to a specific partner and not binding to other molecules. Functionally important binding may occur at a range of affinities from low to high, and design elements may suppress undesired cross-interactions. Post-translational modifications also can alter the chemistry and structure of interactions. "Promiscuous binding" may involve degrees of structural plasticity, which may result in different subsets of residues being important for binding to different partners. "Relative binding specificity" is a characteristic whereby in a biochemical system a molecule interacts with its targets or partners differentially, thereby impacting them distinctively depending on the identity of individual targets or partners.

The term "biocompatible" as used herein refers to causing no clinically relevant tissue irritation, injury, toxic reaction, or immunological reaction to living tissue.

The term "biodegradable" as used herein refers to material that will break down actively or passively over time by simple chemical processes, by action of body enzymes or by other similar biological activity mechanisms.

As used herein, the term "biomarker" (or "biosignature") refers to a peptide, protein, nucleic acid, antibody, gene, metabolite, or any other substance used as an indicator of a biologic state. It is a characteristic that is measured objectively and evaluated as a cellular or molecular indicator of normal biologic processes, pathogenic processes, or pharmacologic responses to a therapeutic intervention. The term "indicator" as used herein refers to any substance, number or ratio derived from a series of observed facts that may reveal relative changes as a function of time; or a signal, sign, mark, note or symptom that is visible or evidence of the existence or presence thereof. Once a proposed biomarker has been validated, it may be used to diagnose disease risk, presence of disease in an individual, or to tailor treatments for the disease in an individual (choices of treatment or administration regimes). In evaluating potential therapies, a biomarker may be used as a surrogate for a natural endpoint, such as survival or irreversible morbidity. If a treatment alters the biomarker, and that alteration has a direct connection to improved health, the biomarker may serve as a surrogate endpoint for evaluating clinical benefit. Clinical endpoints are variables that can be used to measure how patients feel, function or survive. Surrogate endpoints are biomarkers that are intended to substitute for a clinical endpoint; these biomarkers are demonstrated to predict a clinical endpoint with a confidence level acceptable to regulators and the clinical community.

The terms "cell line" and "cultured cell line" are used interchangeably to refer to cells of a single type that have been adapted to grow continuously in the laboratory.

As used herein, the term "cell growth" is the process by which cells accumulate mass and increase in physical size. There are many different examples in nature of how cells can grow. In some cases, cell size is proportional to DNA content. For instance, continued DNA replication in the absence of cell division (called endoreplication) results in increased cell size. Megakaryoblasts, which mature into granular megakaryocytes, the platelet-producing cells of bone marrow, typically grow this way. By a different strategy, adipocytes can grow to approximately 85 to 120 µm by accumulating intracellular lipids. In contrast to endoreplication or lipid accumulation, some terminally differentiated cells, such as neurons and cardiac muscle cells, cease dividing and grow without increasing their DNA content. These cells proportionately increase their macromolecule content (largely protein) to a point necessary to perform their specialized functions. This involves coordination between extracellular cues from nutrients and growth factors and intracellular signaling networks responsible for controlling cellular energy availability and macromolecular synthesis. Perhaps the most tightly regulated cell growth occurs in dividing cells, where cell growth and cell division are clearly separable processes. Dividing cells generally must increase in size with each passage through the cell division cycle to ensure that a consistent average cell size is maintained. For a typical dividing mammalian cell, growth occurs in the G1 phase of the cell cycle and is tightly coordinated with S phase (DNA synthesis) and M phase (mitosis). The combined influence of growth factors, hormones, and nutrient availability provides the external cues for cells to grow. [Guertin, D.A., Sabatini, D.M., "Cell Growth," in The Molecular Basis of Cancer (4th Edn) Mendelsohn, J. et al Eds, Saunders (2015), 179-190].

As used herein, the term "cell proliferation" is meant to refer to the process that results in an increase of the number of cells, and is defined by the balance between cell divisions and cell loss through cell death or differentiation.

As used herein, the term "chemokine" is meant to refer to a class of chemotactic cytokines that orchestrate migration and positioning of immune cells within the tissues. Chemokines bind to seven transmembrane G protein-coupled receptors that trigger intracellular signaling that drives cell polarization, adhesion, and migration [Vilgelm, AE and Richmond, A. Front. Immunol. (2019) doi.org/10.3389/fimmu.2019.00333, citing Griffith, JW et al. Annu. Rev. Immunol. (2014) 32: 659-702; Nagarsheth, N. et al. Nat. Rev. Immunol. (2017) 17: 559-72]. They are divided into four families based upon structure: CXC, CC, CX3C, and C chemokines. The receptors follow a similar nomenclature system, based upon the family of chemokines to which they bind. In addition there is a family of atypical chemokine receptors that do not directly couple to G proteins, but are reported to have a variety of roles in development, homeostasis, inflammatory disease, infection, and cancer [Id., citing Nibbs, RJ, Graham, GJ. Nat. Rev. Immunol. (2013) 13: 815-29].

The term "CTLA-4" or "cytotoxic T-lymphocyte-associated antigen 4" or "CD152" as used herein refers to an inhibitory receptor and immune checkpoint that aids in maintaining self-antigen immunity by dampening T cell responses. It is a counterereceptor to costimulatory molecule CD28.

The term "cognate" signifies two biomolecules that typically interact, e.g., a receptor and its cognate ligand.

The term "compatible" as used herein refers to components of a composition that are capable of being combined with each other in a manner such that there is no interaction that would substantially reduce the efficacy of the composition under ordinary use conditions.

The terms "complete response" or "complete remission" or "CR" as used herein refer to the disappearance of all signs of cancer in response to treatment. This does not always mean the cancer has been cured.

The term "component" as used herein, is meant to refer to a constituent part, element or ingredient.

The term "composition" as used herein, is meant to refer to a material formed by a mixture of two or more substances.

As used herein, the term "condition" as used herein, is meant to refer to a variety of health states and is meant to include disorders or diseases caused by any underlying mechanism or disorder.

The term "consolidation therapy", also called "intensification therapy" and "post-remission therapy" as used herein refers to treatment that is given after cancer has disappeared following the initial therapy. Consolidation therapy is used to kill any cancer cells that may be left in the body.

As used herein, the term "contact" and its various grammatical forms is meant to refer to a state or condition of touching or of immediate or local proximity. Contacting a composition to a target destination may occur by any means of administration known to the skilled artisan.

The term "costimulation" as used herein refers to the second signal required for completion of lymphocyte activation and prevention of anergy, which is supplied by engagement of CD28 by CD80 and CD86 (T cells) and of CD40 by CD40 Ligand (B cells).

The term "costimulatory molecule" as used herein refers to molecules that are displayed on the cell surface that have a role in enhancing the activation of a T cell that is already being stimulated through its TCR. For example, HLA proteins, which present foreign antigen to the T cell receptor, require costimulatory proteins which bind to complementary receptors on the T cell's surface to result in enhanced activation of the T cell. Co-stimulatory molecules are highly active immunomodulatory proteins that play a critical role in the development and maintenance of an adaptive immune response (Kaufman and Wolchok eds., General Principles of Tumor Immunotherapy, Chpt 5, 67-121 (2007)). The two signal hypothesis of T cell response involves the interaction between an antigen bound to an HLA molecule and with its cognate T cell receptor (TCR), and an interaction of a co-stimulatory molecule and its ligand. Specialized APCs, which are carriers of a co-stimulatory second signal, are able to activate T cell responses following binding of the HLA molecule with TCR. By contrast, somatic tissues do not express the second signal and thereby induce T cell unresponsiveness (Id.). Many of the co-stimulatory molecules involved in the two-signal model can be blocked by co-inhibitory molecules that are expressed by normal tissue (Id.). In fact, many types of interacting immunomodulatory molecules expressed on a wide variety of tissues may exert both stimulatory and inhibitory functions depending on the immunologic context (Id.). The term "co-stimulatory receptor" as used herein refers to a cell surface receptor on naive lymphocytes through which they receive signals additional to those received through the antigen receptor, and which are necessary for the full activation of the lymphocyte. Examples are CD30 and CD40 on B cells, and CD27 and CD28 on T cells.

The term "cross-dressing" as used herein and depicted in **FIG. 4** refers to a pathway for cross-presentation. In cross-dressing, dendritic cells acquire preformed MHC class I molecules in complex with antigens from other cells by the process of trogocytotis [Yewdell, JW and Dolan, BP, "Cross-dressers turn on T cells". Nature (2011) 471 (7340): 581-82, citing Joly,. E. and Hudrisier, D. Nature Immunol. (2003) 4: 815; Herrera OB et al. J. Imunol. (2004) 173: 4828-37] or through gap junctions. This allows antigen presentation by acceptor dendritic cells to occur immediately, without any processing. Cross-dressing is used to activate memory T cells, but not naive T cells, in response to viral infection [Id., citing Wakins, LM and Bevan, MJ. Nature (2011) 471: 629-32].

The term "cross-presentation" as used herein and depicted in **FIG. 4** refers to a process by which proteins taken up by dendritic cells from the extracellular milieu can give rise to peptides presented by MNH class I molecules. It enables antigens from extracellular sources to be presented by MHC class I molecules and to activate CD8 T cells.

The term "cross-priming" as used herein refers to activation of CD8 T cells by dendritic cells in which the antigenic peptide presented by MHC class I molecules is derived from an exogenous protein (i.e., by cross-presentation), rather than produced within the dendritic cells directly (cf direct presentation).

The term "culture" and its other grammatical forms as used herein, is meant to refer to a process whereby a population of cells is grown and proliferated on a substrate in an artificial medium.

The term "cytokine" as used herein refers to small soluble protein substances secreted by cells which have a variety of effects on other cells. Cytokines mediate many important physiological functions including growth, development, wound healing, and the immune response. They act by binding to their cell-specific receptors located in the cell membrane, which allows a distinct signal transduction cascade to start in the cell, which eventually will lead to biochemical and phenotypic changes in target cells. Generally, cytokines act locally. They include type I cytokines, which encompass many of the interleukins, as well as several hematopoietic growth factors; type II cytokines, including the interferons and interleukin-10; tumor necrosis factor ("TNF")-related molecules, including TNFα and lymphotoxin; immunoglobulin super-family members, including interleukin 1 ("IL-1"); and the chemokines, a family of molecules that play a critical role in a wide variety of immune and inflammatory functions. The same cytokine can have different effects on a cell depending on the state of the cell. Cytokines often regulate the expression of, and trigger cascades of, other cytokines. Non-limiting examples of cytokines include e.g., IL-1, IL-2, IL-3, IL-4, IL-5, IL-6, IL-7, IL-8, IL-9, IL-10, IL-11, IL-12/IL-23 P40, IL13, IL-15, IL-15/IL15-RA, IL-17, IL-18, IL-21, IL-23, TGF-β, IFNγ, GM-CSF, Groα, MCP-1 and TNF-α.

The term "cytotoxic T lymphocytes" (CTLs) as used herein refers to effector CD8+ T cells. Cytotoxic T cells kill by inducing their targets to undergo apoptosis/programmed cell death via extrinsic and intrinsic pathways.

The term "dendritic cells (DC)" as used herein refers to professional antigen presenting cells, which induce naive T cell activation and effector differentiation. [Patente, TA, et al., Frontiers Immunol. (2019) doi.org/10.3389/fimmu.2018.03176]. Human DCs are identified by their high expression of major histocompatibility complex (MHC) class II molecules (MHC-II) and of CD11c, both of which are found on other cells, like lymphocytes, monocytes and macrophages [Id., citing Carlens J, et al. J Immunol. (2009) 183:5600-7; Drutman SB, et al. J Immunol. (2012) 188:3603-10; Hochweller K, Set al. Eur J Immunol. (2008) 38:2776-83; Huleatt JW, Lefrançois L. J Immunol. (1995) 154:5684-93; Rubtsov AV, et al. Blood (2011) 118:1305-15; Probst HC, et al. Clin Exp Immunol. (2005) 141:398-404; Vermaelen K, Pauwels R. Cytometry (2004) 61A:170-7]. DCs express many other molecules which allow their classification into various subtypes. Although some of the DC subtypes were originally described as macrophages, DC and macrophages have distinct characteristics [Id., citing Delamarre L, Science (2005) 307:1630-4; Geissmann F, et al. Science (2010) 327:656-61; van Montfoort N, et al. Proc Natl Acad Sci USA. (2009) 106:6730-5] and ontogeny, so that, currently, little doubt remains that they belong to distinct lineages [Id., citing Haniffa M, et al. (2013) 120:1-49; Hashimoto D, et al. Immunity (2013) 38:792-804; Hettinger J, et al. Nat Immunol. (2013) 14:821-30; McGovern N, et al. Immunity (2014) 41:465-77; Naik SH, et al. Nature (2013) 496:229-32; Schulz C, et al. Science (2012) 336:86-90; Schraml BU, et al. Cell (2013) 154:843-58; Wang J, et al. Mol Med Rep. (2017) 16:6787-93; Yona S, et al. Immunity (2013) 38:79-91].

The term "derived from" as used herein encompasses any method for receiving, obtaining, or modifying something from a source of origin.

The term "detectable marker" encompasses both selectable markers and assay markers. The term "selectable markers" refers to a variety of gene products to which cells transformed with an expression construct can be selected or screened, including drug-resistance markers, antigenic markers useful in fluorescence-activated cell sorting, adherence markers such as receptors for adherence ligands allowing selective adherence, and the like. "Assay markers" are measurable components whose presence or absence can be detected and correlated to a particular detectable response.

The term "detectable response" as used herein, is meant to refer to any signal or response that may be detected in an assay, which may be performed with or without a detection reagent. Detectable responses include, but are not limited to, radioactive decay and energy (e.g., fluorescent, ultraviolet, infrared, visible) emission, absorption, polarization, fluorescence, phosphorescence, transmission, reflection or resonance transfer. Detectable responses also include chromatographic mobility, turbidity, electrophoretic mobility, mass spectrum, ultraviolet spectrum, infrared spectrum, nuclear magnetic resonance spectrum and x-ray diffraction. Alternatively, a detectable response may be the result of an assay to measure one or more properties of a biologic material, such as melting point, density, conductivity, surface acoustic waves, catalytic activity or elemental composition. A "detection reagent" is any molecule that generates a detectable response indicative of the presence or absence of a substance of interest. Detection reagents include any of a variety of molecules, such as antibodies, nucleic acid sequences and enzymes. To facilitate detection, a detection reagent may comprise a marker.

The term "derived from" as used herein, is meant to encompasses any method for receiving, obtaining, or modifying something from a source of origin.

The term "direct presentation" as used herein and depicted in **FIG. 4** refers to the process by which proteins produced within a given cell give rise to peptides presented by MHC class I molecules. This may refer to APCs (such as dendritic cells), or to nonimmune cells that will become the targets of CTLs.

The term "DAMPs" is an abbreviation for damage-associated molecular patterns. DAMPs are molecules released by stressed or dying cells that bind to pattern recognition molecules (PRMs) and induce inflammation.

The term "differentiate" and its various grammatical forms as used herein refer to the process of development with an increase in the level of organization or complexity of a cell or tissue, accompanied with a more specialized function.

The terms "disease progression" or "progressive disease" as used herein refers to a cancer that continues to grow or spread.

The term "dose" as used herein, is meant to refer to the quantity of a therapeutic substance prescribed to be taken at one time. The term "maximum tolerated dose" as used herein is meant to refer to the highest dose of a drug or treatment that does not cause unacceptable side effects.

The term "dose escalation study" as used herein refers to a type of study where enrolled patients receive different doses of an investigational agent to determine the recommended phase 2 dose.

The term "dose limiting toxicities" as used herein refers to side effects of a treatment that are serious enough to prevent an increase in dose of that treatment.

The term "ECOG performance status scale" as used herein refers to a scale used to assess how a patient's disease is progressing, assess how the disease affects the daily living abilities of the patient, and determine appropriate treatment and prognosis. The scale, which is shown below in Table 3, was developed by the Eastern Cooperative Oncology Group (ECOG), now part of the ECOG-ACRIN Cancer Research Group, Oken M, Creech R, Tormey D, et al. Toxicity and response criteria of the Eastern Cooperative Oncology Group. Am J Clin Oncol. 1982;5:649-655.

**Table 3. ECOG performance status scale.**

| **Grade** | **ECOG Performance Status** |
|---|---|
| 0 | Fully active, able to carry on all pre-disease performance without restriction |
| 1 | Restricted in physically strenuous activity but ambulatory and able to carry out work of a light or sedentary nature, e.g., light house work, office work |
| 2 | Ambulatory and capable of all selfcare but unable to carry out any work activities; up and about more than 50% of waking hours |
| 3 | Capable of only limited selfcare; confined to bed or chair more than 50% of waking hours |
| 4 | Completely disabled; cannot carry on any selfcare; totally confined to bed or chair |
| 5 | Dead |

The term "effective dose" as used herein generally refers to that amount of therapeutic agent sufficient to induce a therapeutic effect. An effective dose may refer to the amount of the therapeutic agent sufficient to delay or minimize the onset of symptoms. An effective dose may also refer to the amount of the therapeutic agent that provides a therapeutic benefit in the treatment or management of a disease, disorder or condition. Further, an effective dose is the amount with respect to a therapeutic agent alone, or in combination with other therapies, that provides a therapeutic benefit in the treatment or management of a disease. An effective dose may also be the amount sufficient to enhance the subject's (e.g., a human's) own immune response. Levels of immunity can be monitored, e.g., by measuring amounts of neutralizing secretory and/or serum antibodies, e.g., by plaque neutralization, complement fixation, enzyme-linked immunosorbent, or microneutralization assay. In the case of a vaccine, an "effective dose" is one that prevents disease and/or reduces the severity of symptoms.

For any therapeutic agent described herein the effective dose may be initially determined from preliminary in vitro studies and/or animal models. A therapeutically effective dose may also be determined from human data. The applied dose may be adjusted based on the relative bioavailability and potency of the administered agent. Adjusting the dose to achieve maximal efficacy based on the methods described above and other well-known methods is within the capabilities of the ordinarily skilled artisan.

General principles for determining therapeutic effectiveness, which may be found in Chapter 1 of Goodman and Gilman's The Pharmacological Basis of Therapeutics, 10th Edition, McGraw-Hill (New York) (2001), incorporated herein by reference, are summarized below.

Pharmacokinetic principles provide a basis for modifying a dosage regimen to obtain a desired degree of therapeutic efficacy with a minimum of unacceptable adverse effects. In situations where an agent's plasma concentration can be measured and related to the therapeutic window, additional guidance for dosage modification can be obtained.

The term "effector cell" as used herein refers to a cell that carries out a final response or function. The main effector cells of the immune system, for example, are activated lymphocytes and phagocytes.

The term "effector functions" as used herein refers to the actions taken by effector cells and antibodies to eliminate foreign entities, and includes, without limitation, cytokine secretion, cytotoxicity, and antibody-mediated clearance.

The term "eligible subject" as used herein refers to a subject that satisfies the requirements to be treated with the immunotherapy of the present disclosure under the professional judgment of the patient's physician. Eligibility criteria may include the subject's age, type and stage of cancer, current health status, medical history, and previous treatments.

The term "enrich" as used herein refers to increasing the proportion of a desired substance, for example, to increase the relative frequency of a subtype of cell compared to its natural frequency in a cell population. Positive selection, negative selection, or both are generally considered necessary to any enrichment scheme. Exemplary selection methods include, without limitation, magnetic separation and FACS.

The term "epigenetic marks" as used herein refer to DNA methylation, histone modifications, chromatin remodeling and microRNA.

The term "epigenetics" as used herein refers to a stably heritable phenotype resulting from changes in a chromosome without alterations in the DNA sequence.

The term "exclusion criteria" as used herein refers to specify characteristics that disqualify subjects from clinical studies and often include factors such as comorbidities or concomitant treatment or factors that could mask the effect of the study treatment.

The term "expand" or "amplify" as used herein with respect to cells refers to increasing in cell number.

As used herein, the term "expression" and its other grammatical forms refers to production of an observable phenotype by a gene, usually by directing the synthesis of a protein. It includes the biosynthesis of mRNA, polypeptide biosynthesis, polypeptide activation, e.g., by post-translational modification, or an activation of expression by changing the subcellular location or by recruitment to chromatin.

The term "extracellular matrix" or "ECM" as used herein refers to a scaffold in a cell's external environment with which the cell interacts via specific cell surface receptors. The extracellular matrix serves many functions, including, but not limited to, providing support and anchorage for cells, segregating one tissue from another tissue, and regulating intracellular communication. Generally, the extracellular matrix is composed of an interlocking mesh of fibrous proteins and glycosaminoglycans (GAGs). Examples of fibrous proteins found in the extracellular matrix include collagen, elastin, fibronectin, and laminin. Examples of GAGs found in the extracellular matrix include proteoglycans (e.g., heparin sulfate), chondroitin sulfate, keratin sulfate, and non-proteoglycan polysaccharide (e.g., hyaluronic acid). The term "proteoglycan" refers to a group of glycoproteins that contain a core protein to which is attached one or more glycosaminoglycans.

As used herein the term "Fas" refers to a type 2 membrane protein found on lymphocytes that belongs to the TNF superfamily. In cells that express Fas, engagement of the cell death receptor Fas by Fas ligand (FasL) results in apoptotic cell death, mediated by caspase activation.

The term "flow cytometry" as used herein, is meant to refer to a tool for interrogating the phenotype and characteristics of cells. It senses cells or particles as they move in a liquid stream through a laser (light amplification by stimulated emission of radiation)/light beam past a sensing area. The relative light-scattering and color-discriminated fluorescence of the microscopic particles is measured. Flow analysis and differentiation of the cells is based on size, granularity, and whether the cell is carrying fluorescent molecules in the form of either antibodies or dyes. As the cell passes through the laser beam, light is scattered in all directions, and the light scattered in the forward direction at low angles (0.5°-10°, inclusive) from the axis is proportional to the square of the radius of a sphere and so to the size of the cell or particle. Light may enter the cell; thus, the 90 ° light (right-angled, side) scatter may be labeled with fluorochrome-linked antibodies or stained with fluorescent membrane, cytoplasmic, or nuclear dyes. Thus, the differentiation of cell types, the presence of membrane receptors and antigens, membrane potential, pH, enzyme activity, and DNA content may be facilitated. Flow cytometers are multiparameter, recording several measurements on each cell; therefore, it is possible to identify a homogeneous subpopulation within a heterogeneous population (Marion G. Macey, Flow cytometry: principles and applications, Humana Press, 2007). Fluorescence-activated cell sorting (FACS), which allows isolation of distinct cell populations too similar in physical characteristics to be separated by size or density, uses fluorescent tags to detect surface proteins that are differentially expressed, allowing fine distinctions to be made among physically homogeneous populations of cells.

The term "forkhead box, subgroup O (FOXO) transcription factors" as used herein refers to a subfamily of a conserved FOX protein family which regulates gene expression, consisting of FOXO1, FOXO3, FOXO4, and FOXO6 in mammals. They bind to DNA through the FoxO-recognized element in the C-terminal basic region of the forkhead DNA-binding domain. Following forkhead binding to DNA, target gene expression is repressed or activated through the FoxO-recognized element in the C-terminal basic region of the forkhead DNA-binding domain (DBD). Following forkhead binding to DNA, target gene expression is repressed or activated through multiple protein-DNA contacts with the primary recognition site located at α-helix H3. [Dai, S. et al. Nucleic Acids Res. (2021) 49 (18): 10235-49] FOXOs control cellular homeostasis, such as stress resistance, cell cycle, cell differentiation, apoptosis, proteostasis, intracellular signaling, metabolism, and autophagy. FOXOs are well known for their functions in oxidative and redox regulation downstream of the insulin/insulin-like growth factor-1 (IGF-1) signaling (IIS) pathway. The pI3K pathway is an important regulator of FOXO activity. FOXOs also play critical functions in DNA damage repair.

The term "GATA binding protein 3" (or "GATA-3") is a transcription factor protein that contains two zinc fingers. It is a member of the GATA family of conserved zinc-finger transcription factors. GATA-3 is a master regulator of type 2 T helper cell development.

The term "graft versus host disease " or "GVHD" as used herein refers to an attack on the tissues of a recipient by mature T cells from a nonidentical donor, which can cause a variety of symptoms, sometimes severe.

The term "graft versus tumor effect" as used herein refers to immune reactivity mediated by donor T cells against the recipient's tumor cells.

The term "haploidentical" as used herein refers to half-matched donors. A haploidentical donor is usually an individual's mother, father, or child. Parents are always a half-match for their children. Siblings (brothers or sisters) have a 50% (1 out of 2) chance of being a half-match for each other.

The term "healthy subject" or "healthy donor" or "healthy adult donor" as used herein refers to a subject having no signs or symptoms of a cancer.

As used herein, the term "immune checkpoints" refers to the array of inhibitory pathways necessary for maintaining self-tolerance and that modulate the duration and extent of immune responses to minimize damage to normal tissue. In T cells, the ultimate amplitude and quality of the immune response, which is initiated through antigen recognition by the TCR, is regulated by a balance between co-stimulatory and inhibitory signals (immune checkpoints). [Pardoll, DM. Nat. Rev. Cancer (2012) 12(4): 252-64]. Immune checkpoint molecules such as PD-1, PD-L1, CTLA-4 are cell surface signaling receptors that play a role in modulating the T-cell response in the tumor microenvironment. Tumor cells have been shown to utilize these checkpoints to their benefit by up-regulating their expression and activity. With the tumor cell's ability to commandeer some immune checkpoint pathways as a mechanism of immune resistance, it has been hypothesized that checkpoint inhibitors that bind to molecules of immune cells to activate or inactivate them may relieve their inhibition of an immune response. Immune checkpoint inhibitors have been reported to block discrete checkpoints in an active host immune response allowing an endogenous anti-cancer immune response to be sustained. Recent discoveries have identified immune checkpoints or targets, like PD-1, PD-L1, PD-L2, CTLA-4, TIGIT, TIM-3, LAG-3, CCR4, OX40, OX40L, IDO, and A2AR, as proteins responsible for immune evasion.

The terms "immune escape" or "immune evasion" as used herein refers to a strategy to evade a host's immune response. It is characterized by the inability of the immune system to eliminate transformed cells prior to and after tumor development. The host's contribution is manifested by its inability to recognize antigens expressed by tumor cells, a phenomenon known as "host ignorance." It happens because of defects in both the innate and adaptive arms of the immune system. The tumor's contribution is manifested by the adaptation of tumor cells to evade the immune system or by developing a microenvironment that suppresses the immune system. [Qian J. et al. (2011) Immune Escape. In: Schwab M. (eds) Encyclopedia of Cancer. Springer, Berlin, Heidelberg. https://doi.org/10.1007/978-3-642-16483-5_2975].

The term "immune homeostasis" refers to the delicate and finely regulated balance of appropriate immune activation and suppression in tissues and organs, driven by a myriad of cellular players and chemical factors. [da Gama Duarte, J. et al. Immunology and Cell Biology (2018) 96: 497-506]

The terms "immune response" and "immune-mediated" are used interchangeably herein to refer to any functional expression of a subject's immune system, against either foreign or self-antigens, whether the consequences of these reactions are beneficial or harmful to the subject. The term "immunological response" to an antigen or composition as used herein is meant to refer to the development in a subject of a humoral and/or a cellular immune response to an antigen. For purposes of the present disclosure, a "humoral immune response" refers to an immune response mediated by antibody molecules, while a "cellular immune response" is one mediated by T-lymphocytes and/or other white blood cells. One aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells. Hence, an immunological response may include one or more of the following effects: the production of antibodies by B-cells; and/or the activation of T cells, suppressor T-cells and/or γδ T-cells directed specifically to an antigen or antigens present in the composition of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

The term "immune phenotype" or "immunotype" as used herein refers to the collective frequency of various immune cell populations and their functional responses to stimuli (cell signaling and antibody responses). [See Kaczorowski, KJ et al. Proc. Nat. Acad. Sci. USA (2017) doi/10.1073/pnas. 1705065114]

The terms "immune surveillance" or "immunological surveillance" are used interchangeably to refer to a monitoring process by the immune system to detect and destroy infected and neoplastically transformed cells in the body.

The term "immune system" as used herein refers to the body's system of defenses against disease, which comprises the innate immune system and the adaptive immune system. The innate immune system provides a non-specific first line of defense against pathogens. It comprises physical barriers (e.g. the skin) and both cellular (granulocytes, natural killer cells) and humoral (complement system) defense mechanisms. The reaction of the innate immune system is immediate, but unlike the adaptive immune system, it does not provide permanent immunity against pathogens. The adaptive immune response is the response of the vertebrate immune system to a specific antigen that typically generates immunological memory.

The term "immunological synapse" ("IS") as used herein refers to a highly structured body that functions to concentrate T cell signaling in a defined area. It is associated with the selective recruitment of signaling molecules and exclusion of negative regulators. The synapse is stabilized by a ring of adhesion molecules, including, for example, LFA1, which binds to ICAM1 on the APC. The immune synapse modulates TCR signaling by several mechanisms. In the earliest stages of immune synapse formation, TCR-containing microclusters are recruited to the central molecular cluster. The TCR responds to two distinct pMHC ligands (agonist and self peptide-MHC) in coagonism rather than nonspecific TCR-MHC interactions, which adds to the overall binding strength of the TCR-p-MHC complex. The strength of the TCR-p-MHC interactions has a role in determining the influence of coreceptors that are recruited to the immune synapse (e.g., CD8). The immune synapse also modulates TCR signaling by regulating interactions between kinases in the TCR pathway and their substrates. [Morris, G. and Allen, PM. Nature Immunol. (2012) doi:10.1038/nm.2190].

Studies have shown that expression of CD5 increases according to the magnitude of the signal delivered by the TCR. Consequently, CD5 is an activation marker of T cells, and CD5 expression reflects the heterogeneity of the signal strength associated with each individual TCR within a polyclonal T cell population. [Voisinne, G. et al. Front. Immunol. (2018) 9:2900]. In homeostasis, high expression of CD5 is considered a surrogate for TCR affinity for self-p-MHC and is positively correlated with expression of the IL-7 receptor. {Morris, GP and Allen, PM. Nature Immunol. (2012) doi: 10.1038/ni.2190}. Another model, termed "coreceptor tuning" proposes a mechanism for the adjustment of homeostasis in the periphery whereby expression of the Il-7 receptor and CD8 are reciprocally regulated. [Id.]

The T cell signaling pathway includes proximate signaling (including phosphorylation of the invariant signaling protein CD3 and early signaling molecules, calcium-mediated signaling (release of intracellular Ca2+ stores and influx of extracellular Ca2+), and GTPase Ras-MAPK signaling. [Id.]

T cell activation is mediated through highly organized and dynamic interaction of TCRs with MHC-peptide complexes at the IS. A mature IS is an aggregation of TCR-based signalosomes (meaning multimolecular complexes) that induce T cell responses and is defined by three concentric rings of clustered molecules. The inner circle is termed the central supramolecular activation cluster (cSMAC) where TCR signaling takes place. The cSMAC contains most of the TCR-MHC peptide complexes, CD28, PKC-theta and Lck, whereas peripheral SMAC (pSMAC) contains proteins involved in cell adhesion, such as integrin LFA-1, cytoskeletal linker talin, and ICAM1. Larger molecules, such as CD43 and CD45, are excluded from the pSMAC and make up the distal SMAC (dSMAC). Inhibitory and costimulatory molecules, such as PD-1, CTLA-4, and ICOS also are aggregated at the region of the IS and play crucial roles in the regulation of T cell activation. [Watanabe, K. et al. Front. Immunol. (2018) 9: 2486, citing Yokosuka, T. et al. Immunity (2010) 33: 326-39]. Secretion of lytic granules occurs within a secretary synapse between CTLs and target cells. The secretory synapse has two separate and distinct domains in cSMAC: one is a signaling domain, which contains the signaling proteins, and another is a secretory domain for exocytosis of cytokines, perforrins and granzymes. The transient polarization and docking of the centrosome to the plasma membrane, which is controlled by Lck signaling, has an important role in the mechanism of directing this secretion. [Id., citing Stinchcombe, JC et al. Nature (2006) 443: 462-5; Stinchcombe, JC et al. Nature (2006) 443: 462-5; Tsun, A. et al. J. Cell Biol. (2011) 192: 663-74].

The intracellular signaling downstream of CARs and the mechanisms of the IS formed by CARS have not been extensively studied. It has been demonstrated that CAR clustering, ZAP70 recruitment to IS, and exclusion of CD45 outside of IS occurs between CD19-specific CAR-T cells and target cells that is similar to TCR activation. Downstream signaling molecules of the TCR, such as CD3zeta, LAT, Lck and ZAP70 are phosphorylated after CD19-CAR-T cell activation by autologous CD19 B cells [Id., citing Karlsson, H. et al. PLoS ONE (2015) 10:e0144787]. The CAR IS does not present a systematic bull's eye structure, which is a characteristic feature of TCR IS. Organization of the actin ring is poor, and actin may not be completely diminished at the center of CAR IS [Id., citing Xiong, W. et al. Mol. Ther. (2018) 26: 963-75]. LFA-1 is disorganized and CAR-tumor antigen complexes form microclusters that are randomly distributed at the CAR immunological synapse [Id., citing Davenport, AJ et al. Proc. Natl Acad. Sci. USA (2018) 115: E2068-76]. While the TCR IS requires 5-10 min to form the bull's eye structure, the CAR IS might not need to form these stable structures because the disorganized multifocal pattern of the CAR IS is sufficient to rapidly induce proximal signaling, which occurs within a short period of time (<2 min). The rapid but short duration of proximal signaling of CAR IS also induces rapid microtubule organizing center (MTOC) to the IS and accelerates the delivery of cytotoxic granules including perforin and granzymes, to the IS [Id., citing Davenport, AJ et al. Proc. Natl Acad. Sci. USA (2018) 115: E2068-76].

The term "immunotherapeutic agent" as used herein refers to a drug, molecule, nucleic acid, protein, composition or cell that provides a therapeutic effect. The term "active" as used herein refers to the ingredient, component or constituent of the compositions of the present invention responsible for the intended therapeutic effect. The terms "immunotherapeutic agent" and "active agent" are used interchangeably herein.

The term "immunotherapeutic component" as used herein refers to a therapeutically effective dosage (i.e., dose and frequency of administration) that eliminates, reduces, or prevents the progression of a particular disease manifestation in a percentage of a population. An example of a commonly used therapeutic component is the ED50, which describes the dose in a particular dosage that is therapeutically effective for a particular disease manifestation in 50% of a population.

The term "immunotherapy" as used herein refers to measures taken using immunological methods and principles to target the hyper or hyo-immune state of an organism, intervene or adjust the organism's immune function artificially, and strengthen or attenuate the immune response so as to treat disease. It enhances the immune system's ability to recognize, target and eliminate cancer cells in the body. [Zhang, Z. et al. Front. Immunol. (2021) 12: Barbari, C. et al. Intl J. Mol. Sci. (2020) 21: 5009]. Some types of immunotherapy only target certain cells of the immune system. Others affect the immune system in a general way.

The term "induction therapy", also called first line therapy, primary therapy and primary treatment, refers to the first treatment given for a disease.

The term "inflammation" as used herein refers to the physiologic process by which vascularized tissues respond to injury. See, e.g., FUNDAMENTAL IMMUNOLOGY, 4th Ed., William E. Paul, ed. Lippincott-Raven Publishers, Philadelphia (1999) at 1051-1053, incorporated herein by reference. During the inflammatory process, cells involved in detoxification and repair are mobilized to the compromised site by inflammatory mediators. Inflammation is often characterized by a strong infiltration of leukocytes at the site of inflammation, particularly neutrophils (polymorphonuclear cells). These cells promote tissue damage by releasing toxic substances at the vascular wall or in uninjured tissue. Traditionally, inflammation has been divided into acute and chronic responses.

The term "acute inflammation" as used herein refers to the rapid, short-lived (minutes to days), relatively uniform response to acute injury characterized by accumulations of fluid, plasma proteins, and neutrophilic leukocytes. Examples of injurious agents that cause acute inflammation include, but are not limited to, pathogens (e.g., bacteria, viruses, parasites), foreign bodies from exogenous (e.g. asbestos) or endogenous (e.g., urate crystals, immune complexes), sources, and physical (e.g., burns) or chemical (e.g., caustics) agents.

The term "chronic inflammation" as used herein refers to inflammation that is of longer duration and which has a vague and indefinite termination. Chronic inflammation takes over when acute inflammation persists, either through incomplete clearance of the initial inflammatory agent or as a result of multiple acute events occurring in the same location. Chronic inflammation, which includes the influx of lymphocytes and macrophages and fibroblast growth, may result in tissue scarring at sites of prolonged or repeated inflammatory activity.

The term "intercellular adhesion molecules" or "ICAMs" as used herein refers to cell-adhesion molecules of the immunoglobulin superfamily that bind to the leukocyte integrin CD11a:CD18 (LFA-1). They are crucial in the binding of lymphocytes and other leukocytes to antigen-presenting cells and endothelial cells.

The term "inhibitor receptor lymphocyte activation gene-3" or "LAG-3" as used herein refers to a member of the immunoglobulin superfamily (IgSF) and binds to major histocompatibility complex (MHC) class II. LAG-3 expression on tumor infiltrating lymphocytes (TILs) is associated with tumor-mediated immune suppression.

The term "human inositol auxotrphy 80 (INO80) chromatin remodeling complex" as used herein refers to a subfamily of chromatin remodeling complexes that plays an important role in nucleosome mobilization during transcription and DNA repair. It binds to the promoter regions of certain actively transcribed genes and mobilizes nucleosomes using the energy provided by ATP. There is strong evidence in support of a direct role for INO80 during DNA replication. [Falbo, KB and Shen, X. Front Biosci (Landmark Ed. (2012) 17: 970-75).

The term "leukemia" as used herein refers to a cancer of blood forming tissues, including bone marrow. Many types exist including, without limitation,acute lymphoblastic leukemia (ALL), acute myeloid leukemia (AML), and chronic lymphocytic leukemia (CLL).

The term "linker for activation of T cells" or "LAT" as used herein refers to a signaling adaptor protein at the immune synapse. Once phosphorylated by ZAP70, LAT recruits cytosolic adaptors including SH2 domain-containing leukocyte phosphoprotein of 76 kDa (SLP76), growth factor receptor-bound protein 2 (GRB2), GRB2-related adaptor protein (GRAP2), as well as enzymes like PhosphoLipase C (PLCγ1) and IL-2 inducible T cell kinase (ITK).

The term "Lck" or "p56Ick" as used herein refers to a Src family kinase recruited to phosphorylate CD3 ITAMs during immune synapse maturation. [Ehrlich, L.I.R. et al. Immunity (2002) 17 (6): 809-22].

The term "lymphocyte" refers to a small white blood cell formed in lymphatic tissue throughout the body and in normal adults making up about 22-28% of the total number of leukocytes in the circulating blood that plays a large role in defending the body against disease. Individual lymphocytes are specialized in that they are committed to respond to a limited set of structurally related antigens. This commitment, which exists before the first contact of the immune system with a given antigen, is expressed by the presence on the lymphocyte's surface membrane of receptors specific for determinants (epitopes) on the antigen. Each lymphocyte possesses a population of receptors, all of which have identical combining sites. One set, or clone, of lymphocytes differs from another clone in the structure of the combining region of its receptors and thus differs in the epitopes that it can recognize. Lymphocytes differ from each other not only in the specificity of their receptors, but also in their functions. Lymphocytes are much more common in the lymphatic system, and include B cells, T cells, natural killer T (NKT) cells, and natural killer (NK) cells. There are two broad categories of lymphocytes, namely T cells and B cells. T-cells are responsible for cell-mediated immunity whereas B-cells are responsible for humoral immunity (relating to antibodies). T-cells are so-named such because these lymphocytes mature in the thymus; B-cells mature in bone marrow. B cells make antibodies that bind to pathogens to enable their destruction. CD4+ (helper) T cells coordinate the immune response. CD8+ (cytotoxic) T cells, NKT, and Natural Killer (NK) cells are able to kill cells of the body that are, e.g., infected by a virus or display an antigenic sequence.

The term "lymphocyte activation" or "activation" refers to stimulation of lymphocytes by specific antigens, nonspecific mitogens, or allogeneic cells resulting in synthesis of RNA, protein and DNA and production of lymphokines, the soluble product of lymphocytes; it is followed by proliferation and differentiation of various effector and memory cells. For example, a mature B cell can be activated by an encounter with an antigen that expresses epitopes that are recognized by its cell surface immunoglobulin (Ig). The activation process may be a direct one, dependent on cross-linkage of membrane Ig molecules by the antigen (cross-linkage-dependent B cell activation) or an indirect one, occurring most efficiently in the context of an intimate interaction with a helper T cell ("cognate help process"). T-cell activation is dependent on the interaction of the TCR/CD3 complex with its cognate ligand, a peptide bound in the groove of a class I or class II MHC molecule. The molecular events set in motion by receptor engagement are complex. Full responsiveness of a T cell requires, in addition to receptor engagement, an accessory cell-delivered costimulatory activity, e.g., engagement of CD28 on the T cell by CD80 and/or CD86 on the antigen presenting cell (APC).

The term "lymphodepletion" as used herein refers to a short course of chemotherapy to kill T cells. It creates a favorable immune environment for CAR T cells, which improves their expansion, persistence and clinical activity while reducing the potential for anti-CAR immune responses. [Wagner, DL et al. Nat. Rev. Clin. Oncol. (2021) 18 (6): 379-93].

The term "lymphoma" as used herein refers to a cancer of the lymphatic system. The lymphatic system includes the lymph nodes, spleen, thymus gland, and bone marrow. Examples include Hodgkin's lymphoma, non-Hodgkin's lymphoma, cutaneous B cell lymphoma; cutaneous T cell lymphoma and Waldenstrom macroglobulinemia.

The term "macrophage" as used herein refers to a mononuclear, actively phagocytic cell arising from monocytic stem cells in the bone marrow. These cells are widely distributed in the body and vary in morphology and motility. Phagocytic activity is typically mediated by serum recognition factors, including certain immunoglobulins and components of the complement system, but also may be nonspecific. Macrophages also are involved in both the production of antibodies and in cell-mediated immune responses, particularly in presenting antigens to lymphocytes. They secrete a variety of immunoregulatory molecules.

The term "maintenance therapy" or "continuous therapy" as used herein refers to the ongoing treatment of cancer after it has responded to induction therapy to prevent relapse.

The terms "Major Histocompatibility Complex (MHC), MHC-like molecule" and "HLA" are used interchangeably herein to refer to cell-surface molecules that display a molecular fraction known as an epitope or an antigen and mediate interactions of leukocytes with other leukocyte or body cells. MHCs are encoded by a large gene group and can be organized into three subgroups- class I, class II, and class III. In humans, the MHC gene complex is called HLA ("Human leukocyte antigen"); in mice, it is called H-2 (for "histocompatibility"). Both species have three main MHC class I genes, which are called HLA-A, HLA-B, and HLA-C in humans, and H2-K, H2-D and H2-L in the mouse. These encode the α chain of the respective MHC class I proteins. The other subunit of an MHC class I molecule is 02-microglobulin. The class II region includes the genes for the α and β chains (designated A and B) of the MHC class II molecules HLA-DR, HLA-DP, and HLA-DQ in humans. Also in the MHC class II region are the genes for the TAP1:TAP2 peptide transporter, the PSMB (or LMP) genes that encode proteasome subunits, the genes encoding the DMα and BMβ chains (DMA and DMB), the genes encoding the α and β chains of the DO molecule (DOA and DOB, respectively), and the gene encoding tapasin (TAPBP). The class II genes encode various other proteins with functions in immunity. The DMA and DMB genes encoding the subunits of the HLA-DM molecule that catalyzes peptide binding to MHC class II molecules are related to the MHC class II genes, as are the DOA and DOB genes that encode the subunits of the regulatory HLA-DO molecule. [Janeway's Immunobiology. 9th ed., GS, Garland Science, Taylor & Francis Group, 2017. pps. 232-233]. In humans, there are three MHC class II isotypes: HLA-DR, HLA-DP, and HLA-DQ, encoded by α and β chain genes within the Human Leukocyte Antigen (HLA) locus on chromosome 6 [Wosen, JE et al. Front. Immunol. (2018) doi.10.3389/fimmu.2018.02144].

The term "MHC restriction" as used herein refers to the requirement that APCs or target cells express MHC molecules that a T cell recognizes as self in order for T cell to respond to the antigen presented by that APC or target cell (T cells will only recognize antigens presented by their own MHC molecules). For example, CD8 T cells bind class I MHC which are expressed on most cells in the body, and CD4 T cells bind class II MHC which are only expressed on specialized APCs.

The abbreviation "MAPK" as used herein refers to Mitogen-Activated Protein Kinase (MAPK) signaling, which activates a three-tiered cascade with MAPK kinase kinases (MAP3K) activating MAPAK kinases (MAP2K) and finally MAPK. MAPKs are protein Ser/Thr kinases that convert extracellular stimuli into a wide range of cellular responses. [Cargnello, M. and Roux, PP, Microbiol. Mol. Biol. Rev. (2011) 75(1): 50-83]. The major MAPK pathways involved in inflammatory diseases are extracellular regulating kinase (ERK), p38 MAPK, and c-Jun NH2-terminal kinase (JNK). Upstream kinases include TGFβ-activated kinase-1 (TAK1) and apoptosis signal-regulating kinase-1 (ASK1). Downstream of p38 MAPK is MAPK activated protein kinase 2 (MAPKAPK2 or MK2).

In canonical signal transduction, p38 MAPK is selectively phosphorylated by MAPKKs (MKK3 and MKK6), which in turn are activated by MAPKKKs, including TGFβ-activated kinase 1 (TAK1), apoptosis signal-regulating kinase 1 (ASK1), mixed-lineage kinase 2 (MLK2) or MLK3. [Qian, F. et al. Curr. Protein Pept. Sci. (2016) 17 (4): 332-42]. The p38 MAPK-mediated signals initiate the activation of several transcriptional factors including CREB, ATF2 and Myc, as well as other kinases including MK2, but also MK3, MNK1/2, and MSK1/2 [Id., citing Obata, T. et al, (2000) Crit. Care Med. 28 (4) Suppl.: N67-N77; Dong, C. et al, "MAP kinases in the immune response," Annu. Rev. Immunol. (2002) 20: 55-72]. Among these distal kinases, the role of MK2 has been determined to be essential for the regulation of innate immune responses, including modulating production of inflammatory cytokines and chemokines, reactive oxygen species (ROS) and nitric oxide (NO). [Qian, F. et al. Curr. Protein Pept. Sci. (2016) 17 (4): 332-42].

As used herein, the terms "marker" or "cell surface marker" are used interchangeably herein to refer to an antigenic determinant or epitope found on the surface of a specific type of cell. Cell surface markers can facilitate the characterization of a cell type, its identification, and eventually its isolation. Cell sorting techniques are based on cellular biomarkers where a cell surface marker(s) may be used for either positive selection or negative selection, i.e., for inclusion or exclusion, from a cell population.

The term "mediated" and its various grammatical forms as used herein refers to depending on, acting by or connected through some intervening agency.

The term "memory cells" as used herein refers to B and T lymphocytes generated during a primary immune response that remain in a quiescent state until fully activated by a subsequent exposure to specific antigen (secondary immune response). Memory cells generally are more sensitive than naive lymphocytes to antigen and respond rapidly on reexposure to the antigen that originally induced them. During an immune response, naive T cells (T_{N}) are primed by antigen-presenting cells (APCs). Depending on the strength and quality of stimulatory signals, proliferating T cells progress along a differentiation pathway that culminates in the generation of terminally differentiated short-lived effector T (T_{EFF}) cells. When antigenic and inflammatory stimuli cease, primed T cells become quiescent and enter into the memory stem cell (T_{SCM}), central memory (T_{CM}) cell or effector memory (T_{EM}) cell pools, depending on the signal strength received. T_{SCM} cells possess stem cell-like attributes to a greater extent than any other memory lymphocyte population. Although both T_{CM} and T_{EM} cells can also undergo self-renewal, the capacity to form diverse progeny is progressively restricted, so that only T_{SCM} cells are capable of generating all three memory subsets and T_{EFF} cells; T_{CM} cells can give rise to T_{CM}, T_{EM} and T_{EFF} cells, and T_{EM} cells can only produce themselves and T_{EFF} cells. [Gattinoni, L. et al. Nature Revs. Cancer 12 (2012) 671-84].

The term "modulate" as used herein means to regulate, alter, adapt, or adjust to a certain measure or proportion.

The terms "multiple myeloma" or "myeloma" refer to a type of bone marrow cancer. It is a rare blood cancer that affects plasma cells.

As used herein, the term "mutation" refers to a change of the DNA sequence within a gene or chromosome of an organism resulting in the creation of a new character or trait not found in the parental type, or the process by which such a change occurs in a chromosome, either through an alteration in the nucleotide sequence of the DNA coding for a gene or through a change in the physical arrangement of a chromosome. Three mechanisms of mutation include substitution (exchange of one base pair for another), addition (the insertion of one or more bases into a sequence), and deletion (loss of one or more base pairs).

The term "naive T cell" as used herein refers to a T cell that has not previously been exposed to an antigen. Naive T cells are conventionally defined by coexpression of the RA isoform of the transmembrane phosphatase CD45, the lymph node homing molecules L-selectin (CD62L) and CCR7, and the costimulatory receptors CD27 and CD28. [De Rosa, SC et al. Nature Med. (2001) 7: 245-48].

The term "neoantigens" as used herein refers to newly formed antigens generated by tumor cells as a result of various tumor-specific alterations, such as genomic mutation, dysregulated RNA splicing, disordered post-translational modification, and integrated viral open reading frames.

The term "nuclear factor of activated T cells proteins" or "NFAT proteins" refers to a family of transcription factors whose activation is controlled by calcineurin, a calcium dependent phosphatase. During periods of sustained elevations of calcium, calcineurin dephosphorylates NFATC1-C4, allowing NFAT to translocate to the nucleus. This nuclear translocation is blocked by cyclosporine A (CSA), which blocks calcineurin activity. Once in the nucleus, NFAT binds to consensus DNA sites and controls gene transcription. Originally identified in T cells as inducers of cytokine gene expression, NFAT proteins play varied roles in cells outside of the immune system. [Horsley, V. and Pavlath, GK. J. Cell Biol. (2002) 156 (5): 771-4].

The abbreviation "NPκB" as used herein refers to a proinflammatory transcription factor that switches on multiple inflammatory genes, including cytokines, chemokines, proteases, and inhibitors of apoptosis, resulting in amplification of the inflammatory response [Barnes, PJ, (2016) Pharmacol. Rev. 68: 788-815]. The molecular pathways involved in NF-κB activation include several kinases. The classic (canonical) pathway for inflammatory stimuli and infections to activate NF-κB signaling involves the IKK (inhibitor of xB kinase) complex, which is composed of two catalytic subunits, IKK-α and IKK-β, and a regulatory subunit IKK-γ (or NFκB essential modulator [Id., citing Hayden, MS and Ghosh, S (2012) Genes Dev. 26: 203-234]. The IKK complex phosphorylates Nf-κB-bound IxBs, targeting them for degradation by the proteasome and thereby releasing NF-κB dimers that are composed of p65 and p50 subunits, which translocate to the nucleus where they bind to κB recognition sites in the promoter regions of inflammatory and immune genes, resulting in their transcriptional activation. This response depends mainly on the catalytic subunit IKK-β (also known as IKK2), which carries out IκB phosphorylation. The noncanonical (alternative) pathway involves the upstream kinase NF-xB-inducing kinase (NIK) that phosphorylates IKK-α homodimers and releases RelB and processes p100 to p52 in response to certain members of the TNF family, such as lymphotoxin-β [Id., citing Sun, SC. (2012) Immunol. Rev. 246: 125-140]. This pathway switches on different gene sets and may mediate different immune functions from the canonical pathway. Dominant-negative IKK-β inhibits most of the proinflammatory functions of NF-κB, whereas inhibiting IKK-α has a role only in response to limited stimuli and in certain cells, such as B-lymphocytes. The noncanonical pathway is involved in development of the immune system and in adaptive immune responses. The coactivator molecule CD40, which is expressed on antigen-presenting cells, such as dendritic cells and macrophages, activates the noncanonical pathway when it interacts with CD40L expressed on lymphocytes [Id., citing Lombardi, V et al. (2010) Int. Arch. Allergy Immunol. 151: 179-89].

The term "NKG2D" as used herein refers to an activating receptor expressed by all NK cells and subsets of T cells (γδ T cells, CD8+ T cells and CD4+ T cells) in humans. It is encoded by the KLRK1 gene (killer cell lectin-like receptor subfamily K, member 1). The NKG2D receptor functions as an activating receptor by virtue of its interactions with the signaling adaptor dimer DAP10 in humans and with DAP10 and DAP12 in mice [Raulet, DH et al. Annu. Rev. Immunol. (2013) 31: 4123-41, citing Champsaur, M. and Lanier, LL. Immunol. REev. (2010) 235: 267-85; Wu, J. et al. Science (1999) 285: 730-32]. When the receptor is ligated, DAP10 provides signals that recruit the p85 subunit of phosphatidylinositol 3-kinase (PI3K) and a complex of GRB2 and VAV1. Engagement of NKG2D on NK cells induces degranulation and cytokine production. However, engagement of NKG2D provides an enhancing or co-stimulatory signal for the activation of CD8+ T cells and probably other T cells.

NKG2D binds to several different ligands, all of which are homologous to MHC class I molecules but have no known role in antigen presentation [Id., citing Raulet, DH. Nat. Rev. Immunol. (2003) 3: 781-90; Champsaur, M. and Lanier, LL. Immunol. Rev. (2010) 235: 267-85; Eagle, RA and Trowsdale, J. Nat. Rev. Immunol. (2007) 7: 737-44; Machuldova, A. et al. Front. Immunol. (2021) 12: 651751, citing Stephens, HA. Trends Immunol. (2001) 22 (7): 378-85]. Like MHC proteins, they exhibit considerable allelic variation. In humans, the NKG2D ligands include MHC class I chain-related protein A (MICA) and MHC class I chain-related protein B (MICB), both encoded by genes in the MHC, and up to six different proteins called Unique long (UL)16-binding proteins (ULBPs), also known as retinoic acid early transcript 1 (RAET1) proteins. Like MHC proteins, the NKG2D ligands exhibit considerable allelic variation.

All NKG2D ligands are encoded by distinct genes in the host's own genome, i.e., the ligands are self-proteins. NKG2D ligands are expressed poorly or not at all by most normal cells but are upregulated in transformed/cancer cells, and virus-infected cells, and, in some cases, stressed cells. This type of recognition process, in which self-coded ligands for activating receptors are induced on unhealthy cells, has been termed "induced self recognition [Id. citing Diefenbach, A. and Raulet, DH. Immunol. Rev. (2001) 181: 170-84], which is distinct from "missing self recognition", a phenomenon in which loss of MHC ligands for NK inhibitory receptors sensitizes cells for elimination by NK cells. Various cellular pathways activated as a result of cellular stress, infection, or tumorigenesis regulate expression of the NKG2D ligands.

The structures of NKG2D-ligand complexes indicate that NKG2D binds diagonally over the α1 and α2 helices of the ligands, much as T-cell receptors bind over MHC molecules. Despite the poor homology of different ligands, some of the key residues that interact with NKG2D are conserved, and the NKG2D residues involved in binding are similar in the different structures.

The term "non-expanded" as used herein, is meant to refer to a cell population that has not been grown in culture (in vitro) to increase the number of cells in the cell population.

The term "objective response rate" or "ORR" as used herein refers to the percentage of people in a study or treatment group who have a partial response or complete response to the treatment within a certain period of time.

The term "overall survival" as used herein refers to the length of time from either the date of diagnosis or the start of treatment for a disease, such as cancer, that patients diagnosed with the disease are still alive.

The term "PAMPs" is an abbreviation for pathogen-associated molecular patterns. PAMPS are structural patterns present in components or products common to a wide variety of microbes, but not host cells. PAMS are ligands for pattern recognition molecules (PRMs).

The term "pattern recognition molecules" or "PRMs" as used herein refer to proteins recognizing PAMPs. Soluble PRMs include the collectins, acute phase proteins and NOD proteins. Membrane-bound PRMs are pattern recognition receptors.

The term "pattern recognition receptors" or "PRRs" refers to widely distributed membrane bound PRMs fixed in either the plasma membrane of a cell or in the membranes of its endocytic vesicles. Includes toll-like receptors (TLRs) and scavenger receptors. Engagement of PRRs induces pro-inflammatory cytokines.

The term "PD-1" or "programmed cell death protein 1" as used herein refers to an inhibitory receptor expressed on the surface of activated T cells. Its ligands, PD-L1 and PD-L2, are expressed on the surface of DCs or macrophages. PD-1 and its ligands PD-L1/PL-L2 act as co-inhibitory factors that can limit the development of the T cell response. PD-L1 is overexpressed on tumor cells or on non-transformed cells in the tumor microenvironment [Pardoll, DM. Nat. Rev. Cancer (2012) 12: 252-64]. PD-L1 expressed on the tumor cells binds to PD-1 receptors on the activated T cells, which leads to the inhibition of the cytotoxic T cells. These deactivated T cells remain inhibited in the tumor microenvironment.

The term "peptide" is used herein to designate a series of amino acid residues, connected one to the other typically by peptide bonds between the alpha-amino and carbonyl groups of the adjacent amino acids. Peptides are typically 9 amino acids in length, but can be as short as 8 amino acids in length, and as long as 14 amino acids in length. A series of amino acids are considered an "oligopeptide" when the amino acid length is greater than about 14 amino acids in length, typically up to about 30 to 40 residues in length. When the amino acid residue length exceeds 40 amino acid residues, the series of amino acid residues is termed a "polypeptide".

As used herein, the term "perforin" as used herein refers to a molecule that can insert into the membrane of target cells and promote lysis of those target cells. Perforin-mediated lysis is enhanced by enzymes called granzymes.

The terms "peripheral blood mononuclear cells" or "PBMCs" are used interchangeably herein to refer to blood cells having a single round nucleus such as, for example, a lymphocyte or a monocyte. PBMCs are a critical component in the immune system's responses to infections.

The term "priming" as used herein refers to the first encounter with a given antigen, which generates a primary adaptive immune response. The term "unprimed cells" (also referred to as virgin, naive, or inexperienced cells) as used herein refers to T cells and B cells that have generated an antigen receptor (TCR for T cells, BCR for B cells) of a particular specificity, but have never encountered the antigen. For example, before helper T cells and B cells can interact to produce specific antibody, the antigen-specific T cell precursors must be primed.

Priming involves several steps: antigen uptake, processing, and cell surface expression bound to class II MHC molecules by an antigen presenting cell, recirculation and antigen-specific trapping of helper T cell precursors in lymphoid tissue, and T cell proliferation and differentiation. [Janeway, CA, Jr., "The priming of helper T cells, Semin. Immunol. (1989) 1(1): 13-20]. Helper T cells express CD4, but not all CD4 T cells are helper cells. Id. The signals required for clonal expansion of helper T cells differ from those required by other CD4 T cells. The critical antigen-presenting cell for helper T cell priming appears to be a macrophage; and the critical second signal for helper T cell growth is the macrophage product interleukin 1 (IL-1). Id. If the primed T cells and/or B cells receive a second, co-stimulatory signal, they become activated T cells or B cells.

The term "progression" as used herein refers to the course of disease as it becomes worse or spreads in the body.

The term "progression-free survival" or PFS" as used herein refers to the length of time during and after the treatment of the disease that a patient lives with the disease but it does not get worse.

The term "proliferate" and its various grammatical forms as used herein is meant to refer to the process that results in an increase of the number of cells, and is defined by the balance between cell division and cell loss through cell death or differentiation.

The term "recurrent cancer" or "recurrence" means a cancer that has come back, usually after a period of time during which the cancer could not be detected. The cancer may come back to the same place as the primary tumor or to another place in the body.

The term "refractory cancer" or "resistant cancer" means a cancer that does not respond to treatment. The cancer may be resistant at the beginning of treatment or it may become resistant during treatment.

The term "relapse" refers to the return of a disease or the signs and symptoms of a disease after a period of improvement.

The terms "relapse-free survival" (RFS) or "disease-free survival" (DFS) mean the length of time after primary treatment for a cancer ends that the patient survives without any signs or symptoms of that cancer.

The term "scFv" as used herein refers to a class of engineered functional antibodies generated by the fusion of the variable heavy (V_{H}) and variable light (V_{H}) domains of an immunoglobulin through a short polypeptide linker.

The term "sign" as used herein refers to a healthcare provider's evidence of disease.

The term "SLAM" (or "signaling lymphocyte activation molecule") as used herein refers to a family of related cell-surface receptors that mediate adhesion between lymphocytes, that includes SLAM, 2B4, CD84, LLy106, Ly9 and CRACC.

The term "solid tumor" as used herein refers to an abnormal mass of tissue that usually does not contain cysts or liquid areas. Solid tumors may be benign (a growth that does not invade nearby tissue or spread to other parts of the body) or malignant (meaning to grow in an uncontrolled way; malignant tumors can invade nearby tissues and spread to other parts of the body through the blood and lymph system). Different types of solid tumors are named for the type of cells that form them. Types of solid tumors are sarcomas, carcinomas, and lymphomas; leukemias (cancers of the blood) generally do not form solid tumors.

A carcinoma is a cancer that begins in the skin or in tissues that line or cover internal organs. An adenocarcinoma is a cancer that forms in the glandular tissue that lines certain internal organs and makes and releases substances in the body, such as mucus, digestive juices, and other fluids. Most cancers of the breast, lung, esophagus, stomach, colon, rectum, pancreas, prostate, and uterus are adenocarcinomas.

A lymphoma is a malignant solid neoplasm of the lymphoid system, which produces immune cells. Abnormal lymphocytes become lymphoma cells, which multiply and collect in the lymph nodes. Over time, these cancerous cells impair the immune system. There are two categories of lymphomas: Hodgkin lymphoma and non-Hodgkin lymphoma. About 12 percent of people with lymphoma have Hodgkin lymphoma. Most non-Hodgkin lymphomas are B-cell lymphomas, and either grow quickly (high-grade) or slowly (low-grade). There are over a dozen types of B-cell non-Hodgkin lymphomas. The rest are T-cell lymphomas.

A sarcoma is a type of cancer that begins in bone or in the soft tissues of the body, including cartilage, fat, muscle, blood vessels, fibrous tissue, or other connective or supportive tissue. Bone and soft tissue sarcomas are the main types of sarcoma. Soft tissue sarcomas can develop in soft tissues like fat, muscle, nerves, fibrous tissues, blood vessels, or deep skin tissues. They can be found in any part of the body. Most of them start in the arms or legs. They can also be found in the trunk, head and neck area, internal organs, and the retroperitoneum. The different types of sarcoma are based on where the cancer forms. For example, osteosarcoma forms in bone, liposarcoma forms in fat, and rhabdomyosarcoma forms in muscle.

The term "somatic recombination" or V(D)J recombination" as used herein refers to site-specific recombination of pre-existing V, D and J gene segments in the immunoglobulin (Ig) and TCR loci to generate unique variable (V) exons. It is tightly regulated during T and B lymphocyte development to generate a highly diverse repertoire of receptors.

The term "standard of care" as used herein refers to treatment for a disease that is accepted and widely used by doctors.

As used herein, the term "stimulate" in any of its grammatical forms as used herein is meant to refer to inducing activation or increasing activity.

The term "stimulate an immune cell" or "stimulating an immune cell" as used herein is meant to refer to a process (e.g., involving a signaling event or stimulus) causing or resulting in a cellular response, such as activation and/or expansion, of an immune cell, e.g. a CD8+ T cell.

The terms "subject" or "individual" or "patient" are used interchangeably to refer to a member of an animal species of mammalian origin, including but not limited to, mouse, rat, cat, goat, sheep, horse, hamster, ferret, pig, dog, guinea pig, rabbit and a primate, such as, for example, a monkey, ape, or human.

The phrase "subject in need thereof' as used herein refers to an eligible patient that (i) will be administered an immunotherapy according to the present disclosure, (ii) is receiving at least one immunotherapy according to the present disclosure; or (iii) has received at least one immunotherapy according to the present disclosure, unless the context and usage of the phrase indicates otherwise.

The term "symptom" as used herein refers to a patient's subjective evidence of disease.

The term "T-bet" as used herein refers to a T_{H}1 cell transcription factor. Differential expression of the T_{H}1 cell transcription factor T bet and a closely related T-box family transcription factor particularly in CD8+ T cells, Eomesodermin (Eomes) facilitates the cooperative maintenance of the pool of antiviral CD8+ T cells during chronic viral infection. [Paley, MA et a., Science (2012) 338: 1220-125]. During chronic infections, T-bet is reduced in virus-specific CD8+ T cells; this reduction correlates with T cell dysfunction. In contrast, Eomes mRNA expression is up-regulated in exhausted CD8+ T cells during chronic infection. [Id.]

The terms "T cell" or "T lymphocyte" or are used interchangeably to refer to cells that mediate a wide range of immunologic functions, including the capacity to help B cells develop into antibody-producing cells, the capacity to increase the microbicidal action of monocytes/macrophages, the inhibition of certain types of immune responses, direct killing of target cells, and mobilization of the inflammatory response. These effects depend on their expression of specific cell surface molecules and the secretion of cytokines. T cells recognize antigens on the surface of antigen presenting cells (APCs) and mediate their functions by interacting with, and altering, the behavior of these APCs. T cells can also be classified based on their function as helper T cells; T cells involved in inducing cellular immunity; suppressor T cells; and cytotoxic T cells. T-cell activation is dependent on the interaction of the TCR/CD3 complex with its cognate ligand, a peptide bound in the groove of a class I or class II MHC molecule. The molecular events set in motion by receptor engagement are complex. Among the earliest steps appears to be the activation of tyrosine kinases leading to the tyrosine phosphorylation of a set of substrates that control several signaling pathways. These include a set of adaptor proteins that link the TCR to the ras pathway, phospholipase Cγ1, the tyrosine phosphorylation of which increases its catalytic activity and engages the inositol phospholipid metabolic pathway, leading to elevation of intracellular free calcium concentration and activation of protein kinase C, and a series of other enzymes that control cellular growth and differentiation. Full responsiveness of a T cell requires, in addition to receptor engagement, an accessory cell-delivered costimulatory activity, e.g., engagement of CD28 on the T cell by CD80 and/or CD86 on the antigen presenting cell (APC). The ultimate amplitude and quality of the T cell immune response, which is initiated through antigen recognition by the TCR, is regulated by a balance between co-stimulatory and inhibitory signals (immune checkpoints).

Although the lineage relationship between T cell subsets remains controversial, T cells cluster in populations that can be arranged as a progressive continuum on the basis of phenotypic, functional and transcriptional attributes. T lymphocytes transition through progressive stages of differentiation that are characterized by a stepwise loss of functional and therapeutic potential in the order from naive T (T_{N}) cells to T memory stem cells (T_{SCM}) (the most immature antigen experienced T cells), to T central memory (T_{CM}) cells, which patrol central lymphoid organs, to T effector memory (T_{EM}) cells, which patrol peripheral tissues. In contrast to T_{N} cells, memory T cells are capable of rapidly releasing cytokines on restimulation. TCM cells more efficiently secrete IL-2 and T_{EM} have an increased capacity for IFNγ release and cytotoxicity. All antigen-experienced T cells upregulate the common IL-2 and IL-15β receptor (IL-2Rβ) conferring the ability to undergo homeostatic proliferation in response to IL-15, and also display high amounts of CD95 (also known as FAS), a receptor that provides either costimulatory or pro-apoptotic signals depending on the efficiency of CD95 signaling complex formation and on which particular intracellular signaling proteins are part of the complex. [Gattinoni, L. et al. Nature Revs. Cancer 12: 671-684].

The term "T cell antigen" as used herein is meant to refer to a protein, lipid (for CD1) or fragment thereof which can be processed into a peptide that can bind to either Class I MHC, Class II MHC, non-classical MHC, or CD1 family molecules (collectively antigen presenting molecules), and in this combination can engage a T cell receptor on a T cell.

The term "T cell epitope" as used herein is meant to refer to a short peptide molecule that binds to a class I or II MHC molecule and that is subsequently recognized by a T cell. T cell epitopes that bind to class I MHC molecules are typically 8-14 amino acids in length, and most typically 9 amino acids in length. T cell epitopes that bind to class II MHC molecules are typically 12-20 amino acids in length. In the case of epitopes that bind to class II MHC molecules, the same T cell epitope may share a common core segment, but differ in the length of the carboxy- and amino-terminal flanking sequences due to the fact that ends of the peptide molecule are not buried in the structure of the class II MHC molecule peptide-binding cleft as they are in the class I MHC molecule peptide-binding cleft.

The term "T cell factor 1" or (TCF-1), encoded by Tcf7, as used herein refers to the key transcription factor of the canonical Wnt signaling pathway. The Wnt signaling pathway is evolutionarily conserved and regulates a variety of fundamental processes such as development, cell-fate specification, and maintenance of tissue homeostasis. In mature T cells, TCF-1 is known to be critical for the generation of the CD8+ T cell memory response. [Escobar, G. et al. Sci Immunol. (2020) 5 (53): eabb9726, citing Raghu;, D. et al. Trends Immunol. (2019) 40: 1149-62].

The term "T cell receptor" (TCR) as used herein refers to a complex of integral membrane proteins that participate in the activation of T cells in response to an antigen. The TCR expressed by the majority of T cells consisting of a heterodimer of α and β chains. A small group of T cells express receptors made of γ and δ chains. Among the α/β T cells are two sublineages: those that express the coreceptor molecule CD4 (CD4+ cells), and those that express CD8 (CD8+ cells). These cells differ in how they recognize antigen and in their effector and regulatory functions. The TCR is composed of four distinct signal transducing subunits (CD3-gamma (γ), -delta (δ), -epsilon-(ε), and zeta (ζ)) that share a common functional sequence, the immunoreceptor tyrosine-based activation motif (ITAM) [Shores, EW et al. J. Exp. Med. (1997) 185 (5): 893-900, citing Robey, E. and Fowlkes, BJ. Annu. Rev. Immunol. (1994) 12: 675-705] within their intracytoplasmic domains [Id., citing Reth, M. Nature (Lond) (1989): 338: 383-4; Samelson, LE and Klausner, RD. J Biol. Chem. (1992) 267: 24913-6]. After TCR engagement, phosphorylation of ITAMs leads to the recruitment of SH2 domain-containing proteins (e.g., tyrosine kinases) to the TCR complex and initiation of the T cell activation cascade [Id., citing Samelson, LE and Klausner, RD. J Biol. Chem. (1992) 267: 24913-6; Weiss, A., and Littman, DR. Cell (1994) 76: 263-74; Irving, BA and Weiss, A. Cell (1991) 64: 891-901; Romeo, C. et al. Cell (1992) 68: 889-97]. The CD3 subunits each contain a single ITAM, whereas ζ contains three ITAMs within its longer cytoplasmic tail. ITAM sequences are conserved but nonidentical. Irving et al constructed a chimeric protein linking the extracellular and transmembrane domains of CD8 to the cytoplasmic domain of the zeta chain and demonstrated that the CD8/zeta chimera was expressed independently of the TCR and was capable of transducing signals that, by criteria of early and late activation, were indistinguishable from those generated by the intact TCR. [Irving, BA and Weiss, A. Cell (1991) 64: 891-901]. Their data showed that CD8/zeta can activate the appropriate signal transduction pathways in the absence of CD3 gamma, delta, and epsilon, and suggested that the role of CD3 zeta is to couple the TCR to intracellular signal transduction mechanisms.

The term "helper T cells" or "T_{H}" cells as used herein refers to effector CD4 T cells that stimulate or "help" B cells to make antibody in response to antigenic challenge. T_{H}2, T_{H}1 and the T_{H}F subsets of effector CD4 T cells can perform this function.

The term "T follicular helper (T_{H}F) cells" as used herein refers to a distinct subset of CD4+ T lymphocytes, specialized in B cell help and in regulation of antibody responses. They develop within secondary lymphoid organs (SLO) and can be identified based on their unique surface phenotype, cytokine secretion profile, and signature transcription factor. They support B cells to produce high-affinity antibodies toward antigens in order to develop a robust humoral immune response and are crucial for the generation of B cell memory. They are essential for infectious disease control and optimal antibody responses after vaccination. Stringent control of their production and function is critically important, both for the induction of an optimal humoral response against thymus-dependent antigens but also for the prevention of self-reactivity. [Gensous, N. et al. Front. Immunol. (2018) doi.org/10.3389/fimmu.2018.01637).

The term "T_{H}1 cells" as used herein refers to a lineage of CD4+ effector T cells that promotes cell-mediated immune responses and is required for host defense against intracellular viral and bacterial pathogens. They are mainly involved in activating macrophages but can also help stimulate B cells to produce antibody. T_{H}1 cells secrete IFN-gamma, IL-2, IL-10, and TNF-alpha/beta. IL-12 and IFN-γ make naive CD4+ T cells highly express T-bet and STAT4 and differentiate to T_{H}1 cells. [Zhang, Y. et al. Adv. Exp. Med. Bio. (2014) 841: 15-44].

The term "T_{H}2 cells" as used herein refers to a lineage of CD4+ effector T cells that secrete IL-4, IL-5, IL-9, IL-13, and IL-17E/IL-25. These cells are required for humoral or antibody-mediated immunity and play an important role in coordinating the immune response to large extracellular pathogens. IL-4 makes naive CD4+ T cells highly express STAT6 and GATA3 and differentiate to T_{H}2 cells. (Zhang, Y. et al. Adv. Exp. Med. Bio. (2014) 841: 15-44)/

The term "T_{H}17 cells" as used herein refers to a CD4+ T-cell subset characterized by production of interleukin-17 (IL-17). IL-17 is a highly inflammatory cytokine with robust effects on stromal cells in many tissues, resulting in production of inflammatory cytokines and recruitment of leukocytes, especially neutrophils, thus creating a link between innate and adaptive immunity. [Tesmer, LA, et al., Immunol. Rev. (2008) 223: 87-113]. The key transcription factor in T_{H}17 cell development is RORyt.

The term "Tpex" as used herein refers to progenitors or precursors of exhausted T cells. [Utzschneider, DT et al. Nature Immunol. (2020) 21: 1256-66].

The term "Treg" or "regulatory T cells" as used herein refers to effector CD4 T cells that inhibit T cell responses and are involved in controlling immune reactions and preventing autoimmunity. The natural regulatory T cell lineage that is produced in the thymus is one subset. The induced regulatory T cells that differentiate from naive CD4 T cells in the periphery in certain cytokine environments is another subset. Tregs are most commonly identified as CD3+CD4+CD25+FoxP3+ cells in both mice and humans. Additional cell surface markers include CD39, 5' Nucleotidase/CD73, CTLA-4, GITR, LAG-3, LRRC32, and Neuropilin-1. Tregs can also be identified based on the secretion of immunosuppressive cytokines including TGF-beta, IL-10, and IL-35. Cell surface molecules CTLA-4, LAG-3, and neuropilin-1 (Nrp1) impair dendritic cell (DC)-mediated conventional T cell activation: CTLA-4 and LAG-3 outcompete CD28 and T cell receptor expressed on conventional T cells for binding to CD80/86 and MHC class II on DCs, and Nrp1 stabilizes DC-Treg contact, thereby preventing antigen presentation to conventional T cells [Ikebuchi, R. et al. Front. Immunol. (2019) doi.org/10.3389/fimmu.2019.01098].

The term "targeted therapy" as used herein refers to a type of cancer treatment that targets proteins that control how cancer cells grow, divide and spread.

The term "therapeutic effect" as used herein is meant to refer to a consequence of treatment, the results of which are judged to be desirable and beneficial. A therapeutic effect can include, directly or indirectly, the arrest, reduction, or elimination of a disease manifestation. A therapeutic effect can also include, directly or indirectly, the arrest reduction or elimination of the progression of a disease manifestation.

The term "therapeutic window" as used herein is meant to refer to a concentration range that provides therapeutic efficacy without unacceptable toxicity. In a drug context, following administration of a dose of a therapeutic agent/drug, its effects usually show a characteristic temporal pattern. A lag period is present before the drug concentration exceeds the minimum effective concentration ("MEC") for the desired effect. Following onset of the response, the intensity of the effect increases as the drug continues to be absorbed and distributed. This reaches a peak, after which drug elimination results in a decline in the effect's intensity that disappears when the drug concentration falls back below the MEC. Accordingly, the duration of a drug's action is determined by the time period over which concentrations exceed the MEC. The therapeutic goal is to obtain and maintain concentrations within the therapeutic window for the desired response with a minimum of toxicity. Drug response below the MEC for the desired effect will be subtherapeutic, whereas for an adverse effect, the probability of toxicity will increase above the MEC. Increasing or decreasing drug dosage shifts the response curve up or down the intensity scale and is used to modulate the drug's effect. Increasing the dose also prolongs a drug's duration of action but at the risk of increasing the likelihood of adverse effects. Accordingly, unless the drug is nontoxic, increasing the dose is not a useful strategy for extending a drug's duration of action.

The treatment of solid tumors by CAR-T cells is complex, multifactorial and has a narrower therapeutic window than does the targeting of CD19 for the treatment of B cell leukemia and non-Hodgkin lymphoma. [Watanabe, et al. Front. Immunol. (2018) 9: 2486]. Possible approaches to expand the therapeutic window of CAR-T cell therapy include:
(1) optimizing CAR affinity and sensing (with the caveat that increasing the affinity of CAR-T cells may reduce or prevent serial killing, promote T cell exhaustion, decrease the generation and persistence of T_{CM} and T_{EFF} [Watanabe, citing Caserta, S. et al. J. Immunol. (2010) 185: 6545-54] or increase the loss of T cells through activation-induced cell death [Id., citing Engels, B. et al. Mol. Ther. (2012) 20: 652-60]; and/or combinatorial antigen recognition through two different antigens on tumor cells. In one such example, CAR-1 can drive only the activation signal (signal 1 of CD3zeta, and CAR2 can drive only co-stimulation (signal 2) through costimulatory molecules (AND logic gated CAR) or OR logic gated CAR [Id., citing Wilkie, S. et al. J. Clin. Immunol. (2012) 32: 105-70; Kloss, CC et al. Nat. Biotechnol. (2013) 31: 71-5; Grada, Z. et al. Mol. Ther. Nucleic Acids (2013) 2: e105; Hegde, M. et al. Mol Ther. (2013) 21: 2087-101; Hegde, M. et al. J. Clin. Invest. (2016) 126: 3036-52];
(2) optimizing immunological synapse formation; e.g., CD28 plus 4-1BB based third generation CARS have been found superior to CD28 based second generation CARS as measured by immunosynapse structure, signaling and function [Id., citing Xiong, W. et al. Mol. Ther. (2018) 26: 963-75];
(3) combination therapies for overcoming tumor heterogeneity and thereby expanding the therapeutic window using oncolytic viruses [Id., citing Watanabe, K. et al. JCI Insight (2018) 3: 99573; Nishio, N. et al. Cancer Res. (2014) 4: 5195-205; Rosewell, Shaw, A. et al. Mol. Ther. (2017) 25: 2440-51; Wing, A. et al. Cancer Immunol. Res. (2018) 6: 605-16];
(4) local delivery of CAR-T cells and therapeutic agents into the tumor bed [Id., citing Tchou, J. et al. Cancer Immunol. Res. (2017) 5: 1152-61];
(5) induction of target antigen expression [Id., citing Garnett, CT et al. Cancer Res. (2004) 64: 7985-94; Hiraga, J. et al. Blood (2009) 113: 4885-93]; or
(6) other modifications to enhance safety.

The term "TIGIT" as used herein refers to a member of the Ig super family and an immune inhibitory receptor.

The term "TIM-3" as used herein refers to a transmembrane protein and immune checkpoint receptor. It is associated with tumor-mediated immune suppression.

The term "tissue-resident memory T cell" or "T_{RM}" as used herein refers to memory lymphocytes that do not migrate after taking up residence in barrier tissues, where they are retained long term. They appear to be specialized for rapid effector function after restimulation with antigen or cytokines at sites of pathogen entry.

The term "tolerance" as used herein refers to a failure to respond to a particular antigen. Tolerance mechanisms that operate in the thymus before the maturation and circulation of T cells are referred to as "central tolerance." Not all antigens of which T cells need to be tolerant are expressed in the thymus, and therefore central tolerance mechanisms alone are insufficient. Additional tolerance mechanisms exist to restrain the numbers and or function of T cells that are reactive to developmental or food antigens, which are not typically expressed. Tolerance acquired by mature circulating T cells in the peripheral tissues is called "peripheral tolerance."

The term "toll-like receptors" or "TLR" as used herein refers to membrane bound pattern recognition receptors (PRRs) that bind to pathogen products.

The terms "TOX" or "thymocytes selection-associated HMG BOX" as used herein refers to a member of a family of transcriptional factors that contain the highly conserved high mobility group box (HMG-box) region. Increasing studies have shown that TOX is involved in maintaining tumors and promoting T cell exhaustion. [Liang, C. et al. Biomark Res. (2021) 9: 20].

The term "toxicity" as used herein refers to the degree to which a substance can harm humans or animals. Acute toxicity involves harmful effects in an organism through a single or short-term exposure.

The term "TRAC" (T cell Receptor Alpha Constant) refers to a gene encoding the constant region of the T cell receptor alpha chain.

The term "transdifferentiation" as used herein refers to the conversion of one mature cell type into another.

The term "transduction" as used herein refers to a process whereby foreign DNA is introduced into another cell via a viral vector.

The term "transfection" as used herein refers to the process of introducing a foreign DNA molecule into a eukaryotic cell by nonviral methods.

The term "treat" or "treating" includes abrogating, substantially inhibiting, slowing or reversing the progression of a disease, condition or disorder, substantially ameliorating clinical or esthetical symptoms of a condition, substantially preventing the appearance of clinical or esthetical symptoms of a disease, condition, or disorder, and protecting from harmful or annoying symptoms. Treating further refers to accomplishing one or more of the following: (a) reducing the severity of the disorder; (b) limiting development of symptoms characteristic of the disorder(s) being treated; (c) limiting worsening of symptoms characteristic of the disorder(s) being treated; (d) limiting recurrence of the disorder(s) in patients that have previously had the disorder(s); and (e) limiting recurrence of symptoms in patients that were previously asymptomatic for the disorder(s).

The term "trogocytosis" as used herein refers to a cellular process whereby a cell acquires a membrane fragment from another cell in a contact-dependent manner allowing for the transfer of surface proteins with functional integrity. [Mattei, F. et al. Science (2022) 25 (10): 105110; doi.org/10.1016/j.isci.2022.105110].

The term "tumor associated antigen" or "TAA" refers to a protein or other molecule that has elevated levels on tumor cells but that is also expressed at lower levels on healthy cells.

The term "tumor associated macrophages" or "TAMs" as used herein refers to an immunosuppressive macrophage subtype found in the tumor microenvironment that is involved in the progression and metastasis of cancer. TAMs are broadly considered M2-like, which can be further classified into the M2a phenotype (induced by IL-4 or IL-13), M2b phenotype (IL-10 high, IL-12 low) and M2c phenotype (TNF-α low) according to distinct signal stimuli. They produce abundant growth factors, extracellular matrix (ECM) remodeling molecules and cytokines for the regulation of cancer proliferation via noncoding RNAs, exosomes and epigenetics [Yan, S. and Wan, G. The FEBS Journal (2021) 288 (21): 6174-86,, citing Qian, BZ and Pollard, JW. (Cell (2010) 141: 39-51]. Activated M2 macrophages distinctively express arginase 1 (ARG1). TAMs can demonstrate direct inhibition on the cytotoxicity of T-lymphocytes through multiple mechanisms and characteristics of tumor evolution, including immune checkpoint engagement via expression, production of inhibitory cytokines [such as IL-10 and transforming growth factor (TGF)-β] and metabolic activities consisting of depletion of l-arginine (or other metabolites) and the production of reactive oxygen species (ROS). The suppressive immune response renders cancer cells capable of escaping from immune surveillance.

The term "tumor infiltrating lymphocytes" or "TILs" as used herein refers to a heterogeneous lymphocyte population mainly composed of T lymphocytes that may consist of numerous antitumor effector and/or regulatory T cells (Tregs) hat have invaded a tumor tissue; TILs are key players in the host's immune response to a tumor. [Wang, J. et al. BMC Cancer (2020) 20: 731].

The term "tumor microenvironment" or "TME" as used herein refers to the dynamic and complex ecosystem in which tumor cells exist.

The term "TME macrophages" as used herein refers to macrophages that arise primarily from bone marrow-derived monocytes that are recruited by tumor or stroma-derived chemokines such as colony-stimulating factor 1 (CSF1; also known as M-CSF) and CCL2, to the tumor microenvironment. M1 and M2 phenotypes are differentiated in response to different signal stimuli and are polarized according to the TME, exhibiting strong plasticity, such that macrophages adopt context-dependent phenotypes when stimulated [Yan, S. and Wan, G. The FEBS Journal (2021) 288 (21): 6174-86, citing Murry, PJ and Wynn, TA. Nat. Rev. Immunol. (2011) 11: 723-37]. Antitumorigenic M1 macrophages express high levels of tumor necrosis factor (TNF), inducible nitric oxide synthase (iNOS; also known as NOS2) and major histocompatibility complex (MHC) class II molecules, whereas pro-tumorigenic M2 macrophages are marked with high levels of arginase 1 (ARG1), interleukin (IL)-10, CD163, CD204 or CD206 expression. The activation of primary macrophages into M1 or M2 phenotype is mainly induced by interferon-regulatory factor/signal transducer and activator of transcription (STAT) signaling pathways [[Id., citing Waqas, SFH et al. in Nuclear Receptors: Methods and Experimental Protocols, MZ Badr. Ed., Springer, New York, NY, pp. 211-24].

The term "tumor specific antigens" or ("TSA") refers to a protein or other molecule found on cancer cells only.

The terms "tumorigenesis" "oncogenesis" and "carcinogenesis" are used interchangeably to refer to the transformation of normal cells into cells-of-origin (COOs) and the development of cells-of-origin into tumors.

The term "variable (V) domain" as used herein refers to the structural unit of an immunoglobulin or TCR chain that is encoded by the corresponding variable (V) exon.

The term "variable (V) region" as used herein refers to the highly variable N-terminal portion of an Ig or TCR molecule composed of the variable domain that contain the antigen binding site.

The term "zeta-chain-associated protein kinase 70" or "ZAP-70" as used herein refers to a non-src family protein kinase that associates with phosphorylated CD3 zeta chain and plays an important role in TCR-CD3 complex signaling. The main substrate of ZAP70 is the transmembrane adaptor LAT.

### Embodiments

According to one aspect, the present disclosure provides a scalable and practical off the shelf allogeneic CAR-T cell immunotherapy for treating cancer, comprising
a) generating by transduction with a viral vector encoding a protein of a virus an expanded population of allogeneic virus-specific T cells (VSTs) comprising T cell receptors (TCRs) specific for the protein of the virus;
b) genetically modifying the population of allogeneic VSTs to stably express up to three chimeric antigen-receptors (CARs), wherein each CAR is specific for a cancer antigen,
   wherein to reduce risk of host versus graft rejection (HVGR), one or more of the CARs comprise:
      (i) a-CD30 CAR expressing a CD30-specific scFv fragment, or
      (ii) a chimeric alloimmune defense receptor (ADR), or
      (iii) a chimeric HLA accessory receptor fused with a cytolytic endodomain of T cell receptor zeta chain, or
      (iv) a-CD30 CAR expressing a CD30-specific scFv fragment, and a chimeric HLA accessory receptor fused with a cytolytic endodomain of T cell receptor zeta chain; or
      (v) a-CD30 CAR expressing a CD30-specific scFv fragment, and a chimeric alloimmune defense receptor (ADR);
      (vi) a chimeric alloimmune defense receptor (ADR), and a chimeric HLA accessory receptor fused with a cytolytic endodomain of T cell receptor zeta chain,
      (vii) or a combination thereof;
   to create a population of cancer killing VST-CAR-T cells;
c) expanding the population of cancer killing VST-CAR-T cells in the presence of one or more cytokines in vitro to achieve a therapeutic dose of at least 40 × 10⁶ to 800 × 10⁶ cancer killing T cells; and
d) infusing eligible patients with the population of VST-CAR-T cells one or more times in an allogeneic setting;
e) creating VST-CAR-T cells that express a nonexhausted PD-1^{neg}, TIGIT^{neg} TCF-1^{hi}TOX^{lo} early T stem cell (T_{SCM}) phenotype by:
   (i) enriching for a population of CD4+ T cells, CD8+ T cells or a combination thereof that express a nonexhausted PD-1^{neg}, TIGIT^{neg} TCF-1^{hi}TOX^{lo} early T stem cell (T_{SCM}) phenotype;
   (ii) generating by transducing the population of CD4+ T cells, CD8+ T cells or a combination thereof with a viral vector encoding a protein of a virus an expanded population of allogeneic virus-specific T cells (VSTs) comprising T cell receptors (TCRs) specific for the protein of the virus;
   (iii) genetically modifying the population of allogeneic VSTs in (ii) to stably express up to three chimeric antigen-receptors (CARs), wherein each CAR is specific for a cancer antigen, wherein to reduce risk of host versus graft rejection (HVGR), one or more of the CARs comprise:
      (1) a-CD30 CAR expressing a CD30-specific scFv fragment, or
      (2) a chimeric alloimmune defense receptor (ADR), or
      (3) a chimeric HLA accessory receptor fused with a cytolytic endodomain of T cell receptor zeta chain, or
      (4) a-CD30 CAR expressing a CD30-specific scFv fragment, and a chimeric HLA accessory receptor fused with a cytolytic endodomain of T cell receptor zeta chain; or
      (5) a-CD30 CAR expressing a CD30-specific scFv fragment, and a chimeric alloimmune defense receptor (ADR); or
      (6) a chimeric alloimmune defense receptor (ADR), and a chimeric HLA accessory receptor fused with a cytolytic endodomain of T cell receptor zeta chain, or
      (7) a combination thereof; and
f) iteratively dosing the patient with the PD-1^{neg}, TIGIT^{neg} TCF-1^{hi}TOX^{lo} early stem cell T cell (T_{SCM}) phenotype VST-CAR-T of step (e) as needed until all cancer in the body is destroyed.

The advantages of the disclosed therapy are that it can limit GVHD potential, it can prevent host-versus graft rejection responses; it allows the creation of batches of the therapy that can be available if redosing is necessary without a prolonged waiting time; and by iteratively dosing the patient with the early stem cell phenotype, the cell therapy has enhanced persistence with an enhanced proliferative potential, compared to a standard CAR therapy, i.e., it remains effective until the cancer in the body of the recipient is destroyed.

### a) generating by transduction with a viral vector encoding a protein of a virus an expanded population of allogeneic virus-specific T cells (VSTs) comprising T cell receptors (TCRs) specific for the protein of the virus (allogeneic virus-specific VSTs);

### Allogeneic T cell sources

According to some embodiments, the allogeneic T cells are derived from umbilical cord blood (UCB). UCB contains an abundance of immune cells, making it a promising source for cellular immunotherapy. [Liu, D-D et al. Frontiers Oncol. (2022) 12: 944248, citing Balassa, K. and Rocha, V. Expert Opin. Biol. Ther. (2018) 18 (2): 121-34; Bachiller, M. et al. J. Immunother. Cancer (2021) 9 (8) doi: 10.1136/jitc-2021-002866. UCB contains T cells with completely different immunological and phenotypic properties from peripheral blood. [Cael, B. et al. , Cancers (2022) 14: 3168, citing Barker, J. et al. Blood (2015) 126: 2778-9; Nitsche, A. et al. BMC Immunol. (2007) 8: 18; Lin, Y. et al. Stem Cell Investig. (2019) 6: 35, citing Li, Y. et al. Transpl. Immunol. (2007) 18: 151-8; Canto, E. et al. Transplanatation (2005) 80: 850-8]. More than 85% of UCB derived T cells have a naive phenotype, allowing them to induce fewer graft versus host disease during transplantation [Id., citing Kwoczek, J. et al. Transfusion (2018) 58: 88-99]. Moreover, they express significantly lower markers of exhaustion (PD1, LAG3, TM3) in comparison to PB-derived T cells, allowing them to have long-term persistence and efficiency. [Cael, B. et al., Cancers (2022) 14: 3168, citing Lin, Y. et al. Stem Cell Invest. (2019) 6: 35]. Cord blood T cells were reported to mediate enhanced antitumor responses compared with peripheral blood T cells in a murine model of B cell lymphoma, which correlated with increased tumor-homing of CCR7high cord blood CD8+ T cells and rapid gain of cytotoxic and Th1 function. [Hiwarkar, P. et al. Blood (2015) 126 (26): 2882-91]. The production of UCB-derived CARTs has been described. [Liu, D-D et al. Frontiers Oncology (2022) 12: 944248; Cael, B. et al. Cancers (2022) 14: 3168].

According to some embodiments, the T cells can be derived from the peripheral blood (PB) of screened healthy adult donors. According to some embodiments, peripheral blood stem cells can be mobilized with an infusion of G-CSF. According to some embodiments, the peripheral blood is obtained by a blood draw by venipuncture. According to some embodiments, the mononuclear cell (MC) population can be collected from screened healthy volunteer donors by leukapheresis, enriched for PBMCs and cryopreserved; the other blood components are returned to the donor.

According to some embodiments, mononuclear cells can be isolated from CB and PB by centrifugation using a density gradient (e.g., Ficoll-Paque) and stored and transported below -120°C until needed.

According to some embodiments, CD3+ T cells can be isolated by negative selection, e.g., using Miltenyi Pan T-cell Isolation Kit, human (130-096-535). Non-target cells, i.e., monocytes, neutrophils, eosinophils, B cells, stem cells, dendritic cells, NK cells, granulocytes, erythroid cells are labeled using a cocktail of biotin-conjugated antibodies. The cocktail contains antibodies against CD14, CD15, CD16, CD19, CD34, CD36, CD56, CD123, and CD235a (Glycophorin A). Subsequently, non-target cells are magnetically labelled with the Pan T Cell MicroBead Cocktail. The non-T cells are retained in a MAXS Column placed in a MACS Separator, while the unlabeled T cells pass through the column and are collected as the enriched, unlabeled T cell fraction.

Experience with infusions of allogeneic MV-VSTs shows that they are well tolerated and reduce the risk of GvHD when administered to immunosuppressed transplant recipients receiving partially HLA-matched and halploidentical stem cell grafts [see, e.g., Haque, T. et al. Blood (2007) 110: 1123-31; Leen, AM et al. Blood (2009) 114 (19): 4283-92 (EBV and adenovirus); Smith, C. et al. Cancer Res. (2012) 72 (5): 1116-25 (AdV); Blyth, E. et al. Blood (2013) 121 (18): 3745-58 (CMV); Melenhorst, JJ et al. Molecu. Therapy (2015) 23 (1): 179-83; Muftuoglu, M. et al. New Engl. J. Med.(2018) 379: 443-53 (BK virus);Tzannou, I. et al. J. Clinical Oncol. (2017) 35 (31): 3547-57 (EBV, AdV, CMV, BK virus (BKV); human herpesvirus6 (HHV6); Tzannou, I. et al. Blood Advances (2019) 3 (17): 2571-80 (CMV); Vasilelou, S. et al Haematologica (2020) 105 (1): 235-43 (respiratory syncytial virus (RSV); influenza A, parainfluenza virus 3 (PIV3); human metapneumovirus (hMPV)); Prockop, S. et al. J. Clin. Invest. (2020) 130(2): 733-47 (EBV); Olson, A. et al. J. Clinical Oncol. (2021) 39: 2710-9; Vasileiou, S. et al. Haematologica (2022) Nov. 10. Doi: 10.3324/haematol.2022,281946 (SARS-CoV-2)].

### Viruses

A virus particle is composed of a viral genome of nucleic acid that is surrounded by a protein coat (capsid). Many animal viruses are surrounded by an outer lipid envelope, which they acquire from the host cell membrane as they leave the virus-infected cell.

Viral genomes may be double- or single-stranded DNA (a DNA virus), or double- or single-stranded RNA (an RNA virus).The Baltimore classification of viruses [Baltimore, D., 1974 Haravey Lecture 70 Series: 57-74) is based on the viral mechanism of mRNA production. Viral genomes may be single-stranded (ss) or double stranded (ds), RNA or DNA, and may or may not use reverse transcriptase. Additionally, single stranded RNA viruses may be either positive sense (+) or negative or antisense (-). This classification places viruses into seven groups, as shown in Table 4:

**Table 4: Baltimore classification of viruses**

| **Group** | **Examples** |
|---|---|
| ds DNA viruses | Adenoviruses, Herpesviruses, Poxviruses |
| ds DNA viruses | + sense DNA (Parvoviruses) |
| dsRNA viruses | Rheoviruses |
| (+) ssRNA viruses | Picornaviruses, Togaviruses |
| (-) ssRNA viruses | Orthomyxoviruses, Rhabdoviruses |
| ssRNA-RT viruses | Retroviruses |
| dsDNA-RT viruses | Hepadnaviruses |

Table 5, taken from https://viralzone.expasy.org/678 (visited Apr. 18, 2023), displays an exemplary list of human viral pathogens, their host, transmission and disease.

**Table 5. Human viral pathogens**

| **Virus** | **Genus, Family** | **Host** | **Transmission** | **Disease** |
|---|---|---|---|---|
| Adeno-associated virus | Dependovirus, Parvoviridae | Human, vertebrates | Respiratory | None |
| Aichi virus | Kobuvirus, Picornaviridae | Human | Fecal-oral | Gastroenteritis |
| Australian bat lyssavirus | Lyssavirus, Rhabdoviridae | Human, bats | Zoonosis, animal bite | Fatal encephalitis |
| BK polyomavirus | Polyomavirus, Polyomaviridae | Human | Respiratory fluids or urine | None |
| Banna virus | Seadornavirus, Reoviridae | Human, cattle, pig, mosquitoes | Zoonosis, arthropod bite | Encephalitis |
| Barmah forest virus | Alphavirus, Togaviridae | Human, marsupials, mosquitoes | Zoonosis, arthropod bite | Fever, joint pain |
| Bunyamwera virus | Orthobunyavirus, Bunyaviridae | Human, mosquitoes | Zoonosis, arthropod bite | Encephalitis |
| Bunyavirus La Crosse | Orthobunyavirus, Bunyaviridae | Human, deer, mosquitoes, tamias | Zoonosis, arthropod bite | Encephalitis |
| Bunyavirus snowshoe hare | Orthobunyavirus, Bunyaviridae | Human, rodents, mosquitoes | Zoonosis, arthropod bite | Encephalitis |
| Cercopithecine herpesvirus | Lymphocryptovirus, Herpesviridae | Human, monkeys | Zoonosis, animal bite | Encephalitis |
| Chandipura virus | Vesiculovirus, Rhabdoviridae | Human, sandflies | Zoonosis, athropod bite | Encephalitis |
| Chikungunya virus | Alphavirus, Togaviridae | Human, monkeys, mosquitoes | Zoonosis, arthropod bite | Fever, joint pain |
| Cosavirus A | Cosavirus, Picomaviridae | Human | Fecal-oral (probable) | |
| Cowpox virus | Orthopoxvirus, Poxviridae | Human, mammals | Zoonosis, contact | None |
| Coxsackievirus | Enterovirus, Picornaviridae | Human | Fecal-oral | Meningitis, myocarditis, paralysis |
| Crimean-Congo hemorrhagic fever virus | Nairovirus, Bunyaviridae | Human, vertebrates, ticks | Zoonosis, arthropod bite | Hemorrhagic fever |
| Dengue virus | Flavivirus, Flaviviridae | Human, mosquitoes | Zoonosis, arthropod bite | Hemorrhagic fever |
| Dhori virus | Thogotovirus, Orthomyxoviridae | Human, ticks | Zoonosis, arthropod bite | Fever, encephalitis |
| Dugbe virus | Nairovirus, Bunyaviridae | Human, ticks | Zoonosis, arthropod bite | Thrombocytopaenia |
| Duvenhage virus | Lyssavirus, Rhabdoviridae | Human, mammals | Zoonosis, animal bite | Fatal encephalitis |
| Eastern chimpanzee simian foamy virus | Simiispumavirus, Retroviridae | Human | Contact, saliva | Nine |
| Eastern equine encephalitis virus | Alphavirus, Togaviridae | Human, birds, mosquitoes | Zoonosis, arthropod bite | Encephalitis |
| Ebolavirus | Ebolavirus, Filoviridae | Human, monkeys, bats | Zoonosis, contact | Hemorrhagic fever |
| Echovirus | Enterovirus, Picornaviridae | Human | Fecal-oral | Common cold |
| Encephalomyocarditis virus | Cardiovirus, Picomaviridae | Human, mouse, rat, pig | Zoonosis | Encephalitis |
| Epstein-Barr virus | Lymphocryptovirus, Herpesviridae | Human | Contact, saliva | Mononucleosis |
| European bat lyssavirus | Lyssavirus, Rhabdovirus | Human, bats | Zoonosis, animal bite | Fatal encephalitis |
| GB virus C/Hepatitis G virus | Pegivirus, Flaviviridae | Human | Blood, occasionally sexual | None |
| Hantaan virus | Hantavirus, Bunyaviridae | Human, rodents | Zoonosis, urine, saliva | Renal or respiratory syndrome |
| Hendra virus | Henipavirus, paramyxoviridae | Human, horse, bats | Zoonosis, animal bite | Encephalitis |
| Hepatitis A virus | Hepatovirus, picornaviridae | Human | Fecal-oral | Hepatitis |
| Hepatitis B virus | Orthohepadnavirus, Hepadnaviridae | Human, Chimpanzees | Sexual contact, blood | Hepatitis |
| Hepatitis C virus | Hepacivirus, Flaviviridae | Human | Sexual, blood | Hepatitis |
| Hepatitis E virus | Hepevirus, Unassigned | Human, pig, monkeys, some rodents, chicken | Zoonosis, food | Hepatitis |
| Hepatitis delta virus | Deltavirus, Unassigned | Human | Sexual contact, blood | Hepatitis |
| Horsepox virus | Orthopoxvirus, Poxviridae | Human, horses | Zoonosis, contact | None |
| Human adenovirus | Mastadenovirus, Adenoviridae | Human | Respiratory, fecal-oral | Respiratory |
| Human astrovirus | Mamastrovirus, Astroviridae | Human | Fecal-oral | Gastroenteritis |
| Human coronavirus | Alphacoronavirus, Coronaviridae | Human | Respiratory | Respiratory |
| Human cytomegalovirus | Cytomegalovirus, Herpesviridae | Human | Contact, urine, saliva | Mononucleosis, pneumonia |
| Human enterovirus 68, 70 | Enterovirus, Picomaviridae | Human | Fecal-oral | Diarrhea, neurological disorder |
| Human herpesvirus 1 | Simplexvirus, Herpesviridae | Human | Sexual contact, saliva | Skin lesions |
| Human herpesvirus 2 | Simplexvirus, Herpesviridae | Human | Sexual contact, saliva | Skin lesions |
| Human herpesvirus 6 | Roseolovirus, Herpesviridae | Human | Respiratory, contact | Skin lesions |
| Human herpesvirus 7 | Roseolovirus, Herpesviridae | Human | Respiratory, contact | Skin lesions |
| Human herpesvirus 8 | Rhadinovirus, Herpesviridae | Human | Sexual contact, saliva | Skin lymphoma |
| Human immunodeficiency virus | Lentivirus, Retroviridae | Human | Sexual contact, blood | AIDS |
| Human metapneumoniavirus (HMPV) | Orthopneumovirus, Pneumoviridae | Human | Respiratory | Respiratory |
| Human papillomavirus 1 | Mupapillomavirus, Papillomaviridae | Human | Contact | Skin warts |
| Human papillomavirus 2 | Alphapapillomavirus, Papillomaviridae | Human | Contact | Skin warts |
| Human papillomavirus 16,18 | Alphapapillomavirus, Papillomaviridae | Human | Sexual | Genital warts, cervical cancer |
| Human parainfluenza | Respirovirus, Paramyxoviridae | Human | Respiratory | Respiratory |
| Human parvovirus B19 | Erythrovirus, Parvoviridae | Human | Respiratory | Skin lesion |
| Human respiratory syncytial virus | Orthopneumovirus, Pneumoviridae | Human | Respiratory | Respiratory |
| Human rhinovirus | Enterovirus, Picornaviridae | Human | Respiratory | Respiratory |
| Human SARS coronavirus | Betacoronavirus, Coronaviridae | Human, bats, palm civet | Zoonosis | Respiratory |
| Human T-lymphotropic virus | Deltaretrovirus, Retroviridae | Human | Sexual contact, maternal-neonatal | Leukemia |
| Human torovirus | Torovirus, Coronaviridae | Human | Fecal-oral | Gastroenteritis |
| Influenza A virus | Influenzavirus A, Orthomyxoviridae | Human, birds, pigs | Respiratory or Zoonosis, animal contact | Flu |
| Influenza B virus | Influenzavirus B, Orthomyxoviridae | Human | Respiratory | Flu |
| Influenza C virus | Influenzavirus C, Orthomyxoviridae | Human | Respiratory | Flu |
| Isfahan virus | Vesiculovirus, Rhabdoviridae | Human, sandflies, gerbils | Zoonosis, arthropod bite | Undocumented, encephalitis? |
| JC polyomavirus | Polyomavirus, Polyomaviridae | Human | Fecal-oral or urine | Encephalitis |
| Japanese encephalitis virus | Flavivirus, Flaviviridae | Human, horses, birds, mosquitoes | Zoonosis, arthropod borne | Encephalitis |
| Junin arenavirus | Arenavirus, Arenaviridae | Human, rodents | Zoonosis, fomite | Hemorrhagic fever |
| KI Polyomavirus | Polyomavirus, Polyomaviridae | Human | Fecal-oral or urine | Encephalitis |
| Lagos bat virus | Lyssavirus, Rhabdoviridae | Human, mammals | Zoonosis, animal bite | Fatal encephalitis |
| Lake Victoria marburgvirus | Marburgvirus, Filoviridae | Human, monkeys, bats | Zoonosis, fomite | Hemorrhagic fever |
| Langat virus | Flavivirus, Flaviviridae | Human, ticks | Zoonosis, arthropod borne | Encephalitis |
| Lassa virus | Arenavirus, Arenaviridae | Human, rats | Zoonosis, fomites | Hemorrhagic fever |
| Louping ill virus | Flavivirus, Flaviviridae | Human, mammals, ticks | Zoonosis, arthropod bite | Encephalitis |
| Lymphocytic choriomeningitis virus | Arenavirus, Arenaviridae | Human, rodents | Zoonosis, fomite | Encephalitis |
| Machupo virus | Arenavirus, Arenaviridae | Human, monkeys, mouse | Zoonosis, fomite | Encephalitis |
| Mammalian orthorubulavirus 5 (Simian virus 5) | Rubulavirus, Paramyxoviridae | Human, dog | Zoonosis, contact | Undocumented |
| Mayaro virus | Alphavirus, Togaviridae | Human, mosquitoes | Zoonosis, arthropod bite | Fever, joint pain |
| Measles virus | Morbilivirus, Paramyxoviridae | Human | Respiratory | Fever, rash |
| Merkel cell polyomavirus | Polyomavirus, Polyomaviridae | Human | - | Merkel cell carcinoma |
| MERS coronavirus | Betacoronavirus, Coronaviridae | Human, Tomb bat | Zoonosis | Respiratory |
| Mengo encephalomyocarditis virus | Cardiovirus, Picomaviridae | Human, mouse, rabbit | Zoonosis | Encephalitis |
| Mokola virus | Lyssavirus, Rhabdoviridae | Human, rodents, cat, dog shrew | Zoonosis, animal bite | Encephalitis |
| Molluscum contagiosum virus | Molluscipoxvirus, Poxviridae | Human | Contact | Skin lesions |
| Monkeypox virus | Orthopoxvirus, Poxviridae | Human, mouse, prairie dog | Zoonosis, contact | Skin lesions |
| Mumps virus | Rubulavirus, Paramyxoviridae | Human | Respiratory, saliva | Mumps |
| Murray valley encephalitis virus | Flavivirus, Flaviviridae | Human, mosquitoes | Zoonosis, arthropod bite | Encephalitis |
| New York virus | Hantavirus, Bunyavirus | Human, mouse | Zoonosis, urine, saliva | Hemorrhagic fever |
| Nipah virus | Henipavirus, Paramyxoviridae | Human, bats | Zoonosis, animal bite | Encephalitis |
| Norwalk virus | Norovirus, Caliciviridae | Human | Fecal-oral | Gastroenteritis |
| O'nyong-nyong virus | Alphavirus, Togaviridae | Human, mosquitoes | Zoonosis, arthropod bite | Fever, joint pain |
| Orf virus | Parapoxvirus, Poxviridae | Human, mammals | Zoonosis, contact | Skin lesions |
| Oropouche virus | Orthobunvavirus, Bunyaviridae | Human, wild animals(sloths) | Zoonosis, arthropod bite | Fever, joint pain |
| Pichinde virus | Arenavirus, Arenaviridae | Human, rat, guinea pig | Zoonosis, fomite | Hemorrhagic fever |
| Poliovirus | Enterovirus, Picornaviridae | Human, mammals | Fecal-oral | Poliomyelitis |
| Punta toro phlebovirus | Phlebovirus, Bunyaviridae | Human, sandflies | Zoonosis, arthropod bite | Hemorrhagic fever |
| Puumala virus | Hantavirus, Bunyavirus | Human, bank vole | Zoonosis, urine, saliva | Hemorrhagic fever |
| Rabies virus | Lyssavirus, Rhabdoviridae | Human, mammals | Zoonosis, animal bite | Fatal encephalitis |
| Rift valley fever virus | Phlebovirus, Bunyaviridae | Human, mammals, mosquitoes, sandflies | Zoonosis, arthropod bite | Hemorrhagic fever |
| Rosavirus A | Rosavirus, Picomaviridae | Human | | |
| Ross river virus | Alphavirus, Togaviridae | Human, mosquitoes, marsupials | Zoonosis, arthropod bite | Fever, joint pain |
| Rotavirus A | Rotavirus, Reoviridae | Human | Fecal-oral | Gastroenteritis |
| Rotavirus B | Rotavirus, Reoviridae | Human | Fecal-oral | Gastroenteritis |
| Rotavirus C | Rotavirus, Reoviridae | Human | Fecal-oral | Gastroenteritis |
| Rubella virus | Rubivirus, Togaviridae | Human | Respiratory | Rubella |
| Sagiyama virus | Alphavirus, Togaviridae | Human, horse, pig, mosquitoes | Zoonosis, arthropod bite | Fever, joint pain |
| Salivirus A | Salivirus, Picomaviridae | Human | | Gastroenteritis |
| Sandfly fever Naples phlebovirus (Toscxana virus) | Phlebovirus, Bunyaviridae | Human, sandflies | Zoonosis, arthropod bite | Hemorrhagic fever |
| Sapporo virus | Sapovirus, Caliciviridae | Human | Fecal-oral | Gastroenteritis |
| SARS coronavirus 2 | Betacoronavirus, Coronaviridae | Human, bats, pangolin? | Respiratory | Covid-19 |
| Semliki forest virus | Alphavirus, Togaviridae | Human, birds, hedgehog, mosquitoes | Zoonosis, arthropod bite | Fever, joint pain |
| Seoul virus | Hantavirus, Bunyavirus | Human, rats | Zoonosis, urine, saliva | Hemorrhagic fever |
| Simian foamy virus | Spumavirus, Retroviridae | Human, monkeys | Zoonosis, contact | None |
| Sindbis virus | Alphavirus, Togaviridae | Human, birds, mosquitoes | Zoonosis, arthropod bite | Pogosta_disease Fever, joint pain |
| Southampton virus | Norovirus, Caliciviridae | Human | Fecal-oral | Gastroenteritis |
| St. louis encephalitis virus | Flavivirus, Flaviviridae | Human, birds, mosquitoes | Zoonosis, arthropod bite | Encephalitis |
| Tick-borne powassan virus | Flavivirus, Flaviviridae | Human, ticks | Zoonosis, arthropod bite | Encephalitis |
| Torque teno virus | Alphatorquevirus, Anelloviridae | Human | Sexual, blood | None |
| Uukuniemi virus | Phlebovirus, Bunyaviridae | Human, ticks | Zoonosis, arthropod bite | Hemorrhagic fever |
| Vaccinia virus | Orthopoxvirus, Poxviridae | Human, mammals | Contact | None |
| Varicella-zoster virus (VZV) | Varicellovirus, Herpesviridae | Human | Respiratory, contact | Varicella |
| Variola virus | Orthopoxvirus, Poxviridae | Human | Respiratory | Variola |
| Venezuelan equine encephalitis virus | Alphavirus, Togaviridae | Human, rodents, mosquitoes | Zoonosis, arthropod bite | Fever, joint pain |
| Vesicular stomatitis virus | Vesiculovirus, Rhabdoviridae | Human, cattle, horse, pig, flies | Zoonosis, athropod bite | Encephalitis |
| Vientoviarus? | Torbevirus, Redondoviridae | Human? | Unknown | Unknown |
| West Nile virus | Flavivirus, Flaviviridae | Human, birds, ticks, mosquitoes | Zoonosis, arthropod bite | Encephalitis |
| Western equine encephalitis virus | Alphavirus, Togaviridae | Human, vertebrates, mosquitoes | Zoonosis, arthropod bite | Fever, joint pain |
| WU polyomavirus | Polyomavirus, Polyomaviridae | Human | Respiratory fluids or urine | None |
| Yaba monkey tumor virus | Orthopoxvirus, Poxviridae | Human, monkeys | Zoonosis, contact | None |
| Yaba-like disease virus | Orthopoxvirus, Poxviridae | Human, monkeys | Zoonosis, contact | None |
| Yellow fever virus | Flavivirus, Flaviviridae | Human, monkeys, mosquitoes | Zoonosis, arthropod bite | Hemorrhagic fever |
| Zika virus | Flavivirus, Flaviviridae | Human, monkeys, mosquitoes | Zoonosis, arthropod bite | Fever, joint pain, rash |

Cytotoxic T cells (T_{c}) and T helper cells (T_{H}) are the key effectors of the cell-mediated adaptive response to viruses. The response begins with the interaction of a T cell receptor with an MHC class II peptide complex on the surface of an antigen-presenting cell (APC). This interaction, together with binding of coreceptors (such as B7 on the T_{H} cell and CD28 on the APC) activates a signal transduction pathway leading to new gene expression in the T_{H} cell. One common result is the secretion of IL2, which binds to the IL-2 receptor on the T_{H} cell in an autocrine loop, stimulating T_{H} cell proliferation. The T_{H} cells recognize antigens bound to MHC class II molecules and produce powerful cytokines that "help" other lymphocytes (both B cells and T cells) by promoting or inhibiting cell division and gene activity. IL-2 also stimulates cytotoxic T cells to differentiate into CTLs that can kill virus-infected cells. When the T cell receptor on CTLs engage an MHC class I-viral peptide complex on the surface of a self cell, the CTL actively injects lethal enzymes that kill the virus-infected cell. [Principles of Virology. Flint, SJ, Enquist LWQ, Krug, RM, Racaniello, VR, Skalka, AM, Eds. (2000) ASM Press, Washington, DC, Chapter 14, pp. 479-516]

According to some embodiments, MNCs can be transduced with one or more viruses to generate multivirus-specific (MV) CTLs. According to some embodiments, selected T cells can be transduced with one or more viruses to generate multivirus-specific T cells (MV-VSTs). According to some embodiments, the virus is one or more selected from Table 2. For example, Papadopouloou et al. demonstrated the feasibility and clinical utility of single-culture VSTs that recognize 12 immunogenic antigens from 5 viruses (Epstein-Barr virus, adenovirus, cytomegalovirus, BK virus, and Human Herpesvirus 6) that frequently cause disease in immunocompromised patients. [Papadopoulou, A. et al. Sci. Transl. Med. (2014) 6 (242): 242ra83].

The following examples illustrate transduction of cytotoxic lymphocytes derived from peripheral blood (PB) and cord blood (CB) with CMV, EBV and AdV to generate CMV, EBV, and AdV-specific VSTs.

***Generation of multivirus-specific CTLs (MV-CTLs)**.* According to some embodiments, multivirus-specific cytotoxic T lymphocytes (MV-CTLs) can be generated from PB as described by Micklethwaite, et al. [Blood (2010) 115 (13): 2695-2703]. For example, a clinical-grade recombinant adenovirus type 5 vector pseudotyped with adenovirus type 35 fibers and encoding CMV-pp65 (Ad5f35CMVpp65) has been used as a source of CMV and AdV antigens [Leen, AM et al. Nat. Med. (2006) 12 (10): 1160-66; Yotnda, P. et al. Gene Ther. (2001) 8: 930-7)]. After overnight activation, mononuclear cells are harvested, pelleted, and transduced with the Ad5f35CMVpp65 vector at a multiplicity of infection (MOI) of 10 international units (IU) per cell (first stimulation).

### Generation of multi-virus specific cytotoxic T lymphocytes (MV-CTLs) from PB

According to some embodiments, MV-CTLs are generated as described by Leen AM et al. [Nat. Med. (2006) 12 (10): 1160-66]. Briefly, the CTLs present at day 9 after the first stimulation (using monocytes infected with the Ad5f35CMVpp65 vector) are restimulated weekly with irradiated EBV-LCLs (40 Gy) transduced with the same vector at an MOI of 100 IU/cell at an effector-stimulator ratio of 4:1. After 2 stimulations, recombinant human IL-2 (Chiron) is added at 100 U/mL.

### Generation of MV CTLs from CB

According to some embodiments, CB-derived MV-CTLs are generated using the same medium as for PB-derived MV-CTLs, except 10% human serum is used instead of FCS. [Hanley, PJ et al. Blood (2009) 114 (9): 1958-67]. Briefly, previously cryopreserved, nonadherent CBMCs are primed with DCs transduced with the Ad5f35CMVpp65 vector at a 20 to 1 ratio in the presence of recombinant human IL-7 (10 ng/mL), IL-12 (10 ng/mL), and IL-15 (5 ng/mL). Cultures are restimulated on day 10 with irradiated autologous LCLs transduced with Ad5f35CMVpp65 at a responder-to-stimulator ratio of 4:1 and with IL-15 (5 ng/mL), and restimulated 1 week later with transduced LCLs at a responder-to-stimulator ratio of 4:1. IL-2 (50-100 U/mL) is added 3 days after the second stimulation and then twice weekly.

### Generation and transduction to express Ad5f35CMVpp65 of DCs from CB

According to some embodiments, CB-derived DCs can be generated as described by Hanley, PJ et al. Blood (2009) 114 (9): 1958-67]. CB-derived mononuclear cells (CBMCs) are thawed and washed twice and resuspended in CellGenix media (CellGenix USA) and plated in DC media (CellGenix media plus 2 mM l-glutamine; GlutaMAX; Invitrogen) for 2 hours at 37°C in a humidified CO2 incubator. Nonadherent cells are removed by gentle washing, then cryopreserved for later stimulation with mature DCs. Loosely adherent cells are cultured in DC media with 800 U/mL granulocyte-macrophage colony-stimulating factor (GM-CSF; sargramostim, Leukine; Immunex) and 500 U/mL interleukin-4 (IL-4; R&D Systems) for 7 days. IL-4 and GM-CSF were again added on day 3. On day 5, the CB-derived DCs are transduced with the clinical-grade Ad5f35CMVpp65 vector at an MOI of 10 IU per cell for 2 hours and matured in a cytokine cocktail of GM-CSF, IL-4, IL-1β, tumor necrosis factor-α, IL-6, (R&D Systems) and prostaglandin E2 (PGE2; Sigma-Aldrich) for 2 days. On day 7, the DCs are harvested and irradiated (30 Gy) and then used to stimulate virus-specific CTLs.

***Generation of EBV transformed LCLs.*** According to some embodiments, PBMCs obtained by density gradient centrifugation are used first to generate EBV-transformed lymphoblastoid B-cell lines (LCLs) for use as antigen presenting cells. Thawed mononuclear cells (5 × 10⁶) derived from PB or CB are infected with the B95-8 strain of EBV and cultured in the presence of cyclosporine A and acyclovir for 2 weeks as described in Smith, CA et al. [J. Hematother. (1995) 4 (2): 73-79]. The resultant LCLs can be maintained in culture and used as antigen-presenting cells for CTL stimulation.

According to some embodiments, the MV-CTLs are activated without preparation of T cells with anti-CD3 and anti-CD28 reagents. According to some embodiments, specific T cell subsets are isolated and activated.

For example, according to some embodiments, CD3+ T cells can be isolated from the MV-CTLs by negative selection, e.g., using Miltenyi Pan T-cell Isolation Kit, human (130-096-535). Non-target cells, i.e., monocytes, neutrophils, eosinophils, B cells, stem cells, dendritic cells, NK cells, granulocytes, erythroid cells are labeled using a cocktail of biotin-conjugated antibodies. The cocktail contains antibodies against CD14, CD15, CD16, CD19, CD34, CD36, CD56, CD123, and CD235a (Glycophorin A). Subsequently, non-target cells are magnetically labelled with the Pan T Cell MicroBead Cocktail. The non-T cells are retained in a MAXS Column placed in a MACS Separator, while the unlabeled T cells pass through the column and are collected as the enriched, unlabeled T cell fraction. According to some embodiments, the MV-VSTs then are activated and expanded in vitro.

According to some embodiments, the MV-CTLs and MV-VSTs are capable of responding to in vivo or ex vivo challenge with the virus(es).

According to some embodiments, the MV- VSTs are washed and cryopreserved. According to some embodiments, the MV-VST cells are at least partially HLA typed for HLA class I alleles (A, B and C), class II alleles (DR, DP, and DQ), or for class I alleles plus class II alleles to establish HLA stocks of the MV-VST cells banked for off-the-shelf use.

According to some embodiments, when needed, the cryopreserved MV-VSTs are thawed and activated in vitro. According to some embodiments, the MV-VSTs are activated by adding human T cell TransAct^{™} (Miltenyi). According to some embodiments the MV-VSTs are activated by anti-CD3 monoclonal antibody (OKT3; Takara) stimulation. According to some embodiments, the MV-VSTs are activated by Dynabeads Human T-Activator CD3/CD28 (ThermoFisher) stimulation.

According to some embodiments, the activated MV- VSTs are expanded.

**b) genetically modifying the population of allogeneic VSTs to stably express up to three chimeric antigen-receptors (CARs), wherein each CAR is specific for a cancer antigen,**
**wherein to reduce risk of host versus graft rejection (HVGR), one or more of the CARs comprise:**
**(i) a-CD30 CAR expressing a CD30-specific scFv fragment, or**
**(ii) a chimeric alloimmune defense receptor (ADR), or**
**(iii) a chimeric HLA accessory receptor fused with a cytolytic endodomain of T cell receptor zeta chain, or**
**(iv) a-CD30 CAR expressing a CD30-specific scFv fragment, and a chimeric HLA accessory receptor fused with a cytolytic endodomain of T cell receptor zeta chain; or**
**(v) a-CD30 CAR expressing a CD30-specific scFv fragment, and a chimeric alloimmune defense receptor (ADR);**
**(vi) a chimeric alloimmune defense receptor (ADR), and a chimeric HLA accessory receptor fused with a cytolytic endodomain of T cell receptor zeta chain,**
**(vii) or a combination thereof.**

TCRs expressed on the surface of T lymphocytes can only recognize the peptide antigens presented to them through MHCs by antigen-presenting cells (APCs). On the other hand, monoclonal antibodies can recognize and bind cell surface-expressed antigens that are not presented by MHCs. This ability has been utilized to redirect the cytotoxicity of various kinds of immune cells toward tumor surface-expressed antigens of interest which can be either tumor specific antigens (TSAs) or tumor associated antigens (TAAs).

According to some embodiments, the VSTs of the present disclosure will be further modified to function as tumor-directed effector cells through expression of one, two or three chimeric antigen receptors (CARs).

Clinical evidence supports this approach. For example, 3 months to 13 years after allogeneic hematopoietic stem cell transplantation, 8 patients were treated with allogeneic donor-derived EBV specific VSTs engineered to express CD19CAR. There were no infusion-related toxicities; the VSTs persisted for a median of 8 weeks in blood and up to 9 weeks at disease sites. The VSTs retained the characteristics of nonmanipulated allogeneic VSTs while gaining antitumor activity without infusion-related toxicities including GvHD. [Cruz. CRY et al, Blood (2013) 122 (17): 2965-73]. In another study [Lapteva, N. et al. Clin. Cancer Res. (2019) 25 (24): 7340-50], 8 HSCT recipients in remission from high-risk CD19+ B cell ALL received donor T cells specific for CMV, EBV and AdV (VSTs) modified with a second generation CD19-CAR. The CD19-CAR-VSTS could be detected by PCR for a median of 182 weeks (range 8 weeks to 5 years), but only in the presence of viral reactivation was there a substantive expansion of CD19.CAR-VSTs and associated B-cell aplasia, despite the presence of significant number of normal B cells in all patients at the time of infusion. Signaling through the CAR was reinforced by concomitant or sequential signaling through the native TCR.

It has been reported that CTLs with native receptor specificity directed to persistent human viruses such as EBV can survive long term after infusion and eradicate even bulky EBV-associated malignancies, such as Hodgkin's disease and nasopharyngeal cancer. Without being limited by theory, a contributing factor to the superior survival and function of the EBV-specific CTLs is that engagement of their native receptors by EBV-infected B cells produces extensive co-stimulation during their preparation ex-vivo and by encounters with (latent) viral antigens on antigen-presenting cells in vivo. [Pule, MA et al. Nature Medicine (2008) dol:10.1038/nm.1882; see also Tanaka, M. et al. Clin. Cancer Res. (2017) 23 (14): 3499-509].

According to some embodiments, the MV-VSTs of step (a) are transduced on RetroNectin (Takara Bio)-coated plates by a retroviral vector comprising a nucleic acid encoding a synthetic chimeric antigen receptor (CAR) that specifically binds a cancer antigen to stably express the cancer antigen-specific CAR. According to some embodiments, the CAR comprises an extracellular antigen recognition domain, a spacer/hinge region and transmembrane domain; and an intracellular signal transduction domain. According to some embodiments, for each antigen, the extracellular antigen recognition domain comprises a single-chain variable fragment (scFv) derived from a monoclonal antibody specific for that antigen. According to some embodiments, each CAR is specific for a tumor associated antigen or a tumor specific antigen. According to some embodiments, the intracellular signal transduction domain comprises a CD3ζ T cell activation chain and may comprise one or more costimulatory molecules, e.g., CD28. According to some embodiments, the CAR can be targeted to the CCR5, AAVSI or T cell receptor α constant (TRAC) loci of the multivirus-specific VSTs. [See, Eyquem, J. et al. Nature (2017) 543 (7643): 113-7, citing Lombardo, A. et al. Nat. Methods (2011) 8: 861-9; Sather, BD et al. Sci. Transl. Med. (2015) 7: 307 ra156].

According to some embodiments, this process is repeated so that the MV-VST populations express up to three synthetic CAR receptors that can specifically target the VSTs to one, two or three selected cancer antigens. According to some embodiments, this can be accomplished by preparing mixtures of MV-VST populations, each of which expresses a distinct antigen-specific CAR. According to some embodiments, two CARs can target specific antigen molecules in a single engineered VST cell. According to some embodiments, three CARs can target specific antigen molecules in a single engineered MV-VST cell. According to some embodiments, delivery of multiple VST-CAR-T cell populations each expressing a different synthetic CAR is accomplished by delivering a mixture of the VST-CAR populations, so that one, two or three CARs are expressed.

According to some embodiments the MV-VST-CAR-T cells are genetically marked with a retroviral vector encoding a marker gene for measuring persistence of the functional MV-VST-CAR-T cells. [See, e.g., Heslop, HE et al. Blood (2010) 115: 925-935].

According to some embodiments, Boolean logic can be applied to "gate" the activity of the CAR-T cells. For example, MV-VST cells with two or more different specific CAR-T molecules can use an "OR" logic gate, which enables the CAR-T cells to have an anti-tumor effect in the presence of either targeted antigen. For example, MV-VST cells with two or more different specific CAR-T molecules can use an "AND" logic gate, which enables the CAR-T cells to have an anti-tumor effect in the presence of the two or more antigens simultaneously, either targeted antigen. For example, MV-VST cells with two or more different specific CAR-T molecules can use an "OR" logic gate, which enables the CAR-T cells to distinguish target cells from nontarget cells.

According to some embodiments, to reduce risk of host versus graft rejection (HVGR), one or more of the CARs comprise:
(i) a-CD30 CAR expressing a CD30-specific scFv fragment; or
(ii) a chimeric alloimmune defense receptor (ADR) [e.g., against 4-1BB (Feiyan, M. Nat. Biotechnol. (2021) 39: 56-63), or
(iii) a chimeric HLA accessory receptor (e.g., beta2-microglobulin fused with a cytolytic endodomain of T cell receptor zeta chain [Quach, D. et al. J. Trans. Med. (2019) 17: 24], or
(iv) a-CD30 CAR expressing a CD30-specific scFv fragment, and a chimeric HLA accessory receptor fused with a cytolytic endodomain of T cell receptor zeta chain; or
(v) a-CD30 CAR expressing a CD30-specific scFv fragment, and a chimeric alloimmune defense receptor (ADR);
(vi) a chimeric alloimmune defense receptor (ADR), and a chimeric HLA accessory receptor fused with a cytolytic endodomain of T cell receptor zeta chain,
(vii) or a combination thereof.

CD30 is a surface marker highly and consistently expressed on malignant Hodgkin Reed-Sternberg neoplastic cells of Hodgkin's lymphoma that shows sequence homology to members of the TNF receptor superfamily. The CD30 antigen is not detected on peripheral blood cells or on resting lymphocytes, but it is present on a subpopulation of physiologically activated T cells and on thymic medulla. [Savoldo, B. et al. Blood (2007) 110 (7): 2620-30, citing Croft, M. Nat. Rev. Immunol. (2003) 3: 609-20]. Hypothetically, expansion of an anti-CD30-specific CTL population could therefore be self-defeating with autodestruction of the CD30 CTL population as the CTLs became activated or the destruction of T cells responding to other infectious antigens. Furthermore, treatment failure occurs following adoptive transfer of Epstein Barr virus (EBV)-specific CTLs (EBV-CTLs) if a subpopulation of malignant cells in the tumor lacks or loses expression of EBV antigens. These concerns proved unfounded. Instead of using an alpha-beta TCR as the basis for the synthetic receptor, Savoldo et al. made a CD30 CAR expressing a CD30-specific scFv fragment cloned in frame with the sequence encoding the human IgG1 CH2-CH3 domains and the transmembrane and cytoplastmic domain of the TCR receptor zeta chain to minimize issues of competition for receptor formation [Id., citing Schumacher, TN. Nat. Rev. Immunol. (2002) 2: 512-9] and to allow binding even to malignant target cells in which the antigen processing machinery had become impaired. [Id., citing Sadelain, M. et al. Nat. Rev. Cancer (2003) 3: 35-45] and were able to express it in EBV-CTLs. The CD30CAR+ EBV-CTLs did not lose their own target antigen specificities conferred by their native TCR while expressing the CD30 CAR and the CD30 CAR did not impede reactivation of CMV- and adenovirus-specific CTLs. The CD30CAR expressing EBV-CTLs killed both autologous EBV+ cells through their native TCR and EBV-/CD30+ targets through their MHC-unrestricted CAR. Moreover, in a xenograft model, the CD30CAR+ EBV-CTLs could be costimulated by EBV-infected cells and produce antitumor effects even against EBV-/CD30+ tumors.

According to some embodiments, the MV-VST-CAR-T cell populations have specificity for virus determinants and for the CAR-specific cancer antigen(s). For example, the MV-VST-CAR-T cell population expressing a CD30CAR can target virus determinants and CD30. For example, the MV-VST-CAR-T cell population expressing a CD30CAR plus one additional CAR specific for a cancer antigen can target virus determinants, CD30, and the cancer antigen targeted by the additional CARs. For example, the MV-VST-CAR-T cell population expressing a CD30CAR plus two additional CARs, each specific for a cancer antigen can target virus determinants, CD30, and the two cancer antigens targeted by the additional CARs. For example, the MV-VST-CAR-T cell population expressing a CD30 CAR and/or a chimeric alloimmune defense receptor can target virus determinants, CD30, two cancer antigens and reduce the risk of HvGD. Similarly, according to some embodiments, the MV-VST-CAR-T cell populations expressing a chimeric alloimmune defense receptor (e.g., against 4-1BB) and/or a chimeric HLA accessory receptor (e.g., Beta2 microglobulin fused with a cytolytic endodomain of the T cell receptor zeta chain; along with one or two additional CARs, each specific for a cancer antigen,can target virus determinants, cancer antigens and reduce the risk of host versus graft rejection (HVGR),

### Cancer Antigens:

Table 6 provides exemplary known tumor associated antigens.

**Table 6. Exemplary known tumor-associated Antigens**

| **Known Tumor Associated Antigens** | |
|---|---|
| AFP (alpha-fetoprotein) | AKAP-4 |
| α6β4 integrin | αvβ3 integrin |
| ALK | alpha-fetoprotein (alpha-fp) |
| Androgen Receptor | B7H3 |
| B7H4 | BAGE |
| BORIS | B-Actin/4/m |
| beta-oncofetal antigen (BOFA) | BCR-ABL1 |
| Beta-catenin | β-Catenin-m |
| Carbonic anhydrase IX | CASP-8/m |
| CEA | CD19 |
| CD30 | CD 147 basigin or EMMPRIN |
| CDK4 | CDK-4/m |
| CDKN2a | CDC27 |
| CDCP 1 | Cyclin A |
| Cyclin B1 | CYP1B1 |
| DDX-43 DEAD-Box Helicase 43 | EGFR |
| EGFR Viii | ELF2- |
| EpCAM | EphA2 |
| Erb-B2-interacting protein (ERBB2IP) | ERG (TMPRSS2ETS fusion gene) |
| ETV6-AML1 (also known as RUNX1) fusion protein | FAP |
| Fibronectin/m | FOLR1 folate receptor alpha (FOLR1), a folate transporter |
| Fos-related antigen 1 | Fucosyl GM1 |
| GAGE | GD2 |
| GDF3 | GloboH |
| GM3 | gp100 |
| GPC3 (glypican-3) | HER-2/neu |
| HER2 - human epidermal growth factor receptor 2 | HLA-A2-R17OJ |
| HGFR - hepatocyte growth factor receptor | HPV E6 E7 |
| HMWMAA | hTERT |
| HSP70-2/m | KMHN1 (also known as CCDC110 (CTA) |
| Kita-kyushu lung cancer antigen 1 (KKLC1) | LCK |
| KRAS | Legumain |
| LDLR/FUT | MAD-CT-1 |
| LMP2 | MAGE-A1 |
| MAF-CT-2 | MAGE-A4 |
| MAGE-A3 | MAGE-A10 |
| MAGE-A6 | MAGE-A12 |
| MAGE-A11 | Type II MAGE (MAGE-D, E, F, G, H_L subfamilies and Necdin) |
| Type I MAGE including MAGE-A, -B, -C. | MART-2/m |
| MART-1 | Merkel cell carcinoma (MCC) |
| MELANA/MARTI | ML-IAP |
| Mesothelin | MRP-3 |
| MUC1 (mucin 1) \ | MUM-1/2 |
| MUM-1/2 | MYCIN |
| MUM-3 (Helicase) | NA17 |
| Myosin-m | NY-ESO-1, also known as LAGE2 and CTAG1A |
| NY-BR-1 | P53 nonmutant |
| P53 mutant | Page4 |
| PAP | PAX3 |
| PAX5 | PDGFR-beta |
| PLAC1 | Polysialic Acid |
| PRAME, (PReferentially expressed Antigen in MElanoma) | Prostate specific antigen (PSA) a |
| prostate specific membrane antigen (PSMA) | Proteinase 3 (PR1) |
| PSCA | PTP-LAR protein tyrosine phosphatase (PTP) LAR |
| Ras-mutant | Redox-perox/m |
| RhoC | RGSS |
| RT-PTP-K/m | SAGE |
| OY-TES1 | SART3 |
| SLe(a) | SPA17 (sperm autoantigenic protein 17) |
| Sperm protein 17 | Synovial sarcoma X breakpoint 1 (SSX1) |
| SSX-2 | STn |
| Survivin | TDL |
| Tie 2 | Tn |
| TPI | TP53 |
| TPBG, also known as WAIF1 | TRP-2 |
| TfR transferrin receptor | TRT - telomerase reverse transcriptase |
| Tyrosinase | VEGFR2 |
| Wilms tumor antigen (WT-1) | XAGE 1 |

Table 7 below provides the top 20 neoantigen producing genes in samples by disease type for the biotype protein-coding from the Cancer Genome Atlas, Genome Data Commons data portal in Exploration mode (visited April 14, 2023).

**Table 7. NEOANTIGEN PRODUCING GENES**

| **DISEASE TYPE** | **TOP 20 MOST FREQUENTLY MUTATED PROTEIN-CODING GENES** | | | | | | | | | | | | | | | | | | | |
|---|---|---|---|---|---|---|---|---|---|---|---|---|---|---|---|---|---|---|---|---|
| GLIOMA | IDH1 | TP53 | ATR X | TTN | PTEN | EGF R | MUC1 6 | CIC | PIK3C A | NFI | FLG | RYR2 | PCLO | PIK3 R1 | LRP2 | HMCN 1 | OBSC N | MUC1 7 | RB1 | PKHD 1 |
| SQUAMOUS CELL NEOPLASMS | TP53 | TTN | MUG 16 | CSM D3 | RYR2 | SYN E1 | LrplB | USH2 A | ZFHX4 | KMT2 D | DNA H5 | XIRP2 | PCLO | PIK3 CA | FAT1 | FAM1 35B | FLG | Nav3 | MUC1 7 | CDK N2A |
| DUCTAL AND LOBULAR NEOPLASMS | TP53 | PIK3 CA | TTN | KRA S | MUC1 6 | CDH 1 | GATA 3 | KMT2 C | RYR2 | SYNE 1 | MAP3 K1 | HMC N1 | FLG | RYR1 | OBSC N | USH2 A | ZFHX4 | SPTA 1 | RYR3 | SMA D4 |
| ADENOMAS AND ADENOCARCIN OMAS | TTN | TP53 | MUG 16 | CSM D3 | RYR2 | LRPI B | APC | SYNE 1 | KRAS | ZFHX 4 | OBSC N | USH2 A | ARID1 A | PTEN | FAT3 | FAT4 | FLG | CSMD 1 | PCLO | PIK3 CA |
| CYSTIC, MUCINOUS AND SEROUS NEOPLASMS | TP53 | TTN | MUG 16 | PIK3 CA | CSMD 3 | RYR 2 | USH2 A | SYNE 1 | DST | HNC N1 | RYR1 | FLG | OBSC N | LRP1 B | CSM D1 | MUC1 7 | KMT2 D | AHN AK | APC | MAC F1 |
| PLASMA CELL TUMORS | KRAS | BCL 7A | IGLL 5 | LTB | NRAS | TTN | TENT 5C | MUG 16 | BMP6 | DIS3 | TMSB 4X | TRAF 3 | CSMD 3 | FAT4 | RYR2 | PCDH 15 | FAT3 | PCLO | NRXN 1 | LRP1 B |
| LYMPHOID LEUKEMIAS | NOTC H1 | KRA S | NRA S | FBX W7 | TTN | PHF6 | PAX5 | FLT3 | PTEN | USP7 | DST | KMT2 D | DNM2 | CREB BP | BCL1 1B | FSIP2 | JAK2 | PTPN 11 | NSD2 | ETV6 |
| NEVI AND MELANOMAS | TTN | MU C16 | DNA H5 | PCL O | BRAF | LRP1 B | CSM D1 | ADG RV1 | CSMD 2 | ANK3 | MGA M | HYDI N | DSCA M | DNA H7 | XIRP 2 | PKHD 1L1 | DNAH 8 | FAT4 | RP1 | APOB |
| MYELOID LEUKEMIAS | NPM1 | DN MT3 A | FLT3 | NRA S | RUNX 1 | TP53 | IDH1 | TET2 | IDH2 | CEBP A | TTN | WT1 | MUC1 6 | PTPN 11 | SRSF 2 | ASXL 1 | GATA 2 | KRAS | STAG2 | DNA H9 |
| TRANSITIONA L CELL PAPILLOMAS AND CARCINOMAS | TTN | TP53 | MUG 16 | KMT 2D | KDM6 A | ARID 1A | SYNE 1 | PIK3 CA | HMCN 1 | RYR2 | MAC F1 | KMT2 C | RB1 | OBSC N | FAT4 | XIRP2 | FLG | DST | DNAH S | STAG 2 |
| NEUROEPITHE LIOMATOUS NEOPLASMS | MUC1 6 | ALK | TTN | FLG | ABCA 13 | FAT2 | FBN2 | AHN AK2 | MUC1 7 | DNA H17 | HMC N1 | K1AA 1109 | PTPNI 1 | INC | SPTA 1 | SYNE 2 | LRP1B | AHN AK | RYR1 | COL4 A1 |
| PARAGANGLIO MAS AND GLOMUS TUMORS | HRAS | NF1 | EPA S1 | TTN | MUC1 6 | RET | ABCA 13 | ATRX | CSDE1 | HUW E1 | KCN H5 | VHL | MUC5 B | AHN AK | RYR1 | CACN AID | ALMS 1 | PLCH 1 | ANAP C7 | PCDH B12 |
| GERM CELL NEOPLASMS | KIT | KRA S | SRC AP | TTN | NRAS | ZFH X4 | BIRC 6 | DMD | NEB | PCLO | MUC5 B | TET1 | ANKR D50 | MAC F1 | LAM A5 | VPS13 B | DNMT 1 | EIF4G 3 | TCOF1 | PIK3 CA |
| THYMIC EPITHELIAL NEOPLASMS | GTF2I | HRA S | TTN | MUC 16 | DNAH 17 | OSGI N1 | PCLO | TP53 | RNF21 3 | L3MB TL3 | ADG RV1 | CSMD 2 | CYLD | DNA H3 | MYO 15A | NRAS | CLIP2 | DMD | SACS | PLXN B2 |
| MYOMATOUS NEOPLASMS | TP53 | RB1 | ATR X | MUC 16 | TTN | LRP1 B | PCLO | PKHD 1L1 | RYR1 | LRP2 | DISP3 | USH2 A | UNC1 3C | CSM D1 | OBSC N | FCGB P | MUC1 7 | CENP E | ARFG EF1 | CUB N |
| COMPLEX MIXED AND STROMAL NEOPLASMS | TP53 | FBX W7 | PIK3 CA | PPP2 R1A | TTN | CHD 4 | PTEN | PIK3 R1 | KRAS | FLNA | ARID 1A | LRP1 | KMT2 D | MGA M | AHN AK | VPS13 B | LRP2 | CSMD 2 | DOP1 A | CSM D3 |
| OSSEOUS AND CHONDROMAT OUS NEOPLASMS | TP53 | MU C16 | TTN | ATR X | RB1 | DNA H3 | CSM D3 | DNA H9 | CNTN AP5 | DMD | PCLO | WASH 6P | DNAJ C6 | DNA H2 | HECT D4 | LAMA 2 | FCRL A | RYR2 | TMEM 63C | SYNE 1 |
| MESOTHELIAL NEOPLASMS | BAPI | NF2 | TP53 | TTN | SETD2 | LATS 2 | FAT4 | SDK1 | KMT2 A | PTCH 1 | ALPK 3 | SNRP 200 | OGDH L | PBR M1 | SETD B1 | NCOA 7 | RALG APA1 | GPRI N2 | FLG | UTP2 0 |
| SOFT TISSUE TUMORS AND SARCOMAS | TP53 | MU C16 | ATR X | TTN | RYR2 | LRP2 | PCDH 15 | OBSC N | MACF 1 | PPFIA 2 | FLG | FAT1 | USH2 A | CSM D3 | MYH 2 | MHY1 4 | PCLO | MUC5 B | DUOX 2 | AHN AK2 |
| LIPOMATOUS NEOPLASMS | MUC1 6 | TTN | TP53 | ATR X | B4GA LNT1 | ADG RV1 | FLG | XIRP 2 | PCLO | FLT4 | SYNE 1 | KIF26 A | KIAA1 217 | FAM4 7C | LRP1 | SLC9C 2 | TRDN | RP1 | SYNE2 | HTT |
| LEUKEMIAS, NOS. | WT1 | NRA S | RUN X1 | CRE BBP | XIRP2 | MUC 4 | SETD 2 | OBSC N | JAK3 | PTPN 11 | MAC F1 | AASD H | MRPL 39 | DYSF | ASXL 1 | DST | DRC7 | VPS13 C | SEL1L | ADG RL3 |
| MATURE B-CELL LYMPHOMAS | BTG2 | KMT 2D | PIMI | Hl-4 | IGLLS | B2M | FAT4 | LRP1 B | CARD 11 | BTG1 | MUC1 6 | PCLO | TSPO AP1 | NFKB II | ZFHX 4 | P2RY8 | KLHL6 | CSMD 1 | SOCS1 | Hl-2 |
| FIBROMATOUS NEOPLASMS | TP53 | MU C16 | ATR X | RB1 | PDE1C | USH2 A | ASTN 2 | MYH 7 | TTN | RIMS 2 | ABCB 5 | SPJKA P | F5 | DSCA M | MGA M | CSMD 1 | MAP3 K5 | TUBG CPS | PIK3C B | SCN4 A |
| EPITHELIAL NEOPLASMS, NOS. | TP53 | PIK3 CA | HMC N1 | TTN | OBSC N | VPS1 3B | CSM D3 | DMD | DNAH 10 | ZFHX 3 | CNTN AP4 | TG | MACF 1 | DGK B | FAM1 35B | PLEC | CACN A1G | IRS4 | LAMA 1 | XDH |
| ACINAR CELL NEOPLASMS | MUC1 6 | TTN | RYR 2 | CSM D3 | XIRP2 | KRA S | PCLO | TP53 | FAT3 | MUC 17 | SHAN K1 | USH2 A | FAT4 | UNCS D | PCDH 11X | OBSC N | CNTN AP2 | NRXN 3 | ZFHX4 | SPTA 1 |
| COMPLEX EPITHELIAL NEOPLASMS | TP53 | TIN | PIK3 CA | SYN E1 | USH2 A | KMT 2D | PCLO | PTEN | ZNF80 4A | TEN M1 | LAM B4 | DDX2 7 | CREB BP | MED 12 | VPS1 3B | SZT2 | CKAP 5 | DST | ADAM TS16 | NUT M1 |
| NOT REPORTED | TP53 | TN | MYH 7B | FMN 2 | NEB | MUC 16 | KANS L1 | SSTR 4 | UGT2 15 | KIF21 B | PPP2 R3A | SLCO 1C1 | ARFG EF1 | ZNF2 80B | PIK3 CA | ABCA 3 | PCDH A6 | MIS18 BP1 | SLCSI A | SYNP O2 |
| NEOPLASMS, NOS | TP53 | TIN | APC | MUC 16 | KRAS | RYR 3 | ADG B | FLG | ANKR D18A | USH2 A | CCDC 168 | OBSC N | DGKB | PRND | ICEI | LRP2 | PIK3C G | CACN A1E | C10orf 71 | BRAF |
| MYELODYSPL ASTIC SYNDROMES | P53 | SRS F2 | ZFP3 6L1 | SNT G1 | PUS7 | CTR9 | GPA3 3 | ANGP TL1 | HECT D3 | CUX1 | MED! 2 | GABR Q | PCOL CE2 | SORB S2 | AP2A 2 | TOPA Z1 | JAKMI P3 | RBP3 | DNAH 17 | SRCA P |
| SYNOVIAL. LIKE NEOPLASMS | TTN | ACS L4 | SPTB | IL27 | LRBA | SP14 0 | TME M74 | PLXN D1 | LAMA 1 | ABL1 | COL4 A2 | CATS PERI | RGS12 | LGAL S7B | NCO A4 | ADGR F1 | EIF4A 3 | MCL1 | BOLL | BEND 3 |
| NERVE SHEATH TUMORS | SDK1 | NF1 | SUZ1 2 | C7 | DCAF 5 | PGA P1 | C6 | SLC4 A4 | ANKR D33 | LARP 1 | COL6 A3 | PTPR U | KCNH 1 | KIT | RSFI | PCDH A4 | ITGAX | SYNG AP1 | TNRC 18 | SEC3 1B |
| CHRONIC MYELOPROLIF ERATIVE DISORDERS | PLXN A2 | TME M23 7 | SUZ1 2 | COL4 A2 | CBLL2 | EIF4 G3 | CACN A1F | PCDH A12 | GPRIN 2 | NPM1 | POM1 21L | FAM8 3F | CNGA 1 | LG12 | STK3 2B | DMXL 2 | DNAA F5 | GPR1 58 | EGFR | FOXP 2 |
| FIBROEPITHEL IAL NEOPLASMS | PKD1 L1 | DDX 10 | BSD C1 | SLIT 2 | HEAT RSB | ZFC3 H1 | QSER 1 | ZBTB 20 | PRPF1 9 | TUBG CP6 | DST | ITGA V | CCDC 120 | NUA K1 | TGFB 3 | NOS2 | FLG | DSC1 | NUTM 1 | LRRC 25 |
| ADNEXAL AND SKIN APPENDAGE NEOPLASMS | NUFIP 1 | NBA S | C21o rf91 | GRIP IN | MMS1 9 | CTD SP1 | EPB4 1 | ZNF2 96 | GABR Q | RBM4 8 | SCN9 A | MYBP C1 | DYSF | RGS2 2 | SEM A3A | POU6F 2 | SOD1 | HTRI D | MBP | OR52 N5 |
| MISCELLANEO US BONE TUMORS | ZC3H 12C | HCL S1 | ICEI | MBT PS2 | AKAP 3 | CAD PS2 | CRTC 1 | FGR | RFXA P | GPR1 56 | TRPM 1 | LGI2 | PRAM EF27 | LGII | NAD K | ARHG AP45 | ZFHX4 | DOCK 8 | CCHC R1 | LMO7 |

In addition, the top 20 neoantigen-producing genes in colorectal cancer: from the Cancer Genome Atlas (TCGA) cohort, obtained from the TSNAdb database are: TTN, MUC16, SYNE1, FAT4, NERB, CCDC168, RYR2, OBSCN, DNAH5, USH2A, DNAH11, APO8, SACS, CSMD1, DNAH17, ADGRV1, DNAH10, PCDH15; DNAH8 AND COL6A3. Bakrurraini, NAAR, et al. Vaccines (2020) 8: 371, citing Wu, J. et al. Genom. Proteom. Bioinform. (2018) 16: 276-82] According to some embodiments, mutated antigens studied in colorectal cancer include OGT; CDX2; U79260 (FTO); TGFβIIR; CASP5; CASP8; MSH03; MARCKS-1; MARCKS-2; CDX2-2; TAF1B-1; PCNXL2-2 TCFL2-2 BAXα+1; SMAD4;FMO5; U2SURP, MED25, FM05 [ Id., citing Newey, A. et al. J. Immunother. Cancer (2019) 7: 309]; ACVR2; TAF1B, ASTEI/HT001; TGFBR2; PTEN [Id., citing Tougeron et al. Mod. Pathol. (2009) 22: 1186-95]; ASTE1; HNF1A; TCF7L2 [Id., citing Maby, P. et al. Cancer Res. (2015) 75: 3446-55].

### c) expanding the population of cancer killing MV-VST-CAR-T cells in vitro to achieve a therapeutic dose of at least 40 × 10⁶ to 800 × 10⁶ cancer killing MV-VST-CAR-T cells;

According to some embodiments the population of MV-VST-CAR-T cells can be expanded in vitro. According to some embodiments, the activated MV-VSTs may be expanded in vitro in the presence of one or more cytokines, e.g., IL-2, IL-7, IL-15, Cieri, N. et al. [Blood (2013) 121 (4): 573-84] teach that IL-7 and IL-15 instruct the generation of human T_{SCM} from naive precursors; Xu et al. [Oncotarget (2016) 7 (50) 82354-68] compare the effects of different cytokines on CART cell phenotype and function; Xu et al [Blood (2014) 123 (24): 3750-9] teach that culturing cells with IL-7 and IL-15 produced greater antitumor activity of CAR-T cells mediated by increased resistance to cell death while preserving their migration to secondary lymphoid organs in a clinical trial.

According to some embodiments the activated and expanded population of MV-VST-CAR-T cells will be immunophenotyped by FACS using anti-human monoclonal antibodies and their biological activity will be measured in vitro.

According to some embodiments, the activated and expanded population of MV-VST-CAR-T cells will be cryofrozen for storage.

### d) Infusing eligible patients with the population of MV-VST-CAR-T cells; and

According to some embodiments, eligible patients will undergo infusion of a target dose of the activated and expanded population of VST-CAR-T cells following lymphodepletion. According to some embodiments, lymphodepletion would consist of conditioning with a combination of low-dose fludarabine and cyclophosphamideor a clinically equivalent regimen.

According to some embodiments, the single target dose ranges from about 40 to 800 × 10⁶ CAR-positive viable T cells. According to some embodiments eligible patients can be dosed with the VST-CAR-T cells in accordance with a treatment plan specifying the dosage, schedule and duration of treatment ("therapeutic regimen") that includes single and multiple dosing schedules.

According to some embodiments, to further decrease the risk of the VST-CAR-T cells being killed by the recipient's cells, recipients can be at least partially HLA matched against HLA class I alleles (A, B and C), class II alleles (DR, DP, and DQ), or class I alleles plus class II alleles of HLA stocks of the VST-CAR-T cells banked for off-the-shelf use.
**e) creating VST-CAR-T cells that express a nonexhausted PD-1^{neg}, TIGIT^{neg} TCF-1^{hi}TOX^{lo} early T stem cell (T_{SCM}) phenotype by:**
**(i) enriching for a population of CD4+ T cells, CD8+ T cells or a combination thereof that express a nonexhausted PD-1^{neg}, TIGIT^{neg} TCF-1^{hi}TOX^{lo} early T stem cell (T_{SCM}) phenotype;**
**(ii) generating by transduction of the population of CD4+ T cells, CD8+ T cells or a combination thereof with a viral vector encoding a protein of a virus an expanded population of allogeneic virus-specific T cells (VSTs) comprising T cell receptors (TCRs) specific for the protein of the virus;**
**(iii) genetically modifying the population of allogeneic VSTs in (ii) to stably express up to three chimeric antigen-receptors (CARs), wherein each CAR is specific for a cancer antigen, wherein to reduce risk of host versus graft rejection (HVGR), one or more of the CARs comprise:**
   **(1) a-CD30 CAR expressing a CD30-specific scFv fragment, or**
   **(2) a chimeric alloimmune defense receptor (ADR), or**
   **(3) a chimeric HLA accessory receptor fused with a cytolytic endodomain of T cell receptor zeta chain, or**
   **(4) a-CD30 CAR expressing a CD30-specific scFv fragment, and a chimeric HLA accessory receptor fused with a cytolytic endodomain of T cell receptor zeta chain; or**
   **(5) a-CD30 CAR expressing a CD30-specific scFv fragment, and a chimeric alloimmune defense receptor (ADR); or**
   **(6) a chimeric alloimmune defense receptor (ADR), and a chimeric HLA accessory receptor fused with a cytolytic endodomain of T cell receptor zeta chain, or**
   **(7) a combination thereof; and**
   **enriching for a population of non-exhausted PD-1^{neg}, TIGIT^{neg} TCF-1^{hi}TOX^{lo} CD4 T cells, CD8+ T cells or a combination thereof with early T stem cell(T_{SCM}) phenotype.**

***Background.*** In humans, substantial heterogeneity in TCF-1 expression by memory cells has been reported, with heightened TCF-1 expression characteristic of more quiescent cells. [Kratchmarov, R. et al. Blood (2018) 2 (14): 1685-90]. It is known that naive cells express the highest levels of TCF-1 protein, whereas T_{CM} express higher levels than T_{EM} and TEMRA. There are three distinct levels of TCF-1 expression: high (TCF-1^{hi}), intermediate (TCF-1^{int}) and low (TCF-1^{lo}). Expression of TCF-1 is reciprocal to the transcription factor T-bet, which also occupies three distinct levels. Cells with TCF-1^{hi}, TCF-1^{int} or TCF-1^{lo} expression are represented at varying frequencies within each of the naive, T_{CM}, T_{EM} and TEMRA compartments. TEMRA (CD45RA+CCR7-) CD8 T cells constitute a preformed effector population with an enhanced expression of effector molecules that can be efficiently activated using TCR stimulation alone or in combination with common-gamma chain cytokines. [Tilly, G. et al. Transplantation (2014) 98: 318-9].

TCF-1^{int} cells had the highest expression of Eomes, which regulates memory cell formation in mice [Kratchmarov, R. et al. Blood (2018) 2 (14): 1685-90, citing Pearce, EL et al. Science (2003) 302(5647): 1041-43]. Lymphoid-enhancer-binding factor 1 (LEF1), which also regulates memory cell formation [Id., citing Zhou, X and Xue, H-H. J. Immunol. (2012) 189 (6): 2722-6] and is part of the same family of Wnt pathway factors as is TCF-1, was expressed coordinately with TCF-1. T-bet expression, which is associated with effector differentiation, was expressed inversely to TCF-1.

***Surface marker phenotypes.*** TCF-1 expression was reciprocal to CD57, a marker of senesxcence and terminal differentiation [Id., citing Brenchley, JM et al. Blood (2003) 101 (7): 2711-20]. Both TCF-1^{hi} and TCF-1^{int} cells exhibited high CD127 expression, associated with long-lived memory populations [Id., citing Mazzucchelli, R. and Durum, SK. Nat. Rev. Immunol. (2007) 7 (2): 144-54]. TCF-1 hi cells show evidence of quiescence, including greater maintenance of the costimulatory receptor CD27 compared with TCF-1^{int} andTCF-1^{lo} cells.

***Cytokine production.*** PBMCs were stimulated with 12-0-tetradecanoylphorbol 13-acetate/ionomycin and analyzed for cytokine and cytolytic molecule expression and degranulation. TCF-1^{hi} cells self-renew following TCR stimulation. TCF-1^{int} cells had the largest capacity for immediate cytokine production, producing greater levels of IFN-gamma than TCF-1^{hi} or TCF-1^{lo} populations. TCF-1^{lo} cells expressed high levels of the cytotoxic granule component granzyme B, although they produced less IFN-gamma and underwent less efficient degranulation than TCF-1^{int} cells following stimulation. Consistent with a quiescent and less differentiated phenotype, TCF-1^{hi} cells had the least immediate effector function.

Naive and T_{CM} populations were sorted and stimulated as above. Following activation, T_{EM} cells are predominantly TCF-1^{int} before stimulation, whereas CCR7-CD57+ cells are mostly TCF1^{lo}, T_{EM} cells exhibit more proliferative capacity than CD57+ cells but lost TCF-1 expression after the first division and did not maintain a TCF-1^{hi} or TCF-1^{int} population. Similarly, following activation and proliferation, CD57+TCF-1^{lo} cells produced more TCF-1^{lo} descendants without evidence of de-differentiation [Id., citing Youngblood, B.e t al. Nature (2017) 552 (7685) 404-9; Akondy, RS. Et al. Nature (2017) 552 (7685): 362-7] into TCF-1^{hi} progeny.

***Stepwise differentiation of TCF-1^{hi} and TCF-1^{lo} populations following activation of naïve CD8+ T cells.*** Prior to division, naïve cells that had been activated for 4 days maintained high levels of CD27, CDR7, and CD127, as well as low levels of Eomes. All proliferating cells downregulate CDR7, but proliferating TCF-1^{lo} cells down regulated CD127 and CD27 to a greater extent than proliferating TCF-1^{hi} cells. All proliferating cells downregulated CCR7, but proliferating TCF-1^{lo} cells downregulated CD127 and CD27 to a greater extent than proliferating TCF-1^{hi} cells. Under these conditions, proliferating TDF-1^{hi} and TCF-1^{lo} cells had comparably high levels of T-bet and comparably low levels of Eomes expression. These results suggest that dividing TCF-1^{hi} cells are more activated than quiescent naive and memory populations and that they function as an intermediate, self-renewing progenitor of more differentiated TCF-1 cells.

### Relationsip of FOXO transcription factors to persistence.

In order to survive over long periods, memory CD8+ T cells persist at a population level by slow but constant self-renewal balanced against programmed cell death. [Utzschneider, DT, et al., Cell Reports (2018) 22: 3454-57]. Along with the unusual property of self-renewal, memory CD8+ T cells display the unique ability to serially transit through phases of activation, growth, and proliferation followed by quiescence. In essence, they exhibit characteristics of multipotent stem cells that simultaneously self-renew and produce progenitors of terminally differentiated cells [Id., citing Gattinoni, L. et al. Nat.Med. (2017) 23: 18-27; Fearon, DT et al., Science (2001) 293: 248-50].

The transcriptional network responsible for the generation of memory CD8+ T cells has been widely studied and found to include the evolutionarily conserved family of Forkhead box O (FOXO) transcription factors. The known cell-type-specific FOXO target genes profoundly affect survival, homing, proliferation, and differentiation of CD8+ T cells and constitute a large proportion of the memory gene expression signature. In particular, the transcription factor FOXO1 has been shown to positively regulate several genes associated with T cell survival and trafficking including Il7ra, Ccr7, Klf2, Sell (CD62L), Tcf7, Eomes, and Bcl2 [Id., citing Hedrick, SM et al., Nat. Rev. Immunol. (2012) 12: 649-61]. FOXO1 also has been shown to play an essential role in the generation of functional memory T cells by the direct or indirect repression of Tbx21 (T-BET), Ifng, and Gzmb (GRANZYME B), hallmarks of effector T cells [Id., citing Hess Michelini, R. et al., J. Exp. Med. (2013) 210: 1189-1200; Rao, RR et al. Immunity (2012) 36: 374-87; Ouyang, W. et al., Immunity (2009) 30: 358-71].

Using an inducible gene deletion system Utzschneider, et al. showed that FOXO1 must be continuously present for the homeostatic proliferation required to maintain a functional memory population. Upon FOX01 deletion after the establishment of memory, there occurred a rapid loss of gene expression characteristic of memory cells combined with a deficiency in homeostatic (lymphopenia-induced) proliferation leading to a continuous decline of the memory T cell population.

A number of studies have explored mechanisms to enable CAR T cells to adopt less-differentiated phenotypes, such as the use of IL-15, small molecule inhibitors such as AKT inhibitors [Chan, JD et al. [Nature (2024) 629: 201-10, citing Klebanoff, CA et al. JCI Insight (2017) 2: e95103, PI3K inhibitors (ClinicalTrials.gov ID NCT03274219), or epigenetic modifiers such as JQ1 [Id., citing Kagoya, Y. et al. J. Clin. Invest. (2016) 126: 3479-94. Although small molecule inhibitors such as AKTi can be applied easily to CAR T cells in culture, the disadvantage for such approaches is that the effect is transient, and once CAR T cells are infused into the patient they differentiate and exhaust in a normal manner.

Two research groups independently found that some CAR-T cells performed better than others when the transcription factor FOXO1 is overexpressed.

Chan, JD et al. [Nature (2024) 629: 201-10] sought to identify transcription factors that could enhance CAR-T cell fitness and efficacy against solid tumors and selected FOXO1 as a primary candidate, based on the significant enrichment of FOXO1 target genes in CAR-T cells cultured with IL-15 and its previously documented role in T cell memory formation [Id., citing Tejera, MM et al. J. Immunol. (2013) 191: 187-99] and metabolism [Id., citing Kousteni, S. Bone (2012) 50: 437-43]. FOXO1 was benchmarked against TCF7 (also known as TCF-1)-, ID3- and JUN-overexpressing CAR-T cells.

They showed that overexpression of FOXO1 promotes a stem-like phenotype in CAR-T cells derived from either healthy human donors or patients, which correlated with improved mitochondrial fitness, persistence and therapeutic efficacy in vivo to a greater extent than other transcriptional regulators upregulated by IL-15, such as TCF7 and ID3. FOXO1 overexpression implemented a broad transcriptional and epigenetic program that led to an enrichment of signatures associated with persistence, but did not preclude FOXO1-expressing CAR T cells from acquiring robust effector function upon antigen stimulation.

Investigating the potential of FOXO1 overexpression in the context of human CAR-T cells, they transduced human T cells with Lewis Y CAR (ClinicalTrials.gov ID NCT03851146)) linked to FOXO1-ADA (a constituitively active variant of FOXO1) via a P2A peptide. The FOXO1-ADA-overexpressing CAR T cells appeared less differentiated, as indicated by an increased frequency of CD62L+CD27+ cells and CD45RA+ cells concomitant with reduced expression of the exhaustion markers LAG3 and TIM3, a phenotype previously associated with improved CAR T cell responses in the clinic [Id., citing Fraietta, JA et al. Nat. Med. (2018) 24: 563-71; Guo, Y. et al. Clin. Cancer Res. (2018) 24: 1277-86]. However, FOXO1-ADA overexpression prevented human CAR T cells from acquiring an effector-like phenotype after activation. Although FOXO1-ADA-overexpressing CAR T cells significantly upregulated CD69 upon co-culture with tumor cells, their capacity to produce IFNγ and TNF was significantly attenuated relative to control CAR T cells. This suggested that expression of constitutively active FOXO1 restricted the capacity of CAR T cells to gain full effector function whereas overexpression of wild-type FOXO1 significantly enhanced the proportion of CD45RA+ and CD62L+CD27+ CAR T cells. In contrast to FOXO1-ADA expressing CAR T cells, wild-type FOXO1-expressing CAR T cells were able to produce similar levels of IFNγ and TNF to control CAR T cells upon co-culture with tumor cells.

They next compared the effects of FOXO1, TCF7 and ID3 on the phenotype of Lewis Y CAR T cells. FOXO1 was significantly more effective in the induction of the CD45RA+CD62L+ population of CD8+ CAR T cells relative to TCF7 and ID3, and the increased proportion of CD45RA+CD62L+ cells following FOXO1 expression was reproducible across multiple donors. FOXO1 overexpression was also associated with increased expression of CCR7 and CX3CR1, and reduced expression of CD39, TIM3 and PD-1. In the context of serial co-cultures with OVCAR-3 or MCF7 tumor cells, FOXO1 overexpression led to a significant increase in the recovery of CD8+ and CD4+ CAR T cells and similar levels of IFNγ and TNF production compared with control CAR T cells. Phenotypic analysis of FOXO1-overexpressing CAR T cells following co-culture with tumor cells revealed a similar expression of PD-1 and TIM3, but reduced expression of CD39 relative to control CAR T cells.

Further analysis of CD8+ and CD4+ CAR-T cells using RNA seq. revealed that FOXO1-overexpressing, but not TCF7-overexpressing, CD8+ CAR T cells exhibited increased expression of genes associated with less-differentiated T cells relative to their control counterparts. Moreover, FOXO1-overexpressing CD8+ CART cells displayed decreased expression of immune checkpoints relative to control counterparts and this effect was also more marked than with TCF7-expressing CAR T cells. After activation, the number of differentially expressed genes (DEGs) between control and FOXO1-expressing CAR T cells was significantly reduced, highlighting that expression of FOXO1 enabled the acquisition of a more stem-like phenotype without preventing the acquisition of effector function.

Furthermore, FOXO1 overexpression resulted in a significant negative enrichment of genes associated with exhaustion [Id., citing Wherry, E. et al. Immunity (2007) 27: 670-84] relative to control CAR T cells, an effect that was not observed following TCF7 overexpression. They next evaluated this in the context of stimulation and resting. In this repeat experiment, they again observed that prior to stimulation FOXO1-expressing CAR T cells exhibited reduced expression of exhaustion-related genes. Following stimulation through the CAR, both control and FOXO1-expressing CAR T cells upregulated these genes such that there was no significant difference between the groups. However, after a period of rest, the negative enrichment for this gene set was restored in FOXO1-expressing CAR T cells, highlighting that these cells exhibit durable protection from the transcription of genes associated with exhaustion.

scRNA-seq. analyses and unbiased clustering analysis confirmed that FOXO1 expression enhanced the proportion of CD8+ CAR-T cells with a more stem like phenotype, notably through enrichment of a cluster (cluster 1) characterized by high expression of KLF2, SELL and IL7R. This enhanced proportion of cluster 1 cells was offset by a reduction in the proportion of a cluster of cells (cluster 0) characterized by high expression of IFITM3 and TNFSF10. Comparing FOXO1-expressing CAR T cells in cluster 1 to control CAR-T cells in cluster 0 revealed a significant enrichment for genes associated with less differentiated cells and reduced expression of genes associated with glycolysis and exhaustion. When the analysis was repeated on only the activated cells, six distinct clusters of CD8+ CAR-T cells were observed. This analysis revealed that FOXO1 over expression led to an enrichment of a cluster (cluster 20 characterized by high expression of MK167 and a concomitant decrease in cluster 5. Comparison of the gene expression profiles of FOXO1-expressing cluster 2 cells and control cluster 5 cells revealed a positive enrichment for E2F target genes and glycolysis related genes, suggesting that FOXO1-expressing CAR-T cells are primed for a proliferative burst post-activation.

Epigenetic analysis showed that FOXO1 overexpression resulted in reduced expression of transcription factors previously observed to be upregulated in exhausted CAR T cells [Id., citing Lynn, RC et al. Nature (2019) 576: 293-300], including members of the AP-1 family such as IRF8 and FOSB.

Doan, AE et al. [Nature (2024) 629: 211] independently tested the hypothesis that overexpressing memory-associated transcription factors could reprogram CAR-T cells to durably persist and maintain anti-tumor activity. They showed that the transcription factor FOXO1 is responsible for promoting memory and restraining exhaustion in human CAR T cells. Pharmacological inhibition or gene editing of endogenous FOXO1 diminished the expression of memory-associated genes, promoted an exhaustion-like phenotype and impaired the antitumour activity of CAR T cells. Overexpression of FOXO1 induced a geneexpression program consistent with T cell memory and increased chromatin accessibility at FOXO1-binding motifs. CAR T cells that overexpressed FOXO1 retained their function, memory potential and metabolic fitness in settings of chronic stimulation, and exhibited enhanced persistence and tumor control in vivo.

They also examined TCF1, a transcription factor that defines stem-like or memory T cell populations that exhibit an increased capacity to respond to immune checkpoint blockade [Id., citing Fraietta, JA et al. (2018) Nat. Med. 24: 563-71; Chen, GM et al. Cancer Discov. (2021) 11: 2186-99; Krishna, S. et al. Science (2020) 370: 1328-34; Sade-Feldman, M. et al. Cell (20018) 175: 998-1013; Im, SJ et al. Nature (2016) 537: 417-21; Siddiqui, I. et al. Immunity (2019) 50: 195-211; Shan, Q. et al. Cell Mol. Immunol. (2021) 18: 1262077; Wu, T. et al. Sci. Immunol. (2016) 1: eaai8593; Tsui, C. et al. Nature (2022) 609: 354-60; Wang, Y. et al. Front. Immunol. (2019) 10: 169; Giuffrida, L. et al. Mol. Ther. (2020) 28: 2379-93; Hudson, WH et al. Immunity (2019) 51: 1043-58; Gattinoni, L. et al. Nat. Med. (2009) 15: 808-13]. Overexpressing TCF-1 did not enforce memory gene expression programs or enhance antitumor activity in vivo, which contradicts reports in mice [Id., citing Shan, Q. et al. Cell Mol. Immunol. (2021) 18: 1262077; Wu, T. et al. Sci. Immunol. (2016) 1: eaai8593]. Instead, TCF1 overexpressing cells exhibited a gene expression signature associated with Tpex cells, and manifested functional hallmarks of exhaustion during chronic stimulation, consistent with other studies. [Id., citing Chen, Z. et al. Immunity (2019) 51: 840-55; Roth, TL et al. Cell (2020) 181: 728-44].

Doan et al suggest that one possible interpretation is that constituitive TCF1 overexpression skews human engineered T cells toward a more exhausted or Tpex cell-like state, and/or that TCF7-expressing Tpex cells do not have a substantial role in CAR-T cell responses.

An alternative interpretation is that FOXO1, rather than TCF1, is mainly responsible for endowing tumor-reactive T cells with a stem-like or progenitor phenotype, and that TCF7 expression is merely a readout for FOXO1 activity. For example, results showed that deletion of endogenous TCF7 in overexpressed FOXO1 did not affect FOXO1-mediated transcriptional reprogramming or augmented antitumor function in vivo. Surface markers and transcription factors that are often co-expressed in TCF7+ cells are FOXO1 target genes [Id., citing Tsui, C. et al. Nature (2022) 609: 354-60], and an empiric FOXO1 regulon significantly correlated with TCF7 expression and clinical responses in samples of CAR T cells from patients, further supporting this notion. Conditional deletion of Foxo1 in mature mouse T cells diminished the frequency of Tcf7-expressing Tpex cells [Id., citing Utzschneider, DT et al. ell Rep. (2018) 22: 3454-67], suggesting that FOXO1 might promote cell states that are normally associated with high levels of Tcf7 expression.

According to some embodiments, CD4+ T cells, CD8+ T cells, or CD4+ and CD8+ T cells comprising an early stem cell phenotype can be derived from umbilical cord blood (UCB). More than 85% of UCB derived T cells have a naive phenotype (e.g., CD45RA+CCR7+), allowing them to induce fewer graft versus host disease during transplantation [Liu, D-D et al. Frontiers Oncol. (2022) 12: 944248, citing Kwoczek, J. et al. Transfusion (2018) 58: 88-99].

According to some embodiments, CD4+ T cells, CD8+ T cells, or CD4+ T cells and CD8+ T cells comprising an early stem cell phenotype can be derived from adult peripheral blood (PB). According to some embodiments, peripheral blood stem cells can be mobilized with an infusion of G-CSF. According to some embodiments, the peripheral blood is obtained by a blood draw by venipuncture. According to some embodiments, the mononuclear cell (MC) population can be collected from screened healthy volunteer donors by leukapheresis, enriched for PBMCs and cryopreserved; the other blood components are returned to the donor.

For example, CD45RA+ naive T cells can be isolated using a commercial human Naive Pan T Cell Isolation Kit (e.g., MACS, Miltenyi Biotec) and naive CD8+ T cells can be enriched by depleting CD4+ T cells, for example, with a CD4+ T Cell Isolation Kit (MACS, Miltenyi Biotech.

According to some embodiments, the receipient can be at least partially HLA matched against the umbilical cord blood or adult peripheral blood.

According to some embodiments, a protocol for preserving early memory T cells in peripheral blood comprises (1) enriching naive T cells (CD45RA+CCR7+CD62L+CD95^{neg}CD45RO^{neg}); (2) activating the naive T cells with soluble anti-CD3/CD28 antibodies (ImmunoCult^{™}); (3) using PD-1 and TIGIT expression identifying and separating functional from dysfunctional T_{STEM} progenitors; (4) transducing the functional activated naive T cell population with a CAR; and (5) expanding the functional activated transduced cells for 3 days in the presence of IL-7 and IL-15, [Meyran, D. et al. Sci. Trans. Med. (2023) 15: eabk 1900]. This protocol has been reported to result in significantly increased T_{SCM} (CD62L+CD95+CD45RA+). Two distinct CD95+CCR7+CD8+ memory T cell subsets, namely T_{STEM} and T_{PEX}, were identified [Id., citing Galletti, G. et al. Nat. Immunol. (2020) 21: 1552-62]. While the T_{STEM} progenitors lacked expression of PD-1 and TIGIT and were committed to a functional lineage, T_{PEX} progenitors expressing PD-1 and TIGIT were committed to a dysfunctional, exhausted-like lineage.

According to some embodiments, the CD4+ T cells, CD8+ T cells, or CD4+ and CD8+ T cells comprising early stem cell phenotype TCF-1^{hi} Tox^{lo} expression can be modulated by epigenetic modification as needed.

Enormous advances have been made in our understanding of how genetic and epigenetic mechanisms regulate physiological and pathological gene expression by global projects, such as the Encyclopedia of DNA Elements (ENCODE, 2003), The Cancer Genome Atlas (TCGA, 2006), the International Cancer Genome Consortium (ICGC, 2008), the National Institutes of Health Roadmap Epigenomics Mapping Consortium (2008), and the European Community initiative BLUEPRINT (2011). [Nebbioso, A. et al. PLoS Genetics (2018) 14 (6): e1007362]. By applying next-generation sequencing-based approaches, these projects revealed epigenomic profiles in both healthy and pathological conditions. Epigenomic profiling has greatly enhanced our understanding of complex human diseases, including cancer. The International Human Epigenome Consortium (IHEC, 2010) [Id., citing Stunnenberg, HG et al. Cell (2016) 167: 1897] was founded to coordinate international efforts with the aim of producing reference maps of at least 1,000 epigenomes for key cellular states relevant to health and disease [Id., citing Abbott, A. Nature (2010) 463: 596-7] and to disseminate data to improve clinical applications. In 2012, ENCODE annotated functional elements within the entire genome, identifying regions of transcription, transcription factor (TF) association, chromatin structure, and histone modification in 147 different cell types [Id., citing Consortium EP Nature (2012) 489: 57-74]. In 2015, the Roadmap Consortium extended ENCODE findings, clarifying the role of epigenetic mechanisms in human biology and disease [Id., citing Roadmap Epigenomics C. et al. Nature (2015) 518: 317-30] and creating a publicly accessible collection of 127 human epigenomes. Roadmap scientists matched this epigenomic dataset to trait- and disease-associated variants identified by genome-wide association studies (GWAS). The scientific achievements of IHEC was partially released as a package in Cell and Cell Press-associated journals (http://www.cell.com/consortium/IHEC), Each of these references is incorporated herein by reference.

***Background.*** Epigenetic gene expression patterns that cannot be attributed to DNA sequence variations are tightly controlled by the 3-dimensional architecture of chromatin and the action of multi-protein complexes, especially RNA polymerase, which transcribes DNA into RNA. In the nucleus, genomic DNA is wrapped around histones into nucleosome subunits that are condensed into chromatin. Highly condensed chromatin, termed heterochromatin, contains mostly inactive genes. In contrast, euchromatin has a more open structure and contains active genes. Chromatin structure is dynamically regulated by DNA methylation, nucleosome positioning, and histone modifications. [ Bennett, RL and Licht, JD. Targeting Epigenetics in Cancer. Annu. Rev. Pharmacol. Toicol. (2018) 58: 187-207]

As depicted in **Fig. 5**, a wide range of post-translational modifications can mark histones, such as acetylation, methylation, ubiquitination or phosphorylation. Histone modifications bring about chromatin remodeling by two main mechanisms. First, marks such as acetylation or phosphorylation neutralize the positive charge of lysines, weakening interactions among nucleosomes and between DNA and histones to increase chromatin accessibility [Bennett, RL and Licht, JD. Annu. Rev. Pharmacol. Toxicol. (2018) 58: 187-207, citing Luger, K. and Richmond, TJ. Curr. Opin. Genetics & Devel. (1998) 8: 140-6; Steger, DJ and Workman, JL. BioEssays: news and reviews in molecular, cellular and devel. biol. (1996) 18: 875-84]. Second, histone modifications may serve as docking sites for additional histone modifying enzymes or specific chromatin factors that regulate chromatin architecture and/or gene expression.

Unlike regulation of genetic events, epigenetic regulation is a dynamic and reversible process. Proteins that carry out these epigenetic modifications can be thought of as writers, readers and erasers. Epigenetic writers catalyze the addition of epigenetic marks onto either DNA or histones, most commonly on "tails" of histones that extend from the octamer structure. Readers recognize or are recruited to a specific epigenetic mark. Erasers remove epigenetic marks.

### Targeting DNA methylation

DNA methylation plays a key role in regulating chromatin architecture and gene expression. It usually occurs on cytosines that precede a guanine (CpG). The presence of endogenous palindromic CpG methylation patterns in the genome and transmission of these methylation marks through the germline has now been well established. The majority of CpG dinucleotides are concentrated within CpG-rich DNA regions termed "CpG islands" that are located near transcription start sites (TSSs) at about 70% of gene promoters [Id., citing Saxonov, S. et al. Proc. Natl Acad. Sci. USA (2006) 103: 1412-7]. In addition, orphan CpG islands located far from TSSs and CpG dinucleotides in gene bodies or enhancers may be methylation targets that can regulate gene expression [Illingworth, RS et al. PLoS Genetics (2010) 6: e1001134; Zilberman, D. et al. Nature Genetics (2007) 39: 61-9]. Hypermethylation of CpG islands may lead to silencing of the corresponding gene by precluding the binding of transcription factors and recruitment of methyl-CpG binding proteins that interact with repressive histone modifying enzymes [Id., citing Herman, JG, and Baylin, SB. New Engl. J. Med. 92003] 349: 2042-54; Rodriguez, C. et al. Biochem. Biophys. Res. Commun. (2010) 392: 129-34].

DNA hypomethylating agents and histone deacetylase inhibitors are approved for some hematologic malignancies, including T-cell lymphoma (vorinostat 2006, romidepsin 2009), multiple myeloma (panobinostat, 2015) and myelodysplastic syndrome (MDS) (decitabine or azacitidine). Developed originally as high-dose cytotoxic anti-leukemia agents it is now understood that at low dose the cytosine analogues 5'-azacytidine (Aza, Vidaza) and 5-aza-2'-deoxycytidine (Decitabine, Dacogen) inhibit DNMT activity resulting in hypomethylation. While hypomethylating agents have been shown to reverse promoter methylation and reactivate silenced tumor suppressor gene expression, other mechanisms may also be important [Id., citing Tsai, HC et al., Mol. Cell Biol. (2008) 28: 752-71] for instance, treatment with decitabine causes the formation of DNA-DNMT adducts and subsequent double-stranded DNA breaks resulting in G2 arrest [Id., citing Palii, SS et al. Molec. Cell Biol. (2008) 28: 752-71]. In addition, DNMTs have been found in complexes with histone modifying enzymes and a global increase of histone H3 and H4 acetylation has been observed after treatment with Aza [Id., citing Gore, SD et al. Cancer Res. (2006) 66: 6361-9; Fuks, F. et al. Nature Genetics (2000) 24: 88-91; Fuks, F. et al. Nucleic Acids Res. (2003) 31: 2305-12]. Furthermore, decitabine stimulates nuclear localization of IRF7 in colon cancer cells to cause expression of toxic endogenous retroviral sequences independent of promoter methylation [Id., citing Roulois, D. et al. Cell (2015) 162: 961-73].

Defects in demethylation of DNA can also lead to aberrant hypermethylation and altered expression of genes that drive neoplasia. Isocitrate dehydrogenases (IDHs) catalyze the oxidative decarboxylation of isocitrate to α-ketoglutarate and reduce NAD(P)+ to NAD(P)H. Compounds specifically targeting mutant IDH have been developed (AG-120 and AG-221) and are in clinical trials for patients with advanced hematologic malignancies [Id., citing Stein, EM et al. Blood (2015) 126: 323]. These agents would be expected to block the production of 2-hydroxygluterate (2-HG) from α-ketoglutarate by the mutant enzymes and allow DNA and histone methylation patterns to normalize.

Mutant IDH1/2 reduces α-ketoglutarate to 2-hydroxygluterate (2-HG), a competitive inhibitor of the TET family of DNA hydroxylases [Id., citing Ward, PS et al. Cancer Cell (2010) 17: 225-34]. In addition, mutations and translocations of TET2 have been observed in numerous hematologic malignancies and associated with poor prognosis in AML [Id., citing Delhommeau, F. et al. New Engl. J. Med. (2009) 360: 2289-301]. TET enzymes normally convert 5'-methylcytosine to 5'-hydroxymethylcytosine, an important step in cytosine demethylation and inhibition of this mechanism results in DNA hypermethylation [Id., citing Figueroa, ME et al. (2010) 18: 553-67]. Furthermore, 2-HG has been reported to inhibit α-ketoglutarate dependent Jumonji domain lysine demethylases and activate mTOR [Id., citing Lu, C. et al. Nature (2012) 483: 474-8; Carbonneau, M. et al. Nature Communmic. (2016) 7: 12700].

### Targeting histone acetylation

Acetylation of lysine on histone tails is highly dynamic and important for regulation of chromatin structure, transcription and DNA repair. Two competing enzyme families, histone lysine acetyltransferases (HATs) and histone deacetylases (HDACs), regulate histone acetylation. The about 30 known HATs are classified into two groups based on their capacity to acetylate nucleosomal histones. Type A HATs are located in the nucleus and acetylate chromatin bound histones and nuclear proteins. Type B HATs acetylate newly translated, but not nucleosomal, histones H3 and H4. Based on structural and functional homology the type A HATs are further categorized into families that include: GCN5, MYST, p300/CBP, transcriptional coactivators and steroid receptor coactivators. HATs catalyze the transfer of an acetyl group from acetyl-CoA to the amino group of a histone lysine residue. Upon acetylation, the positive charge on lysine is neutralized diminishing the interaction of histones with DNA. In general this leads to a more open chromatin structure that is accessible to binding of proteins such as transcription factors. Thus, acetylation is associated with transcriptional activation while deacetylation is associated with gene repression. [Bennett, RL and Licht, JD. Annu. Rev. Pharmacol. Toicol. (2018) 58: 187-207].

Many reports demonstrate that HDACs are overexpressed in cancers resulting in global loss of histone acetylation and silenced tumor suppressor gene expression. HDACs are divided into four classes based on their homology and structure. Classes I, II and IV are comprised of Zn-dependent HDACs while class III is made up of the NAD-dependent sirtuins. Class I HDACs (1, 2, 3 and 8) and class II HDACs (4, 5, 6, 7, 9 and 10) have been reported to play roles in tumorigenesis. HDACi work through many mechanisms to promote cell cycle arrest, induce differentiation and activate apoptosis pathways in cancer cells. Numerous synthetic or natural product HDAC inhibitors (HDACi) have been developed for cancers that display increased HDAC activity or HAT mutations. Based on their chemical structure, HDACi can be organized into groups consisting of hydroxamates, benzamides, cyclic peptides or short-chain fatty acids. Because HDACs are Zn2+ dependent many HDACi target the Zn2+ ion in the active site of HDACs to inhibit their enzymatic activity. Nonselective broad spectrum HDACi that inhibit all zinc dependent HDACs include the hydroxamate class agents vorinostat, belinostat and panobinostat. Vorinostat induces cell cycle arrest, promotes apoptosis, sensitizes cells to other chemotherapy and has been approved to treat patients with cutaneous T-cell lymphoma (CTCL) [Id., citing Xue, K. et al. J. Cancer Research Clinical Oncology (2016) 142: 379-87]. Belinostat was approved to treat peripheral T-cell lymphomas (PTCL) and panobinostat to treat multiple myeloma (MM). These drugs are also in clinical trials to treat solid tumors. Nonselective HDACi such as vorinostat can reverse aberrant epigenetic chromatin changes to reactivate tumor suppressor genes such as p21 [Id., citing Richon, VM et al. Proc. Natl Acad. Sci. USA (2000) 97: 10014-9]. In addition, HDACs have multiple functions in the cell and also target non-histone proteins. For instance, acetylation enhances the activity of some transcription factors such as p53 and GATA-1 [Id., citing Terui, T. et al. Cancer Res. (2003) 63: 8948-54; Lamonica, JM et al. Blood (2006) 108: 3736-8].

### Targeting histone methylation & demethylation

Dynamic methylation of nucleosomal histones plays an important role in regulation of gene expression. The two major families of histone methyl transferases are lysine methyl transferases (KMTs) and protein arginine methyltransferases (PRMTs). In recent years mono-, di- or tri-methylation of histone lysine methylation has been characterized to alter affinity of reader proteins to the methylated histones. Specific lysine methylation marks on H3K4, H3K36 and H3K79 are associated with activation of gene expression while marks associated with repression are H3K9, H3K27 and H4K20 [Id., citing Barski, A. et al. Cell (2007) 129: 823-37]. Importantly, different methylation states (e.g. me0, me1, me2, me3) of the same lysine residue occur in different contexts and may have distinct functional consequences.

Two classes of lysine demethylases (KDMs) govern demethylation of histones: the amine oxidases that include lysine-specific demethylase 1 (LSD1) and the α-ketoglutarate-dependent Jumonji domain (JmjC) containing demethylases. The LSD-family KDMs only demethylate mono- and dimethylated lysines while the JmjC demethylases remove methyl from all three states of lysine methylation [Id., citing Shi, Y. et al. Cell 92004] 119: 941-53; Tsukada, Y. et al. Nature (2006) 439: 811-6]. Target specificity of KDMs is often regulated by their participation in different complexes.

### Targeting readers of epigenetic marks

Epigenetic reader proteins recognize and bind chromatin or histone modifications to either directly induce chromatin structural changes (e.g. compaction, remodeling) recruit secondary chromatin modifiers or serve as scaffold proteins for various nuclear processes such as transcription, replication or repair. Acetylated lysines are binding sites for proteins containing bromodomains such as the Bromodomain and Extra Terminal (BET) proteins BRD2, BRD3, BRD4 and BRDT. Readers that recognize methyl-lysine include MBT domain, Tudor domain, and Chromodomain proteins [Id., citing Kim, J. et al. EMBO Reports (20006) 7: 397-403]. Chromatin reader proteins can include several types of reader domains and binding at a specific chromatin site may depend on adjacent histone modifications.

***Targeting miRNA signatures.*** Specific miRNA signatures have been correlated with molecular subtypes (tumor aggressiveness, drug response, and patient outcome), for example, in breast cancer. [Nebbioso, A. et al. PLoS Genetics (2018) 14 (6): e1007362., citing Blenkiron, C. et al. Genome Biol. (2007) 8: R214]; de Rinaldis, E. et al. BMC Genomics (2013) 14: 643; Dvinge, H. et al. Nature (2013) 497: 378-82; van Schooneveld, E. et al. Breast Cancer Res. (2012) 14: R34; Fkih, M. et al. Cell Oncol. (Dordr) (2015) 38: 433-42].

### Role of epigenetic mechanisms in T cell functional and dysfunctional states

It is now understood that activation of CD8+ T cells to CD 8+ effector cells entails a transition from naive cells to a state where thousands of regions of DNA and chromatin are epigenetically modified to facilitate the drastic functional change. Upon clearance of the pathogen, most CD8+ T cells die. Long-lived memory CD8 T cells are derived from a subset of effector T cells through a process of dedifferentiation, whereby the subset of cells that gives rise to memory cells acquires de novo DNA methylation programs at naive-associated genes and became demethylated at loci of classically defined effector molecules, thus retaining many epigenetic marks of their effector state. This epigenetic marking likely accounts for the ability of memory CD8+Tcells to rapidly recall proliferation and effector function upon a second encounter with the pathogen. [Youngblood, B. et al. Nature (2017) 552 (7685): 404-9].

***T cell exhaustion.*** Accumulating evidence suggests that the altered functional state of exhausted T cells is broadly associated with an epigenetic program that is distinct from either an acute effector or memory state. [Paulken, KE et al. Science (2016) 3354: 1160-65; Philip, M. et al. Nature (2017) 545: 452-6; Jadhav, RR, et al. Proc. Natl Acad. Sci. USA (2019) 116 (28): 14113-8:

As reported by Jadhav, et al., a characteristic feature of exhausted CD8 T cells is expression of various inhibitory receptors [Jadhav, RR, et al. Proc. Natl Acad. Sci. USA (2019) 116 (28): 14113-8, citing Barber, DL et al. Nature (2006) 439: 682-87; Blackbum, SD et al. Nat. Immunol. (2009) 10: 29-37].

Im, et al. [Id., citing Im, SJ et al. Natue (2016) 537: 417-21]identified a novel population of PD-1 (programmed cell death 1)+ TCF-1 (T cell factor 1)+ virus-specific CD8 T cells that function as resource cells during chronic LCMV infection and provide the proliferative burst seen after PD-1 blockade. Such CD8 T cells also have been found in other chronic infections in mice and humans [Id., citing He, R. et al. Nature (2016) 537: 412-28; Ultzschneider, D.T. et al. Immunity (2016) 45: 415-27; Wu, T. et al. Sci. Immunol (2016) 1: eaai8593; Leong, YA et al. Nast. Immunol. (2016) 17: 1187-96; Miles, B. et al. PLoS Pathog. (2016) 12: e1005924; Mylvaganam, GH et al. Proc. Natl Acad. Sci. USA (2017) 114: 1976-8]. In addition, these cells have been identified in human cancer; the reported data suggests that the frequency of these cells is associated with the clinical outcome of checkpoint immunotherapy [Id., citing Sade-Feldman, M. et al. Cell (2018) 175: 998-1013; Soddoqio. O/ et al. Immunity (2019) 50: 195-211; Miller, BC et al. Nat. Immunol. (2019) (20) 326-36]. These CD8 T cells exhibit stem-like properties undergoing self-renewal and also differentiating into the terminally exhausted CD8 T cells.

Assay for transposase-accessible chromatin using sequencing (ATAC-seq) analysis of these stem-like CD8 T cells from LCMV chronically infected mice was compared with the epigenetic profile of more terminally differentiated (exhausted) CD8 T cells and with the epigenetic signature of effector and memory CD8 T cells generated following an acute LCMV infection. Sites of differential accessibility using DESeq2 [Id., citing Love, MI et al. Genome Biol. 15, 550 (2014) were identified to determine the epigenomic markers defining the stem-like CD8 T cells and exhausted CD8 cells.

Results showed that the epigenetic signature of the stem-like CD8 T cells from chronically infected mice was different, not only from the exhausted CD8 T cells but also distinct from the epigenetic profile of effector and memory CD8 T cells generated during acute infection.

In brief, Jadhav, et al. found that TCF-1 is essential for the generation of the stem-like CD8 T cells, CCR7 is important for localization in the T zones, and CD28 signaling is required for the rescue and proliferation of stem-like CD8 T cells after PD-1 blockade. [Id., citing Im S. J., et al., Nature (2016) 537: 417-421; Kamphorst A. O., et al., Science (2017) 355, 1423-1427].

ATAC-seq analysis showed that stem-like CD8 T cells had a unique signature implicating activity of HMG (TCF) and RHD (NF-κB) transcription factor family members in contrast to higher accessibility to ETS and RUNX motifs in exhausted CD8 T cells. In addition, regulatory regions of the transcription factors Tcf7 and Id3 were more accessible in stem-like cells whereas Prdm1 and Id2 were more accessible in exhausted CD8 T cells. Both the stem-like CD8 T cells and exhausted CD8 T cells generated during chronic infection were strikingly different from CD8 T cell subsets from acute infection. These TCF1+PD-1+ stem-like CD8 T cells therefore represent a specific adaptation of the CD8 T cell response to chronic antigen stimulation.

Using a chronic stimulation assay, Belk et al developed an in vitro system of chronic antigen stimulation to model T cell exhaustion. [Belk, JA et al. Cancer Cell (2022) 40: 768-86] In this model, T cells are continuously stimulated with an antibody agonist against the TCR, and over the course of 10 days, the cells develop an exhausted epigenetic profile. Using this system, Belk et al performed genome-wide CRISPR-Cas9 screens of over 90,000 sgRNAs to systematically identify genes involved in the transition to T cell exhaustion, which identified an enrichment of epigenetic factors. [Belk, JA et al. Cancer Cell (2022) 40: 768-86]. In vivo CRISPR screen in murine and human tumor models demonstrated that perturbation of the INO80 and BAF chromatin remodeling complexes improved T cell persistence in tumors. In vivo Perturb-seq revealed distinct transcriptional roles of each complex, and that depletion of canonical BAF complex members, including *Arid1a, Smarccl.* and *Smarcd2*, resulted in the maintenance of an effector program and down regulation of exhaustion-related genes in tumor-infiltrating cells. When transferred into tumor-bearing mice, *Arid1a* depletion limited the acquisition of exhaustion -associated chromatin accessibility. *Arid1a*-deficient CD8+ T cells retained open regions of chromatin around genes encoding key functional molecules like IFNgamma and less accessibility near genes like PDCD1, CD39 and Tox, which are associated with exhaustion, and led to improved antitumor immunity.

Similar to inhibition of *Arid1a,* blocking the function of DNA methylation enzyme DNMT3A can also prevent the acquisition of the exhausted epigenetic state and limit the severity of the exhaustion, allowing T cells to remain functional for longer time.[ Kissick, H. and Ahmed, R. Cancer Cell (2022) 40: 708-10, citing Ghoneim, HE et al., Cell (2017) 170: 142-57; Prinzing, B. et al. Sci. Transl. Med. (2021) 13: eabh0272].

An exemplary process for creating VST-CAR-T cells that express a nonexhausted PD-1^{neg} TIGIT^{neg} TCF-1^{hi} TOX^{lo} early T stem cell (T_{SCM}) phenotype comprises:
(i) enriching for a population of CD4+ T cells, CD8+ T cells or a combination thereof that express a nonexhausted PD-1^{neg}, TIGIT^{neg} TCF-1^{hi}TOX^{lo} early T stem cell (T_{SCM}) phenotype;
(ii) generating by transduction of the population of CD4+ T cells, CD8+ T cells or a combination thereof with a viral vector encoding a protein of a virus an expanded population of allogeneic virus-specific T cells (VSTs) comprising T cell receptors (TCRs) specific for the protein of the virus; and
(iii) genetically modifying the population of allogeneic VSTs in (ii) to stably express up to three chimeric antigen-receptors (CARs), wherein each CAR is specific for a cancer antigen, wherein to reduce risk of host versus graft rejection (HVGR), one or more of the CARs comprise:
   (1) a-CD30 CAR expressing a CD30-specific scFv fragment, or
   (2) a chimeric alloimmune defense receptor (ADR), or
   (3) a chimeric HLA accessory receptor fused with a cytolytic endodomain of T cell receptor zeta chain, or
   (4) a-CD30 CAR expressing a CD30-specific scFv fragment, and a chimeric HLA accessory receptor fused with a cytolytic endodomain of T cell receptor zeta chain; or
   (5) a-CD30 CAR expressing a CD30-specific scFv fragment, and a chimeric alloimmune defense receptor (ADR); or
   (6) a chimeric alloimmune defense receptor (ADR), and a chimeric HLA accessory receptor fused with a cytolytic endodomain of T cell receptor zeta chain, or
   (7) a combination thereof.

According to some embodiments, one or more of the following methods can be used to generate a population of nonexhausted PD-1^{neg}, TIGIT^{neg} TCF-1^{hi}TOX^{lo} early T stem cell (T_{SCM}) phenotype.

According to some embodiments, the population of CD4+ T cells, CD8+ T cells or combination thereof can be genetically modified to overexpress FOX01, which is associated with increased TCF-1 expression and decreased TOX expression to generate the population of a nonexhausted PD-1^{neg}, TIGIT^{neg} TCF-1^{hi}TOX^{lo} early T stem cell (T_{SCM}) phenotype.

According to some embodiments, biallelic disruption of TET2 via CAR integration into intron 9 [Ali, A. et al. Molecular Therapy (2024) 32 (6): doi.org/10.1016/j.ymthe.2024.06.007, citing Fraietta, JA et al. Nature (2018) 558: 307-312] can be used to generate the population of a nonexhausted PD-1^{neg}, TIGIT^{neg} TCF-1^{hi}TOX^{lo} early T stem cell (T_{SCM}) phenotype.

According to some embodiments, deletion of DNMT3A, a methyltransferase and epigenetic modifier [Ali, A. et al. Molecular Therapy (2024) 32 (6): doi.org/10.1016/j.ymthe.2024.06.007, citing Prinzing, B. et al. Sci. Transl. Med. (2021) 13: eabh0272] can be used to generate a nonexhausted PD-1^{neg}, TIGIT^{neg} TCF-1^{hi}TOX^{lo} early T stem cell (T_{SCM}) phenotype.

According to some embodiments, disrupting histone methylation patterns by disrupting histone methyltransferase SUV39H1 [Id., citing Jain, N. et al. Cancer Discov. (2024) 14: 142-57; Lopez-Cobo, S. et al. Cancer Discov. (2024) 14: 120-41] can be used to generate a nonexhausted PD-1^{neg}, TIGIT^{neg} TCF-1^{hi}TOX^{lo} early T stem cell (T_{SCM}) phenotype.

According to some embodiments, CAR-T cells generated in the presence of exogenous transforming factor beta to promote chromatin accessibility at resident memorty T cell gene loci and to downregulate genes that dictate T cell commitment to recirculation [Id., citing Jung, IY et al., Cell Rep. Med. (2023) 4: 101053] can be used to generate a nonexhausted PD-1^{neg}, TIGIT^{neg} TCF-1^{hi}TOX^{lo} early T stem cell (T_{SCM}) phenotype.

According to some embodiments, Iosine exposure (INO) [Id., citing Klysz, DD et al. Preprint at bioRxiv. https://doi.org/10.1101/2023.04.21.537859] to induce epigenetic reprogramming in CAR-T cells, leading to enhanced chromatin accessibility at sternness gene loci and transcription factor binding motifs associated with memory differention, can be used to generate a nonexhausted PD-1^{neg}, TIGIT^{neg} TCF-1^{hi}TOX^{lo} early T stem cell (T_{SCM}) phenotype.

According to some embodiments, EZH2 inhibition [Id., citing Weber, EW et al., Science (2021) 372: eaba 1786]] and overexpression of c-Jun (Id., citing Lynn, RC et al. Nature (2019) 576: 293-300) can be used to generate a nonexhausted PD-1^{neg}, TIGIT^{neg} TCF-1^{hi}TOX^{lo} early T stem cell (T_{SCM}) phenotype.

CD4+ T cells, CD8+ T cells or both CD4+ T cells and CD8+ T cells expressing a nonexhausted phenotype are identified by flow cytometry, optionally separated by negative selection methods, and stored and transported below -120° C until needed.

According to some embodiments, the population of CD4+ T cells, CD8+ T cells, or both CD4+ T cells and CD8+ T cells expressing a nonexhausted PD-1^{neg} TIGIT^{neg} TCF-1^{hi} TOX ^{lo} early T stem cell (T^{SCM}) phenotype is expanded in vitro in the presence of one or more cytokines, e.g., IL-7, IL-15, IL-21 [Gattinoni, L. et al. Nature Reviews Cancer (2012) 12: 671-84; Zeng, R. et al. J. Exp. Medf. (2005) 139-48; Li, Y. et al. J. Immunol. (2005) 175: 2261-69]. According to some embodiments, in vitro assays are used to measure effector function of the population of CD4+ T cells, CD8+ T cells, or both CD4+ T cells and CD8+ T cells expressing a nonexhausted phenotype, e.g., cytokine secretion and cytotoxicity.

According to some embodiments, the recipient can be at least partially HLA matched against the umbilical cord blood or the adult peripheral blood.

According to some embodiments, the population of CD4+ T cells, CD8+ T cells or both CD4+ T cells and CD8+ T cells expressing a nonexhausted phenotype comprising a PD1^{neg}TIGIT^{neg}TCF-1^{hi}TOXl^{o} early stem cell (T_{SCM}) phenotype is expanded in vitro in the presence of an epigenetic modification agent. Examples of epigenetic modification agents include, without limitation, belinostat, vorinostat, romidepsin, panobinostat, decitabine, or azacitidine.

### (f) iteratively dosing the patient with a new nonexhausted batch of the allogeneic population of CD4+ T cells, CD8+ T cells or both CD4+ T cells and CD8+T cells PD-1^{neg} TIGIT^{neg} TCF-1^{hi} TOX^{lo} early stem cell T cell (T_{SCM}) expressing the nonexhausted phenotype of step (e) as needed until all cancer in the body is destroyed

According to some embodiments, the population of functional CD4+ T cells,CD8+ T cells or both CD4+ T cells and CD8+ T cells expressing the nonexhausted phenotype can be prepared in batches that can be available if redosing is necessary without a prolonged waiting time. According to some embodiments, the cell therapy remains effective until the cancer in the body of the recipient is destroyed.

According to some embodiments, a new nonexhausted batch of the population of functional CD4+ T cells, CD8+ T cells or both CD4+ T cells and CD8+ T cells expressing the nonexhausted phenotype can be administered to the subject periodically, as needed, until all cancer in the body is destroyed.

According to some embodiments, the method further includes administering an immune checkpoint inhibitor at a standard dose for the indication as established and described on the package insert. Examples of commercially available checkpoint inhibitors include anti-PD1 (e.g., lambrolizumab, pembrolizumab (KEYTRUDA^{®}) and nivolumab (OPDIVO^{®}), anti-PD-L1 (MPDL3280A, Atezolizumab, TECENTRIQ^{®}), and anti-CTLA4 (ipilimumab, YERVOY^{®}). For example, the recommended dose and schedule of pembrolizumab for NSCLC is 200 mg intravenously every three weeks. The recommended dose and schedule for ipilimumab injection for unresectable or metastatic melanoma is 3 mg/kg every 3 weeks for a total of 4 doses. The recommended dose and schedule for advanced renal cell carcinoma is nivolumam 3 mg/kg followed by YERVOY 1 mg/kg on the same day, every 3 weeks for 4 doses, then nivolumab 240 mg every 2 weeks or 480 mg every 4 weeks. The recommended dose and schedule for hepatocellular carcinoma is nivolumag 1 mg/kg followed by YERVOY 3 mg/kg on the same day every 3 weeks for 4 doses, then nivolumab 240 mg every 2 weeks or 480 mg every 4 weeks. The recommended dose and schedule for imetastatic non-small cell lung cancer is nivolumab 3 mg/kg every 2 weeks with YERVOY 1 mg/kg every 6 weeks.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges which may independently be included in the smaller ranges is also encompassed within the invention, subject to any specifically excluded limit in the stated range. Where the stated range includes one or both of the limits, ranges excluding either both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, exemplary methods and materials have been described. All publications mentioned herein are incorporated herein by reference to disclose and described the methods and/or materials in connection with which the publications are cited.

It must be noted that as used herein and in the appended claims, the singular forms "a", "and", and "the" include plural references unless the context clearly dictates otherwise.

The publications discussed herein are provided solely for their disclosure prior to the filing date of the present application and each is incorporated by reference in its entirety. Nothing herein is to be construed as an admission that the present invention is not entitled to antedate such publication by virtue of prior invention. Further, the dates of publication provided may be different from the actual publication dates which may need to be independently confirmed.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Centigrade, and pressure is at or near atmospheric.

### Example 1.

Multi-viral antigen specific T cell receptor targeted (VST) allogeneic CAR T cells are generated by transduction with a viral vector encoding proteins of CMV and EBV from healthy donor T cells and cord blood T cells into which an alloimmune defense receptor (ADR) anti-CD30 CAR and a tumor antigen specific CD19 CAR are then inserted. These CAR T cells are then enhanced for sternness using genetic engineering methods that result in increased production of T_{scm} CD4 and CD8 CAR-T cells (e.g., by overexpressing FOXO1; or by biallelic disruption of TET2 via CAR integration into intron 9; or by deleting DNMT3A; or by disrupting histone methyltransferase SUV39H1; or by generating the CAR-T cells in presence of exogenous transforming factor beta; or by exposure to Iosine; or by EZH2 inhibition and overexpression of c-Jun; or by a combination thereof) to be tested in a mouse Daudi (Burkitt's) CD19-expressing lymphoma model as described by Ruggeri Barbara, N. et al. [J. Immunother. Cancer (2024) 12:e008656. Doi: 10.11.36/jitc-2023-008656, which is incorporated herein by reference].

To assess efficacy in vivo, the CAR-T cells are evaluated in a disseminated Daudi (Burkitt's) lymphoma xenograft model in NOD.Cg-Prkdc^{scid} Il2rg^{tm1Wjl/}SzJ (NSG) mice (Jackson Laboratory). Fifteen 9-week-old female mice are housed together in cages and acclimated for 2 weeks. Five experimental mice per group were used to determine statistical significance of treatment effect on group survival. All mice are preconditioned with a 30 mg/kg dose of busulfan 7 days prior to Daudi inoculation. On day 0, mice receive a 3×106 dose of luciferase-labeled Daudi (Daudi-luc+) cells via intravenous administration. In order to identify the most killing potential and the most persistence, seven days post-tumor cell inoculation, mice are randomized based on body weight and treated by intravenous injection with
(1) vehicle (n=5; control group),
(2) 3×10⁶ human multi-virus specific T cells transduced with a viral vector encoding viral proteins of CMV plus EBV transduced with anti-CD30 CAR and tumor antigen specific CD19 CAR (n=5) cells derived from either healthy donor T cells or cord blood T cells; and
(3) 3 × 10⁶ human multivirus-specific T cells transduced with a viral vector encoding viral proteins of CMV plus EBV transduced with anti-CD30 CAR and tumor antigen specific CD19 CAR (n=5) cells derived from either healthy donor T cells or cord blood T cells expressing a nonexhausted phenotype comprising a PD1^{neg}TIGIT^{neg}TCF-1^{hi}TOX^{lo} early stem cell (T_{SCM}) phenotype genetically engineered: to overexpress FOXO1; or by biallelic disruption of TET2 via CAR integration into intron 9; or by deleting DNMT3A; or by disrupting histone methyltransferase SUV39H1; or by generating the CAR-T cells in presence of exogenous transforming factor beta; or by exposure to Iosine; or by EZH2 inhibition and overexpression of c-Jun; or by a combination thereof.

CAR-T antitumor efficacy is monitored via bioluminescence imaging and survival. On day 122, surviving mice (n=5) are rechallenged with 3×10⁶ Daudi-luc+ cells via intravenous administration. Age-matched NSG mice (n=5) serve as controls and are injected with the same dose of Daudi-luc+ cells. Blood, spleen, and bone marrow samples are collected on days 185-215 from the remaining mice (n=4) and analyzed by flow cytometry for the presence and phenotype of persisting CAR-T cells.

While the present invention has been described with reference to the specific embodiments thereof it should be understood by those skilled in the art that various changes may be made and equivalents may be substituted without departing from the true spirit and scope of the invention. In addition, many modifications may be made to adopt a particular situation, material, composition of matter, process, process step or steps, to the objective spirit and scope of the present invention. All such modifications are intended to be within the scope of the claims appended hereto.

## Claims

1. An (off-the shelf) allogeneic CAR-T cell immunotherapy method, suitable for treating a cancer, comprising preparing a composition by:
a) creating a population of allogeneic multivirus-specific T cells (MV-VSTs), having a reduced risk of graft versus host disease (GVHD), by transducing allogeneic T cells with a viral vector encoding a protein of a virus, wherein the population of VSTs comprise T cell receptors (TCRs) specific for the protein of the virus;
b) creating a population of cancer killing VST-CAR-T cells, by genetically modifying the (population of) allogeneic VSTs to stably express up to three chimeric antigen-receptors (CARs),
wherein each CAR is specific for a cancer antigen,
wherein (so as to reduce risk of host versus graft rejection (HVGR)), one or
more of the CARs can comprise:
(i) a-CD30 CAR expressing a CD30-specific scFv fragment, or
(ii) a chimeric alloimmune defense receptor (ADR), or
(iii) a chimeric HLA accessory receptor fused with a cytolytic endodomain of T cell receptor zeta chain, or
(iv) a CD30 CAR expressing a CD30-specific scFv fragment, and a chimeric HLA accessory receptor fused with a cytolytic endodomain of T cell receptor zeta chain; or
(v) a CD30 CAR expressing a CD30-specific scFv fragment, and a chimeric alloimmune defense receptor (ADR); and/or
(vi) a chimeric alloimmune defense receptor (ADR), and a chimeric HLA accessory receptor fused with a cytolytic endodomain of T cell receptor zeta chain, and/or
(vii) a combination thereof;
c) expanding the population of allogeneic VST-CAR-T cells in (b) in the presence of one or more cytokines in vitro to achieve a therapeutic dose (of at least 40 × 10⁶ to 800 × 10⁶ cancer killing T cells);
d) suitable for infusing an eligible patient with the allogeneic VST-CAR-T cells in (c) (one or more times in an allogeneic setting);
e) creating VST-CAR-T cells that express a nonexhausted PD-1^{neg}, TIGIT^{neg} TCF-1^{hi}TOX^{lo} early T stem cell (T_{SCM}) phenotype, suitably by:
(i) enriching for a population of CD4+ T cells, CD8+ T cells or a combination thereof that express a nonexhausted PD-1^{neg}, TIGIT^{neg} TCF-1^{hi}TOX^{lo} early T stem cell (T_{SCM}) phenotype;
(ii) generating by transducing the population of CD4+ T cells, CD8+ T cells or a combination thereof with a viral vector encoding a protein of a virus an expanded population of allogeneic virus-specific T cells (VSTs) comprising T cell receptors (TCRs) specific for the protein of the virus;
(iii) genetically modifying the population of allogeneic VSTs in (ii) to stably express up to three chimeric antigen-receptors (CARs), wherein each CAR is specific for a cancer antigen, wherein to reduce risk of host versus graft rejection (HVGR), one or more of the CARs comprise:
(1) a CD30 CAR expressing a CD30-specific scFv fragment, or
(2) a chimeric alloimmune defense receptor (ADR), or
(3) a chimeric HLA accessory receptor fused with a cytolytic endodomain of T cell receptor zeta chain, or
(4) a CD30 CAR expressing a CD30-specific scFv fragment, and a chimeric HLA accessory receptor fused with a cytolytic endodomain of T cell receptor zeta chain; or
(5) a CD30 CAR expressing a CD30-specific scFv fragment, and a chimeric alloimmune defense receptor (ADR); or
(6) a chimeric alloimmune defense receptor (ADR), and a chimeric HLA accessory receptor fused with a cytolytic endodomain of T cell receptor zeta chain; and/ or
(7) a combination thereof;
f) suitable for iteratively dosing the patient with the PD-1^{neg} TIGIT^{neg} TCF-1^{hi}TOX^{lo} early stem cell T cell (T_{SCM}) phenotype VST-CAR-T of step (e) as needed (until all cancer in the body is destroyed).

2. The method according to claim 1, wherein the allogeneic T cells of step (a)
are derived from umbilical cord blood (UCB); or
are derived from adult peripheral blood (PB); and/or
are derived from adult peripheral blood (PB), after mobilization of peripheral blood stem cells (with an infusion of G-CSF).

3. The method according to claim 2 or 3, wherein CD3+ allogeneic T cells are isolated from the allogeneic T cells by negative selection.

4. The method according to any preceding claim, wherein step (a) comprises transducing CD3+ allogeneic T cells with a viral vector (encoding proteins of one or more viruses).

5. The method according to claim 4, wherein the viruses are selected from the viruses in table 5.

6. The method according to any preceding claim, wherein in step (b) and/or step (e) the VSTs are transduced by a retroviral vector, suitably comprising a nucleic acid encoding a synthetic chimeric antigen receptor (CAR) that can specifically bind the cancer antigen (so as to stably express the cancer antigen-specific CAR).

7. The method according to claim 6,
wherein the cancer antigen is a tumor associated antigen and/or a tumor selective antigen; and/or
wherein for each cancer antigen, the CAR can comprise an extracellular antigen recognition domain comprising a single-chain variable fragment (scFv) derived from a monoclonal antibody specific for the antigen, a spacer/hinge region and transmembrane domain; and/or an intracellular signal transduction domain comprising a CD3ζ T cell activation chain and optionally one or more costimulatory molecule(s).

8. The method according to any preceding claim, wherein
the alloimmune defense receptor (ADR) is an anti 4-1BB receptor;
the HLA accessory receptor comprises beta2-microglobulin fused with the cytolytic endodomain of the T cell receptor zeta chain; and/or
the alloimmune defense receptor (ADR) is an anti 4-1BB receptor and/or the HLA accessory receptor comprises beta2-microglobulin fused with the cytolytic endodomain of the T cell receptor zeta chain.

9. The method according to claim 7, wherein the tumor-associated antigen is selected from table 6 and/or wherein the tumor specific antigen is encoded by a gene from table 7.

10. The method according to any preceding claim, wherein in step (c), the cytokine is one or more of IL-2, IL-7, and/or IL-15.

11. The method according to any preceding claim, wherein in step (d), the recipient eligible patient can undergo infusion of the population of VST-CAR-T cells (following lymphodepleting chemotherapy).

12. The method according to claim 11, wherein the lymphodepleting chemotherapy comprises cyclophosphamide (CYTOXAN^{®}) therapy (such as 500-600 mg/m2 e.g. for 3 days) and/or fludarabine (such as 20 mg/m2 e.g. for 3-4 days), suitablywith a stagger (of up to 7 days) between participants (to evaluate dose limiting toxicities or an equivalent clinical regimen).

13. The method according to any preceding claim, wherein (to further decrease the risk of the VST-CAR-T cells being killed by the recipient's cells), the recipient eligible patient can be at least partially HLA matched (against HLA stocks of the VST-CAR-T cells).

14. The method according to any preceding claim, wherein in step (e), the population of CD4+ T cells, CD8+ T cells or both CD4+ T cells and CD8+ T cells expressing a nonexhausted phenotype comprise a PD-1^{neg}TIGIT^{neg}TCF-1^{hi}TOX^{lo} early stem cell (T_{SCM}) phenotype
are derived from umbilical cord blood (UCB) and/or
are derived from healthy donor adult peripheral blood.

15. The method according to claim 14 wherein the recipient can be at least partially HLA matched against the umbilical cord blood or adult peripheral blood.

16. The method according to claim 14, wherein the population of CD4+ T cells, CD8+ T cells, or both CD4+ and CD8+ T cells is isolated from mononuclear cells (by negative selection).

17. The method according to claim 14, wherein the population of CD4+ T cells, CD8+ T cells or both CD4+ T cells and CD8+ T cells expressing a nonexhausted phenotype comprising a PD1^{neg}TIGIT^{neg}TCF-1^{hi}TOX^{lo} early stem cell (T_{SCM}) phenotype
(a) is expanded in vitro in the presence of one or more cytokines; or
(b) is expanded in vitro in the presence of an epigenetic modification agent; and/or
(c) is expanded in vitro in the presence of one or more cytokines and an epigenetic modification agent.

18. The method according to claim 17, wherein the epigenetic modification agent is selected from belinostat, vorinostat, romidepsin, panobinostat, decitabine, and/or azacitidine.

19. The method according to claim 17, wherein the cytokine is at least two cytokines selected from IL-7, IL-15, and/or IL-21.

20. The method according to claim 17, wherein an expanded nonexhausted population of CD4+ T cells, CD8+ T cells, or CD4+ T cells and CD8+ T cells expressing a nonexhausted phenotype comprising a PD-1^{neg}TIGIT^{neg}TCF-1^{hi}TOX^{lo} early stem cell (T_{SCM}) phenotype of step (e) can be administered to the subject, periodically as needed.

21. The method according to any preceding claim, wherein the method further includes administering an approved immune checkpoint inhibitor (at a dose standard for the cancer indication).

22. The method according to claim 21, wherein the checkpoint inhibitor comprises anti-PD1 (e.g., lambrolizumab, pembrolizumab (KEYTRUDA^{®}) and nivolumab (OPDIVO^{®}), anti-PD-L1 (MPDL3280A, Atezolizumab, TECENTRIQ^{®}), and/or anti-CTLA4 (ipilimumab, YERVOY^{®}).

23. The method according to any preceding claim, wherein the cancer is a solid tumor, a leukemia, a lymphoma, or a plasma cell dyscrasia (myeloma).

24. The method according to any preceding claim, wherein the population of CD4+ T cells, CD8+ T cells or both CD4+ T cells and CD8+ T cells expressing a nonexhausted phenotype comprising a PD1^{neg}TIGIT^{neg}TCF-1^{hi}TOX^{lo} early stem cell (T_{SCM}) phenotype is genetically engineered:
a. to overexpress FOXO1; or
b. by biallelic disruption of TET2 via CAR integration into intron 9; or
c. by deleting DNMT3A; or
d. by disrupting histone methyltransferase SUV39H1; or
e. by generating the CAR-T cells in presence of exogenous transforming factor beta; or
f. by exposure to Iosine; or
g. by EZH2 inhibition and overexpression of c-Jun; and/or
h. or by a combination thereof.

25. A composition prepared by, or preparable by, a method according to any preceding claim, said composition being suitable for use in a method of treating cancer.
